# EUROPEAN PATENT APPLICATION

(11) **EP 2 301 922 A1**
(43) Date of publication of application: **30.03.2011**
(21) Application number: 09766638.2
(22) Date of filing: 16.06.2009
(51) Int. Cl.: C07D 213/64, A61K 31/4412, A61K 31/4436, A61K 31/4439, A61K 31/444, A61K 31/538, A61K 31/5415, A61P 7/02, A61P 9/00, A61P 9/10, A61P 43/00, C07D 213/69, C07D 213/79, C07D 401/06, C07D 409/12, C07D 413/06, C07D 413/10, C07D 413/14, C07D 417/06, C07D 417/12

(54) **PYRIDONE COMPOUND**

(30) Priority: 17.06.2008 JP 2008157818
(71) Applicant: Astellas Pharma Inc., Tokyo 103-8411 (JP)
(72) Inventor: KAMIKUBO, Takashi, Tokyo 103-8411 (JP); MIURA, Masanori, Tokyo 103-8411 (JP); OKUDA, Takao, Tokyo 103-8411 (JP); MAKI, Keisuke, Tokyo 103-8411 (JP); HIRAYAMA, Fukushi, Tokyo 103-8411 (JP); MORITOMO, Ayako, Tokyo 103-8411 (JP); KOMIYA, Yuriko, Tokyo 103-8411 (JP); MATSUURA, Keisuke, Tokyo 103-8411 (JP); IBUKA, Ryotaro, Tokyo 103-8411 (JP)
(74) Representative: Gille Hrabal Struck Neidlein Prop Roos
(86) International application number: PCT/JP2009/060925
(87) International publication number: WO 2009/154190

(57) **Abstract**

[Problems] A useful compound which can be used as a pharmaceutical, in particular, an agent for treating peripheral arterial occlusive disease is provided.

[Means for Solution] The present inventors have conducted extensive studies on EP4 receptor agonists, and as a result, have found that a novel pyridone compound, in which a group having an acidic group is substituted at the 1-position of the pyridone ring, the 6-position is bonded with an aromatic ring group via a linking part, and the linking part contains a nitrogen atom, has an excellent EP4 receptor agonistic action, thereby completing the present invention. Since the compound of the present invention has an excellent EP4 receptor agonistic action, it is useful as a pharmaceutical, in particular, as an agent for treating peripheral arterial occlusive disease.

SEQUENCE LISTING

<110> Astellas Pharma Inc.

<120> Pyridone compounds

<130> A09009

<150> JP2008/157818
<151> 2008-06-17

<160> 1

<210> 1

<211> 1501

<212> DNA

<213> Rat

<400> 1
aagctgtgta ctactgacca ccatcatgtc catccccgga gtcaacgcgt ccttctcctc 60
cactccggag aggttgaaca gcccagtgac cattcccgca gtgatgttta tcttcggggt 120
ggtgggcaac ctggtggcca tcgtagtatt gtgcaagtcg cgcaaggagc agaaggagac 180
taccttttac actctggtat gtgggctggc tgtcactgac ctactgggca cattgttggt 240
aagcccagtg accatcgcca catacatgaa gggccagtgg cccggagacc aggcattgtg 300
tgactacagc accttcatcc tacttttctt cggcctgtcg ggtctcagca tcatctgtgc 360
catgagcatt gagcgctacc tggccatcaa ccacgcctac ttctacagcc actacgtgga 420
caagcggctg gccggtctca cgctcttcgc cgtctatgca tctaacgtgc tcttctgcgc 480
actgcccaac atgggcctgg gtaggtccga gcggcagtac ccggggacct ggtgcttcat 540
cgactggacc accaacgtaa cggcctacgc cgccttctct tacatgtacg cgggcttcag 600

ttccttcctc atcctcgcca ccgtgctctg caatgtgctg gtgtgcggcg cgctgctccg 660
catgctccgc cagttcatgc gccgcacctc gctgggcacg gagcagcacc acgcggccgc 720
tgcagcagcg gtggcttcgg tggcctgtcg gggtcacgcg gccgcctccc cagccctgca 780
gcgcctcagt gactttcgcc gccgcaggag cttccggcgc atcgcgggtg cagagatcca 840
gatggtcatc ttactcatcg ccacctctct ggtggtgctc atctgctcca ttccgctcgt 900
ggtgcgagtg ttcatcaacc agttatatca gccaagtgtg gtgaaagaca tcagcagaaa 960
cccggatttg caggccatca gaattgcttc tgtgaacccc atcctggacc cttggatcta 1020
catccttctt cggaagactg tgctcagtaa agccatagaa aagatcaagt gcctcttctg 1080
ccgcattggt ggttctggca gagacggttc agcacagcac tgctcagaga gtcggaggac 1140
atcttctgcc atgtctggcc actcccgctc cttcctctcg cgggagttga gggagatcag 1200
cagcacctct cacaccctcc tatacctgcc agacctaact gaaagcagcc tcggaggcaa 1260
gaatttgctt ccaggtacgc atggcatggg cctgacccaa gcagacacca cctcgctgag 1320
aactttgcga atttcagaga cctcagactc ctcccagggc caggactctg agagtgtctt 1380
gttggtggat gaggttagtg ggagccagag agaggagcct gcctctaagg ggaactctct 1440
gcaagtcacg ttccccagtg aaacgctgaa attatctgaa aaatgtatat agtagcttaa 1500
a 1501

## Description

### Technical Field

The present invention relates to a pharmaceutical, in particular, a pyridone compound which is useful as an agent for treating peripheral arterial occlusive disease.

### Background Art

Peripheral arterial occlusive disease, caused by artery stenosis/occlusion due to arteriosclerosis and thrombus formation, thus leading the peripheral, in particular, the lower extremities into ischemia, is a disease with symptoms such as coldness, intermittent claudication, pain, ulcers/necrosis of the lower extremities, and the like. As for the diagnosis and treatment of the peripheral arterial occlusive disease, the guidelines are provided in "Trans Atlantic Inter-Society Consensus for Management of Peripheral Arterial Disease (TASC) II" (Eur. J. Vasc. Endovasc. Surg, 2007, 33 (1), S1). For the improvement of the symptoms of the lower extremities, it is important to improve the blood flow into the ischemic part, and treatment for promoting the resumption of the blood circulation by a pharmaceutical or physical method is carried out. For a drug therapy, drugs having a vasodilating action or a platelet aggregation inhibiting action have been used.

PGE2 is known as one of the metabolites in an arachidonic acid cascade. The PGE2 exhibits various physiological activities such as a pain inducing and increasing action, a pro-inflammatory action, an anti-inflammatory action, an uterine contractile action, a digestive peristalsis promoting action, an awaking action, a gastric acid secretion inhibiting action, a hypotensive action, a platelet aggregation inhibiting action, an angiogenic action, and the like. It has become clear that there are four subtypes of PGE2 receptors, EP1, EP2, EP3 and EP4, which have wide distributions in various tissues. The activation of the EP1 receptor is believed to cause the increase in intracellular Ca²⁺. For the EP3 receptor, there exist the receptors having different pathways for second-messenger systems. The activation of the EP2 and EP4 receptors is believed to cause the activation of an adenylate cyclase, and thus to increase the intracellular cAMP level (Phsiol. Rev., 1999, 79, 1193).

The EP4 receptor is associated with smooth muscle relaxation through the increase in cAMP (Br. J. Pharmacol., 2001, 134, 313). Further, it is suggested that the platelet aggregation inhibiting action is exhibited via EP4 in that the expression of the EP4 receptors (Circulation, 2001, 104, 1176) and the cAMP increasing action by PGE2 (Prostaglandins, 1996, 52, 175) are also demonstrated in the platelets. From this, the EP4 agonist is expected to be an agent for treating peripheral arterial occlusive disease, which exhibits a blood flow improving action. In addition to these, it is believed that it is useful as an agent for treating renal diseases, inflammatory diseases, bone diseases, gastric mucosal protection, glaucoma, and the like, from the viewpoint that EP4 receptor is associated with increase in the renal blood flow (Am. J. Physiol. 279, F755, 2000), inhibition of the mesangium cell proliferation (Kid. Int., 1999, 56, 589), inhibition of the inflammatory cytokine production (Biochem. Pharmacol., 2001, 61, 1153), osteogenesis (Proc. Natl. Acad. Sci. U.S.A., 2002, 99, 4580), secretion of the gastrointestinal mucus (Gastroenterology, 1999, 117, 1352), intraocular pressure control (Patent Documents 1 to 5), and the like.

As compounds having an EP4 receptor agonistic action, following Patent Documents 1 to 7 are reported.
In Patent Document 1, it has been reported that a compound represented by the following formula (A) has an EP4 receptor agonistic action, and is thus useful for the treatment of glaucoma, osteoporosis, and the like. 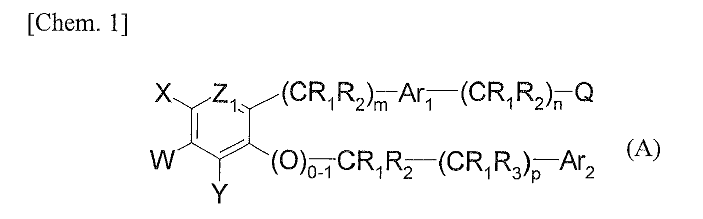 (For the symbols in the formula, refer to the publication.)

In Patent Document 2, it has been reported that a compound represented by the following formula (B) has an EP4 receptor agonistic action, and is thus useful for the treatment of glaucoma, osteoporosis, and the like. 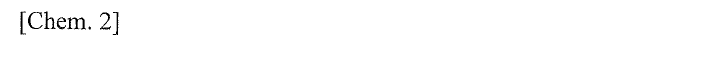 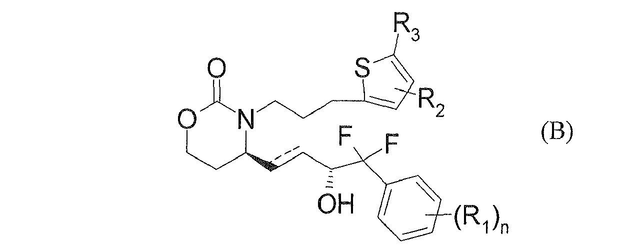 (For the symbols in the formula, refer to the publication.)

In Patent Document 3, it has been reported that a compound represented by the following formula (C) has an EP4 receptor agonistic action, and is thus useful for the treatment of glaucoma, osteoporosis, and the like. 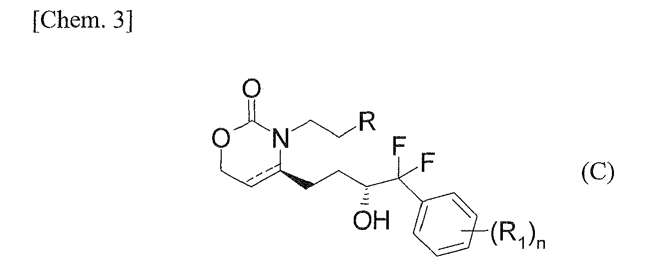 (For the symbols in the formula, refer to the publication.)

In Patent Document 4, it has been reported that a compound represented by the following formula (D) has an EP4 receptor agonistic action, and is thus useful for the treatment of glaucoma, inflammatory bowel disease, and the like. 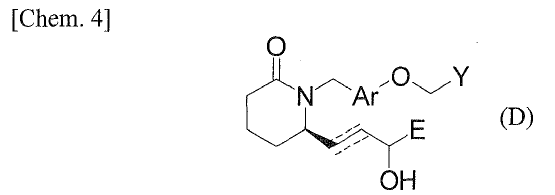 (For the symbols in the formula, refer to the publication.)

In Patent Document 5, it has been reported that a compound represented by the following formula (E) has an EP4 receptor agonistic action, and is thus useful for the treatment of glaucoma, ocular hypertension, and the like. 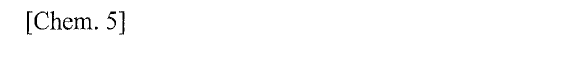 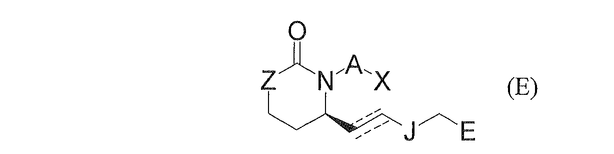 (For the symbols in the formula, refer to the publication.)

In Patent Document 6, it has been reported that a compound represented by the following formula (F) has an EP4 receptor agonistic action, and is thus useful for the treatment of osteoporosis, and other bone diseases. 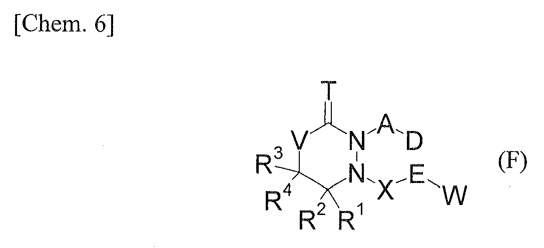 (For the symbols in the formula, refer to the publication.)

In Patent Document 7, which is an application filed by the present Applicant(s) and published after the priority date of the present application, it is reported that a compound represented by the following formula (G) has an EPR4 agonistic action and is useful against peripheral arterial occlusive disease.

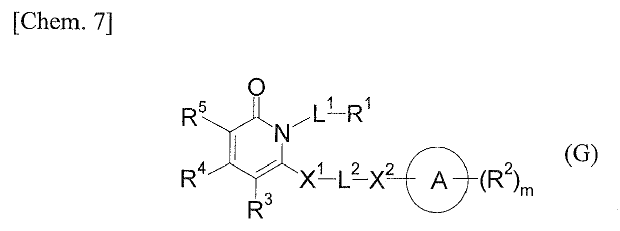

Furthermore, the following compounds are reported as a pyridone derivative.
In Patent Document 8, it has been reported that a compound represented by the following formula (H) is useful as an agent for controlling plant disease. Also, it is reported that a compound represented by the following formula (H-1) is useful as a synthesis intermediate. However, there is no disclosure or suggestion of its usefulness as a pharmaceutical. 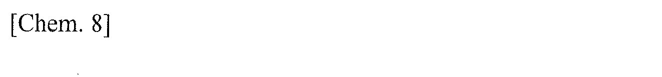 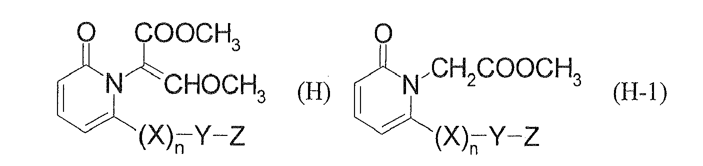 (For the symbols in the formula, refer to the publication)

In Patent Document 9, it has been reported that a wide range of the compound represented by the following formula (J) exhibit an LXR modulating action, and is thus useful for the treatment of hypercholesterolemia, diabetes, and the like. However, there is no description of specific compounds included in the present invention. In addition, there is no description of the action on the EP4 receptor and the usefulness against peripheral arterial occlusive disease. 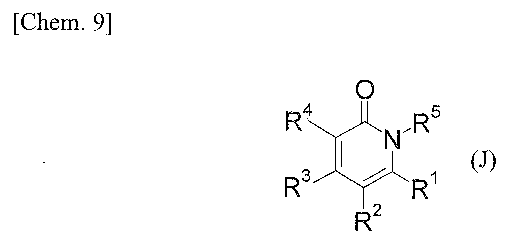 (For the symbols in the formula, refer to the publication)

### List of the Documents

### Patent Documents

[Patent Document 1] Pamphlet of International Publication No. WO2005/116010
[Patent Document 2] Pamphlet of International Publication No. WO2007/014454
[Patent Document 3] Pamphlet of International Publication No. WO2007/014462
[Patent Document 4] Pamphlet of International Publication No. WO2006/052630
[Patent Document 5] Pamphlet of International Publication No. WO2006/014207
[Patent Document 6] Pamphlet of International Publication No. WO2006/080323
[Patent Document 7] Pamphlet of International Publication No. WO2008/149965
[Patent Document 8] Specification of European Patent Application Publication No. 535980
[Patent Document 9] Pamphlet of International Publication No. WO2003/059884

### Summary of the Invention

### Problem that the Invention is to Solve

It is an object of the present invention to provide a novel compound which is useful as a novel pharmaceutical having a selective agonistic action to a prostaglandin EP4 receptor, in particular, an agent for treating peripheral arterial occlusive disease.

### Means for Solving the Problem

The present inventors have conducted extensive studies on compounds having a selective agonistic action to a prostaglandin EP4 receptor, and as a result, have found that a novel pyridone derivative in which a group having an acidic group is substituted at the 1-position in the pyridone ring, the 6-position is bonded with various nitrogen-containing hetero ring groups or aryl via a linking part, and the linking part contains a nitrogen atom has an excellent EP4 receptor agonistic action, thereby completing the present invention.
That is, the present invention relates to a compound represented by the formula (I) or a pharmaceutically acceptable salt thereof. 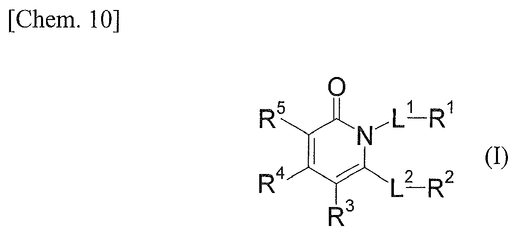 [The symbols in the formula have the following meanings:
L¹: lower alkylene, lower alkenylene, -(lower alkylene)-O-(lower alkylene)-, or -(lower alkylene)-S-(lower alkylene)-, in which lower alkylene and lower alkenylene in L¹ may each be substituted,
L²: lower alkylene, lower alkenylene, -C(O)-, -(lower alkylene)-C(O)-, or -(lower alkenylene)-C(O)-, in which lower alkylene and lower alkenylene in L² may each be substituted,
R¹: R⁶ or a group represented by the following formula (II): 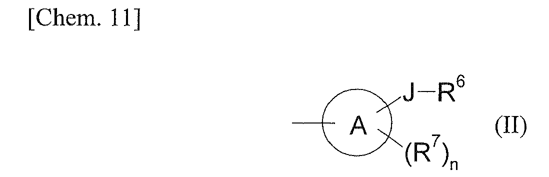
Ring A: aryl or heteroaryl,
R⁶: -CO₂R⁰, -C(O)N(R⁰)₂, -C(O)N(H)S(O)₂-R⁸, -C(O)N(H)S(O)₂N(R⁰)-R⁸, -N(R⁰)C(O)N(H)S(O)₂-R⁸, or a group represented by any one of the following formulae (III) to (XIV): 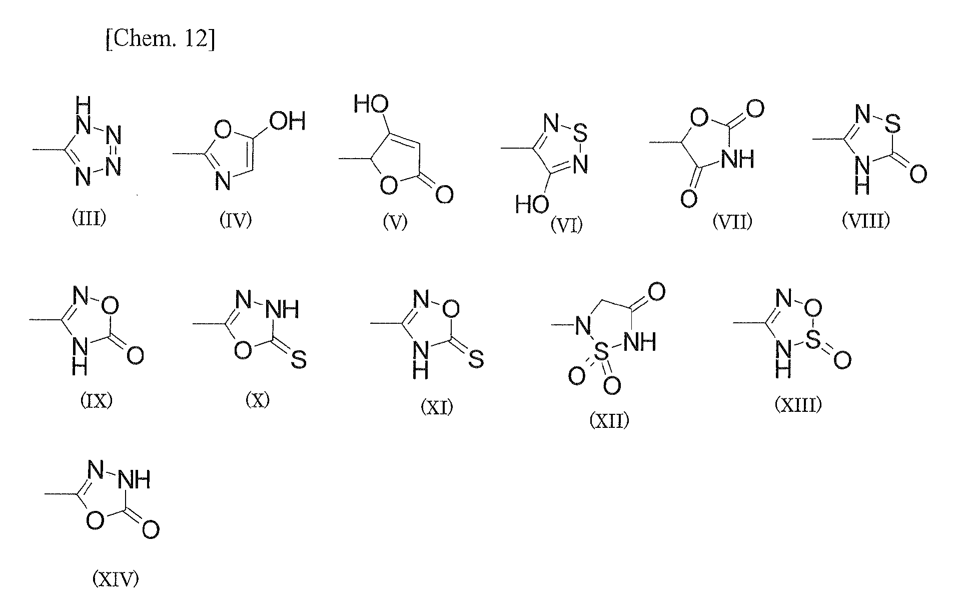
R⁰: the same as or different from each other, each representing -H or lower alkyl,
R⁸: lower alkyl, halogeno-lower alkyl, -(lower alkylene)-OR⁰, or -(lower alkylene)-OC(O)R⁰,
J: a single bond or lower alkylene,
R⁷: the same as or different from each other, each representing lower alkyl, lower alkenyl, halogen, halogeno-lower alkyl, -OR⁰, -O-(halogeno-lower alkyl), -O-(cycloalkyl), -O-(lower alkylene)-OR⁰, -O-(lower alkylene)-aryl, -OC(O)R⁰, -N(R⁰)₂, -(lower alkylene)-OH, -(lower alkylene)-OR⁰, -(lower alkylene)-N(R⁰)₂, -(lower alkylene)-cycloalkyl, -(lower alkylene)-aryl, -CO-R⁰, -S(O)₂-R⁰, -CO₂R⁰, -C(O)N(R⁰)₂, aryl, or a hetero ring group, in which aryl and the hetero ring group in R⁷ may each be substituted,
n: an integer of 0 to 3,
R²: -N(R⁰)-lower alkyl or a group represented by any one of the following formulae (XV) to (XVII): 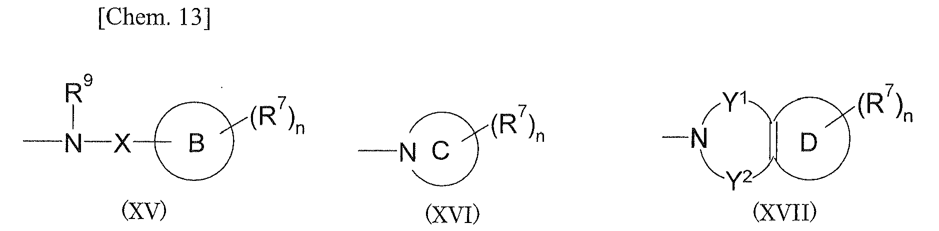
Ring B: cycloalkyl, aryl, or a hetero ring,
Ring C: a nitrogen-containing saturated hetero ring,
Ring D: aryl or heteroaryl,
X: a single bond, lower alkylene, -C(O)-, -C(O)-(lower alkylene)-, or -(lower alkylene)-O-,
R⁹: H, lower alkyl, -C(O)R⁰, or aryl, in which aryl in R⁹ may be substituted,
Y¹ and Y²: the same as or different from each other, each representing a single bond, -[C(R¹⁰)(R1¹⁾]ₛ-, -[C(R¹⁰)(R¹¹)]ₛ-Q-, -Q-[C(R¹⁰)(R¹¹)]ₛ-, or -[C(R¹⁰)(R¹¹)]ₛ-Q-[C(R¹⁰)(R¹¹)]ₜ-,
R¹⁰ and R¹¹: the same as or different from each other, each representing H, lower alkyl, halogen, halogeno-lower alkyl, -OR⁰, -N(R⁰)₂, -(lower alkylene)-OH, -(lower alkylene)-OR⁰, -(lower alkylene)-N(R⁰)₂, or a hetero ring group, or R¹⁰ and R¹¹ on the same carbon atom may be combined to form oxo,
Q: O, S(O)p, or N(R¹²),
R¹²: H, lower alkyl, -C(O)R⁰, or -S(O)₂-(lower alkyl),
s and t: the same as or different from each other, each representing an integer of 1 to 4,
p: an integer of 0 to 2, and
R³, R⁴, and R⁵: the same as or different from each other, each representing H, halogen, -CN, lower alkyl, lower alkenyl, halogeno-lower alkyl, -OR⁰, -O-(halogeno-lower alkyl), -(lower alkylene)-OR⁰, -(lower alkylene)-N(R⁰)₂, -CO₂R⁰, -C(O)N(R⁰)₂, cycloalkyl, or aryl, in which aryl in R³, R⁴, and R⁵ may be substituted].
(In this connection, these symbols represent the same meanings below in the present specification unless otherwise particularly explained.)

Further, the present invention also relates to a pharmaceutical composition comprising the pyridone compound represented by the formula (I) or a pharmaceutically acceptable salt thereof, and a pharmaceutically acceptable carrier, in particular, a pharmaceutical composition which is an EP4 agonist, or an agent for preventing or treating peripheral arterial occlusive disease. That is, the present invention relates to;
(1) a pharmaceutical composition comprising the compound represented by the formula (I) or a pharmaceutically acceptable salt thereof, and a pharmaceutically acceptable carrier,
(2) the pharmaceutical composition as described in (1) which is an EP4 agonist,
(3) the pharmaceutical composition as described in (1) which is an agent for preventing or treating peripheral arterial occlusive disease,
(4) use of the compound represented by the formula (I) or a pharmaceutically acceptable salt thereof for the manufacture of an agent for preventing or treating peripheral arterial occlusive disease, and
(5) a method for preventing or treating peripheral arterial occlusive disease, comprising administering to a patient an effective amount of the compound represented by the formula (I) or a pharmaceutically acceptable salt thereof.

### Effects of the Invention

Since the compound of the formula (I) has an EP4 receptor agonistic action, it is useful as an agent for preventing and/or treating peripheral arterial occlusive disease and the like.

### Mode for Carrying Out the Invention

Hereinbelow, the present invention will be described in detail.
In the present specification, the "lower alkyl" is linear or branched alkyl having 1 to 6 carbon atoms (which is hereinafter simply referred to as C₁₋₆) in a certain embodiment, and examples thereof include methyl, ethyl, n-propyl, isopropyl, n-butyl, isobutyl, sec-butyl, tert-butyl, n-pentyl, n-hexyl group, and the like. In another embodiment, it is a C₁₋₄ alkyl group, in a further embodiment, it is methyl, ethyl, n-propyl, isopropyl, n-butyl, or sec-butyl group, and in a further embodiment, it is methyl, ethyl, or n-propyl.

The "lower alkenyl" is preferably linear or branched C₂₋₆ alkenyl, specifically, vinyl, allyl, isopropenyl, butenyl, pentenyl, 1-methylvinyl, 1-methyl-2-propenyl, 1,3-butadienyl, 1,3-pentadienyl group, or the like. More preferably, it is C₂₋₄ alkenyl, and particularly preferably, vinyl, allyl, or isopropenyl.

The "lower alkylene" is linear or branched C₁₋₆ alkylene in a certain embodiment, and examples thereof include methylene, ethylene, trimethylene, tetramethylene, pentamethylene, hexamethylene, propylene, methylmethylene, ethylethylene, 1,2-dimethylethylene, 1,1,2,2-tetramethylethylene group, and the like. In another embodiment, it is C₁₋₄ alkylene group, in a further embodiment, it is methylene, ethylene, trimethylene, tetramethylene, pentamethylene, or hexamethylene group, and in a further embodiment, it is methylene or ethylene.

The "lower alkenylene" is linear or branched C₂₋₆ alkenylene in a certain embodiment, and examples thereof include vinylene, ethylidene, propenylene, butenylene, pentenylene, hexenylene, 1,3-butadienylene, 1,3-pentadienylene group, and the like. In another embodiment, it is a C₂₋₄ alkenylene group, and in a further embodiment, it is vinylene or a propenylene group.

The "cycloalkyl" is a C₃₋₁₀ saturated hydrocarbon ring group in a certain embodiment, and may have a bridge. Specifically, it is cyclopropyl, cyclobutyl, cyclopentyl, cyclohexyl, cycloheptyl, cyclooctyl, norbornyl, adamantyl group, or the like. In another embodiment, it is C₃₋₈ cycloalkyl group, and in a further embodiment, it is cyclopropyl, cyclobutyl, cyclopentyl, or cyclohexyl group.

The "halogen" means F, Cl, Br, or I.
The "halogeno-lower alkyl" is C₁₋₆ alkyl substituted with one or more halogen. In a certain embodiment, it is lower alkyl substituted with 1 to 5 halogen, in another embodiment, it is fluoromethyl, difluoromethyl, trifluoromethyl, 2,2,2-trifluoroethyl, or pentafluoroethyl, and in a further embodiment, trifluoromethyl.

The "aryl" refers to a C₆₋₁₄ monocyclic to tricyclic aromatic hydrocarbon ring group, in a certain embodiment, it is phenyl or naphthyl, and in another embodiment, it is phenyl.

The "hetero ring" group is a ring group containing i) a monocyclic 3- to 8-membered, in a certain embodiment, a monocyclic 5- to 7-membered hetero ring, containing 1 to 4 hetero atoms selected from O, S and N, or ii) a bicyclic or tricyclic hetero ring containing 1 to 5 hetero atoms selected from oxygen, sulfur, and nitrogen, which is formed by the condensation of the monocyclic hetero ring with one or two rings selected from the group consisting of a monocyclic hetero ring, a benzene ring, a C₅₋₈ cycloalkyl ring, and a C₅₋₈ cycloalkenyl ring. The ring atom, sulfur or nitrogen, may be oxidized to form an oxide or a dioxide. Specifically, it is aziridinyl, azetidinyl, pyrrolidinyl, piperidinyl, azepanyl, piperazinyl, morpholinyl, thiomorpholinyl, oxetanyl, tetrahydrofuranyl, tetrahydropyranyl, tetrahydrothienyl, tetrahydrothiopyranyl, pyrrolyl, imidazolyl, pyrazolyl, triazolyl, tetrazolyl, pyridyl, pyrimidinyl, pyrazinyl, furyl, thienyl, oxazolyl, oxadiazolyl, thiazolyl, thiadiazolyl, indolyl, indazolyl, benzoimidazolyl, imidazopyridyl, quinolyl, quinazolyl, quinoxalinyl, naphthylidinyl, benzofuranyl, benzothienyl, benzoxazolyl, benzothiazolyl, carbazolyl, dihydroindolyl, tetrahydroquinolyl, tetrahydroisoquinolyl, quinuclidinyl group, or the like. In another embodiment, it is a 5- to 10-membered monocyclic or bicyclic hetero ring group, and in a further embodiment, it is pyrrolidyl, piperidinyl, piperazinyl, morpholinyl, thiomorpholinyl, pyridyl, furyl, or thienyl group.

The "heteroaryl" means a ring group containing i) a 5- to 6-membered monocyclic heteroaryl containing 1 to 4 hetero atoms selected from O, S, and N, or ii) a bicyclic 8- to 10-membered hetero ring and a tricyclic 11- to 14-membered hetero ring, each containing 1 to 5 hetero atoms selected from O, S, and N, which are each formed by the condensation of the monocyclic heteroaryl with one or two rings selected from the group consisting of a monocyclic heteroaryl and a benzene ring; among the above-described "hetero ring" groups. The ring atom, S or N, may be oxidized to form an oxide. Specifically, the it is pyrrolyl, imidazolyl, pyrazolyl, triazolyl, tetrazolyl, pyridyl, pyrimidinyl, pyrazinyl, furyl, thienyl, oxazolyl, oxadiazolyl, thiazolyl, thiadiazolyl, indolyl, indazolyl, benzoimidazolyl, imidazopyridyl, quinolyl, quinazolyl, quinoxalinyl, naphthylidinyl, benzofuranyl, benzothienyl, benzoxazolyl, benzothiazolyl, or carbazolyl group, and in another embodiment, it is pyridyl, furyl, or thienyl group.

The "nitrogen-containing saturated hetero ring" group means a saturated hetero ring group containing at least one nitrogen atom, among the above-described "hetero ring" groups, and in a certain embodiment, it is a monocyclic 5- to 7-membered nitrogen-containing saturated hetero ring. Specifically, it is aziridinyl, azetidinyl, pyrrolidinyl, piperidinyl, azepanyl, piperazinyl, morpholinyl, or thiomorpholinyl group, and in another embodiment, it is pyrrolidinyl, piperidinyl, piperazinyl, morpholinyl, or thiomorpholinyl group.

The "which may be substituted" refers to "which is unsubstituted" or "which is substituted with 1 to 5 substituents which are the same as or different from each other". The "which is substituted" refers to "which is substituted with 1 to 5 substituents which are the same as or different from each other". Further, if it has a plurality of substituents, the substituents may be the same as or different from each other.

Examples of the substituents in the "lower alkylene" and the "lower alkenylene", which may each be substituted, in L¹; and the "lower alkylene" and the "lower alkenylene", which may each be substituted, in L^{1a} include halogen or -OR⁰.
Examples of the substituents in the "lower alkylene" and the "lower alkenylene", which may each be substituted, in L²; and the "lower alkylene" and the "lower alkenylene", which may each be substituted, in L^{2a} include halogen or -OR⁰.
Examples of the substituents in the "aryl" which may be substituted in R⁷; the "aryl" which may be substituted in R^{7a}; the "aryl" which may be substituted in R⁹; and the "aryl" which may be substituted in R³, R⁴, and R⁵ include a group selected from the group consisting of lower alkyl, halogen, halogeno-lower alkyl, -OR⁰, and -O-(halogeno-lower alkyl).
Examples of the substituents acceptable in the "hetero ring" group which may be substituted in R⁷; and the "hetero ring" group which may be substituted in R^{7a} include a group selected from the group consisting of lower alkyl, halogen, halogeno-lower alkyl, -OR⁰, -O-halogeno-lower alkyl, and oxo.

The "selective" in the "the selective agonist to the EP4 receptor" means that the agonistic actions shown in Test Examples 2 and 3 as described below are higher in the subtype EP4 of the prostaglandin receptor than the subtypes EP1, EP2, and EP3. The difference in the agonistic actions is preferably 5-fold or more, more preferably 10-fold or more, and even more preferably 100-fold or more.

Certain embodiments of the compound of the present invention will be described below.
(a) The compound, in which L¹ is lower alkylene which may be substituted, in another embodiment, lower alkylene, in a further embodiment, linear C₂₋₄ alkylene, and in a further embodiment, ethylene.
(b) The compound, in which L² is lower alkylene or -(lower alkylene)-C(O)-, in another embodiment, lower alkylene, and in a further embodiment, methylene.
(c) The compound, in which R¹ is -CO₂R⁰, in another embodiment, a group represented by the formula (II), in a further embodiment, phenyl which is substituted with a group selected from the group consisting of -CO₂R⁰, and the groups represented by the following formula (III), the following formula (IX), the following formula (X), and the following formula (XIV): 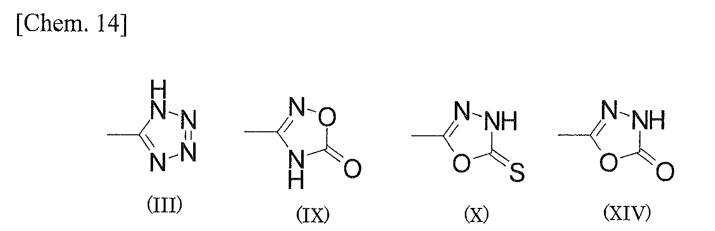 and which may be further substituted with 1 to 3 lower alkyl or halogen, and in a further embodiment, phenyl which may be substituted with -CO₂H.
(d) The compound, in which Ring A is aryl, and in another embodiment, phenyl.
(e) The compound, in which R⁶ is -CO₂R⁰, in another embodiment, -CO₂H, in a further embodiment, a group represented by the following formula (III), the following formula (IX), the following formula (X), or the following formula (XIV): 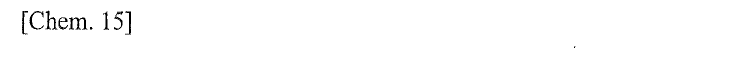 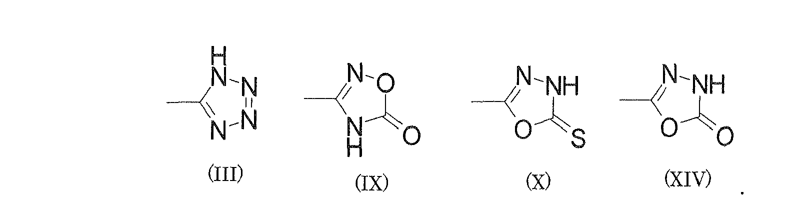
(f) The compound, in which J is a single bond.
(g) The compound, in which R⁷ is halogen, lower alkyl, halogeno-lower alkyl, -OR⁰, or -O-halogeno-lower alkyl.
(h) The compound, in which R² is -N(lower alkyl)₂ or a group represented by the formula (XV) above; in another embodiment, -N(lower alkyl)₂, -N(R⁰)-cycloalkyl, -N(R⁰)-(aryl which may be substituted with 1 to 3 groups selected from R⁷), or -N(R⁰)-(lower alkylene)-(aryl which may be substituted with 1 to 3 groups selected from R⁷); in a further embodiment, -N(lower alkyl)₂, -N(R⁰)-cycloalkyl, -N(R⁰)-(aryl which may be substituted with 1 to 3 groups selected from the group consisting of halogen, lower alkyl, halogeno-lower alkyl, -OR⁰, and -O-halogeno-lower alkyl), or -N(R⁰)-(lower alkylene)-(aryl which may be substituted with 1 to 3 groups selected from the group consisting of halogen, lower alkyl, halogeno-lower alkyl, -OR⁰, and -O-halogeno-lower alkyl); in a further embodiment, -N(R⁰)-(phenyl which may be substituted with 1 to 3 groups selected from R⁷), and in a further embodiment, -N(R⁰)-(phenyl which may be substituted with 1 to 3 groups selected from the group consisting of halogen, lower alkyl, halogeno-lower alkyl, -OR⁰, and -O-halogeno-lower alkyl).
(i) The compound, in which R² is a group represented by the formula (XVI) above; in another embodiment, a group in which Ring C of the formula (XVI) above is a monocyclic 5- to 7-membered nitrogen-containing saturated hetero ring; in a further embodiment, a group in which Ring C of the formula (XVI) above is a monocyclic 5- to 7-membered nitrogen-containing saturated hetero ring, and R⁷ is halogen, lower alkyl, lower alkenyl, halogeno-lower alkyl, -OR⁰, -O-halogeno-lower alkyl, or -(lower alkylene)-cycloalkyl; in a further embodiment, a group in which Ring C of the formula (XVI) above is pyrrolidin-1-yl, and in a further embodiment, Ring C of the formula (XVI) above is pyrrolidin-1-yl, and R⁷ is halogen, lower alkyl, lower alkenyl, halogeno-lower alkyl, -OR⁰, -O-halogeno-lower alkyl, or -(lower alkylene)-cycloalkyl.
(j) The compound, in which R² is a group represented by the formula (XVII) above, in another embodiment, 2,3-dihydro-1H-indol-1-yl which may be substituted with 1 to 3 groups selected from R⁷ at the 4- to 7-positions; in a further embodiment, 2,3-dihydro-1H-indol-1-yl which may be substituted with 1 to 3 groups selected from the group consisting of halogen, lower alkyl, lower alkenyl, halogeno-lower alkyl, -OR⁰, and -O-halogeno-lower alkyl at the 4- to 7-positions.
(k) The compound, in which Ring B is aryl, and in another embodiment, phenyl.
(l) The compound, in which Ring C is a monocyclic 5- to 7-membered nitrogen-containing saturated hetero ring, and in another embodiment, pyrrolidin-1-yl.
(m) The compound, in which the ring formed by combination of Ring D, Y¹, and Y² with a nitrogen atom is 2,3-dihydro-1H-indol-1-yl.
(n) The compound, in which X is a single bond or lower alkylene, and in another embodiment, a single bond.
(o) The compound, in which R³ is halogen, lower alkyl, or cycloalkyl, and in another embodiment, Cl, methyl, ethyl, or cyclopropyl.
(p) The compound, in which R⁴ is H.
(q) The compound, in which R⁵ is H, halogen, or lower alkyl, and in another embodiment, methyl or Cl.
(r) The compound formed from the combinations of two or more embodiments of (a) to (q) above.

Further, other embodiments of the compound represented by the formula (I) are shown below.
(1) The compound of the formula (I), in which R⁴ is -H.
(2) The compound as described in (1), in which R⁵ is -H, halogen, or lower alkyl.
(3) The compound as described in (2), in which R³ is halogen, lower alkyl, or cycloalkyl.
(4) The compound as described in (3), in which L¹ is lower alkylene.
(5) The compound as described in (4), in which L² is lower alkylene.
(6) The compound as described in (5), in which R¹ is -CO₂R⁰; or phenyl which is substituted with a group selected from the group consisting of -CO₂R⁰ and the groups represented by the following formula (III), the following formula (IX), the following formula (X), and the following formula (XIV): 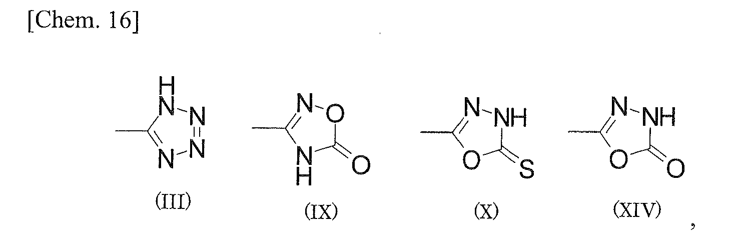 and which may be further substituted with 1 to 3 lower alkyl or halogen.
(7) The compound as described in (6), in which R² is -N(lower alkyl)₂; -N(R⁰)-cycloalkyl; -N(R⁰)-(aryl which may be substituted with 1 to 3 groups selected from the group consisting of halogen, lower alkyl, halogeno-lower alkyl, -OR⁰, and -O-halogeno-lower alkyl); or -N(R⁰)-(lower alkylene)-(aryl which may be substituted with 1 to 3 groups selected from the group consisting of halogen, lower alkyl, halogeno-lower alkyl, -OR⁰, and -O-halogeno-lower alkyl).
(8) The compound as described in (6), in which R² is a group described by the following formula (XVI): 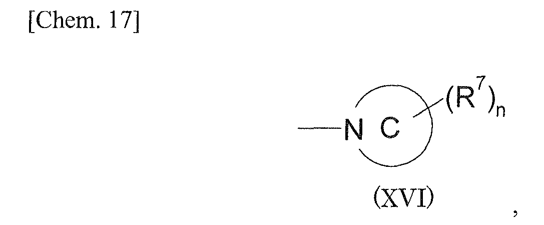 Ring C is a monocyclic 5- to 7-membered nitrogen-containing saturated hetero ring, and R⁷ is halogen, lower alkyl, lower alkenyl, halogeno-lower alkyl, -OR⁰, -O-halogeno-lower alkyl, or -(lower alkylene)-cycloalkyl.
(9) The compound as described in (6), in which R² is 2,3-dihydro-1H-indol-1-yl in which 1 to 3 groups selected from the group consisting of halogen, lower alkyl, lower alkenyl, halogeno-lower alkyl, -OR⁰, and -O-halogeno-lower alkyl may be substituted at the 4- to 7-positions.
(10) The compound of the formula (I), which is selected from the group consisting of:
   4-{2-[6-{[(2S)-2-butylpyrrolidin-1-yl]methyl}-3,5-dichloro-2-oxopyridin-1(2H)-yl]ethyl}benzoic acid,
   4-{2-[3,5-dichloro-6-{[(2R)-2-isobutylpyrrolidin-1-yl]methyl}-2-oxopyridin-1(2H)-yl]ethyl}benzoic acid,
   4-{2-[3,5-dichloro-2-oxo-6-{[(2S)-2-propylpyrrolidin-1-yl]methyllpyridin-1(2H)-yl]ethyl}benzoic acid,
   3,5-dichloro-6-{[(2S)-2-propylpyrrolidin-1-yl]methyl}-1-{2-[4-(1H-tetrazol-5-yl)phenyl]ethyl}pyridin-2(1H)-one,
   3,5-dichloro-1-{2-[4-(5-oxo-4,5-dihydro-1,3,4-oxadiazol-2-yl)phenyl]ethyl}-6-{[(2S)-2-propylpyrrolidin-1-yl]methyl}pyridin-2(1H)-one,
   4-{2-[3,5-dichloro-6-{[cyclopentyl(methyl)amino]methyl}-2-oxopyridin-1(2H)-yl]ethyl}benzoic acid,
   3,5-dichloro-1-{2-[4-(5-oxo-4,5-dihydro-1,3,4-oxadiazol-2-yl)phenyl]ethyl}-6-{[(2R)-2-isobutylpyrrolidin-1-yl]methyl}pyridin-2(1H)-one,
   4-{2-[5-cyclopropyl-6-{[(2R)-2-isobutylpyrrolidin-1-yl]methyl}-3-methyl-2-oxopyridin-1(2H)-yl]ethyl}benzoic acid,
   4-{2-[6-{[(2R)-2-isobutylpyrrolidin-1-yl]methyl}-3,5-dimethyl-2-oxopyridin-1(2H)-yl]ethyl}benzoic acid,
   1-{2-[4-(5-oxo-4,5-dihydro-1,3,4-oxadiazol-2-yl)phenyl]ethyl}-6-{[(2R)-2-isobutylpyrrolidin-1-yl]methyl }-3,5-dimethylpyridin-2(1H)-one,
   5-cyclopropyl-1-{2-[4-(5-oxo-4,5-dihydro-1,3,4-oxadiazol-2-yl)phenyl]ethyl}-6-{[(2R)-2-isobutylpyrrolidin-1-yl]methyl}-3-methylpyridin-2(1H)-one,
   4-2-[3,5-dichloro-2-oxo-6-({[3-(trifluoromethoxy)phenyl]amino}methyl)pyridin-1(2H)-yl]methyl}benzoic acid,
   4-{2-[3-chloro-5-cyclopropyl-2-oxo-6-{[(2S)-2-propylpyrrolidin-1-yl]methyl}pyridin-1(2H)-yl]ethyl}benzoic acid,
   4-{2-[3-chloro-5-cyclopropyl-6-{[(2R)-2-isobutylpyrrolidin-1-yl]methyl}-2-oxopyridin-1(2H)-yl]ethyl}benzoic acid,
   4-{2-[3-chloro-5-ethyl-6-{[(2R)-2-isobutylpyrrolidin-1-yl]methyl}-2-oxopyridin-1(2H)-yl]ethyl}benzoic acid,
   4-{2-[3,5-dichloro-6-{[methyl(3-methylphenyl)amino]methyl}-2-oxopyridin-1(2H)-yl]ethyl}benzoic acid,
   4-{2-[3,5-dichloro-6-({methyl[3-(trifluoromethyl)phenyl]amino}methyl)-2-oxopyridin-1(2H)-yl]methyl}benzoic acid,
   4-{2-[3,5-dichloro-6-{[(3-chlorophenyl)(methyl)amino]methyl}-2-oxopyridin-1(2H)-yl]ethyl}benzoic acid,
   4-{2-[3-chloro-5-cyclopropyl-6-{[methyl(3-methylphenyl)amino]methyl}-2-oxopyridin-1(2H)-yl]ethyl}benzoic acid,
   4-(2-{3,5-dichloro-6-[(6-ethyl-2,3-dihydro-1H-indol-1-yl)methyl]-2-oxopyridin-1(2H)-yl}ethyl)benzoic acid,
   4-{2-[3,5-dichloro-2-oxo-6-{[6-(trifluoromethoxy)-2,3-dihydro-1H-indol-1-yl]methyl}pyridin-1(2H)-yl]ethyl}benzoic acid,
   4-(2-{3,5-dichloro-6-[(6-ethoxy-2,3-dihydro-1H-indol-1-yl)methyl]-2-oxopyridin-1(2H)-yl}ethyl)benzoic acid,
   4-(2-{3,5-dichloro-6-[(6-fluoro-2,3-dihydro-1H-indol-1-yl)methyl]-2-oxopyridin-1(2H)-yl}ethyl)benzoic acid,
   4-(2-{3,5-dichloro-6-[(6-fluoro-7-methyl-2,3-dihydro-1H-indol-1-yl)methyl]-2-oxopyridin-1(2H)-yl}ethyl)benzoic acid,
   4-(2-{3,5-dichloro-6-[(7-ethyl-2,3-dihydro-1H-indol-1-yl)methyl]-2-oxopyridin-1(2H)-yl}ethyl)benzoic acid, and
   4-(2-{3,5-dichloro-6-[(7-methyl-2,3-dihydro-1H-indol-1-yl)methyl]-2-oxopyridin-1(2H)-yl}ethyl)benzoic acid,
or a pharmaceutically acceptable salt thereof.

(11) A compound of the formula (I^{a}) or a pharmaceutically acceptable salt thereof: 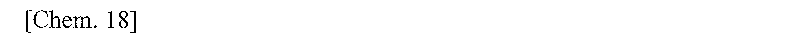 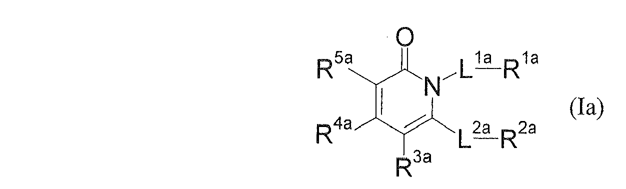 [the symbols in the formula represent the following meanings:
   L^{1a}: lower alkylene or lower alkenylene, which may each be substituted,
   L^{2a}: lower alkylene, lower alkenylene, -C(O)-, -(lower alkylene)-C(O)-, or -(lower alkenylene)-C(O)-, in which lower alkylene and lower alkenylene in L^{2a} may each be substituted,
   R^{1a}: R^{6a} or a group represented by the following formula (II^{a}): 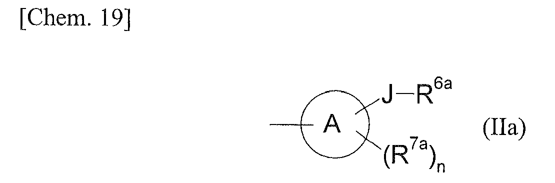
   Ring A: aryl or heteroaryl,
   R^{6a}_{:} -CO₂R⁰, -C(O)N(H)S(O)₂-R^{8a}, -C(O)N(H)S(O)₂N(R⁰)-R^{8a}, -N(R⁰)C(O)N(H)S(O)₂-R^{8a}, or a group represented by any one of the following formulae (III) to (XIII): 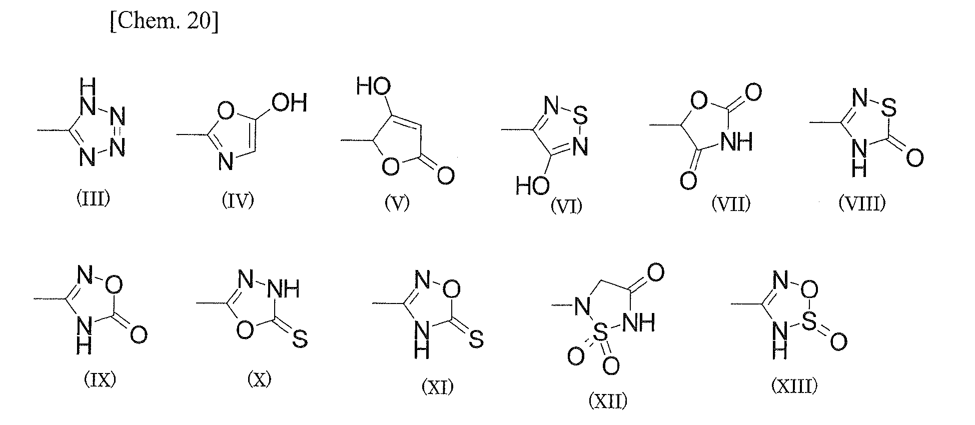
   R⁰: the same as or different from each other, each representing H or lower alkyl,
   R^{8a}: lower alkyl, halogeno-lower alkyl, or -(lower alkylene)-OR⁰,
   J: a single bond or lower alkylene,
   R^{7a}: the same as or different from each other, each representing lower alkyl, halogen, halogeno-lower alkyl, -OR⁰, -O-(halogeno-lower alkyl), -O-(cycloalkyl), -O-(lower alkylene)-OR⁰, -N(R⁰)₂, -(lower alkylene)-OH, -(lower alkylene)-OR⁰, -(lower alkylene)-N(R⁰)₂, -CO-R⁰, -S(O)₂-R⁰, -CO₂R⁰, -C(O)N(R⁰)₂, aryl, or a hetero ring group, in which aryl and the hetero ring group in R^{7a} may be substituted,
   n: an integer of 0 to 3,
   R^{2a}: a group represented by any one of the following formulae (XV^{a}) to (XVII^{a}): 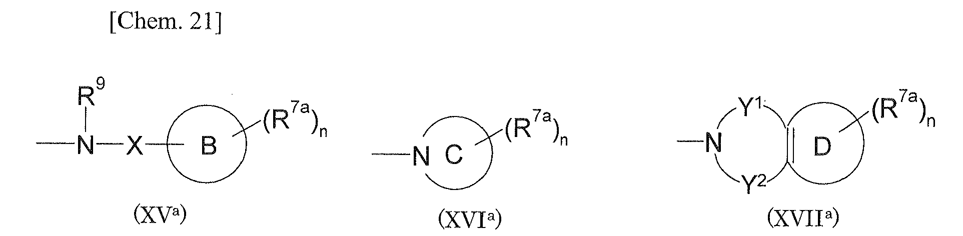
   Ring B: cycloalkyl, aryl, or a hetero ring,
   Ring C: a nitrogen-containing saturated hetero ring,
   Ring D: aryl or heteroaryl,
   X: a single bond, lower alkylene, -C(O)-, -C(O)-(lower alkylene)-, or -(lower alkylene)-O-,
   R⁹: H, lower alkyl, -C(O)R⁰, or aryl, in which aryl in R⁹ may be substituted,
   Y¹ and Y²: the same as or different from each other, each representing a single bond, -[C(R¹⁰)(R¹¹)]ₛ-, -[C(R¹⁰)(R¹¹)]ₛ-Q-, -Q-[C(R¹⁰)(R¹¹)]ₛ-, or -[C(R¹⁰)(R¹¹)]ₛ-Q-[C(R¹⁰)(R¹¹)]ₜ-,
   R¹⁰ and R¹¹: the same as or different from each other, each representing H, lower alkyl, halogen, halogeno-lower alkyl, -OR⁰, -N(R⁰)₂, -(lower alkylene)-OH, -(lower alkylene)-OR⁰, -(lower alkylene)-N(R⁰)₂, or a hetero ring group, or R¹⁰ and R¹¹ on the same carbon atom may be combined to form oxo,
   Q: O, S(O)p, or N(R¹²),
   R¹²: H, lower alkyl, -C(O)R⁰, or -S(O)₂-(lower alkyl).
   s and t: the same as or different from each other, each representing an integer of 1 to 4,
   p: an integer of 0 to 2, and
   R^{3a}, R^{4a}, and R^{5a}_{:} the same as or different from each other, each representing H, halogen, -CN, lower alkyl, halogeno-lower alkyl, -OR⁰, -O-(halogeno-lower alkyl), -CO₂R⁰, -C(O)N(R⁰)₂, or cycloalkyl].

The compound of the formula (I) may in some cases exist in the form of other tautomers or geometrical isomers, depending on the kinds of the substituents. In the present specification, the compound may be described in only one form of the isomers, but the present invention includes such isomers, isolated forms of the isomers, or a mixture thereof.
Furthermore, the compound of the formula (I) may have asymmetric carbon atoms or axial asymmetries in some cases, and correspondingly, it may exist in the form of optical isomers such as an (R)-form, an (S)-form, and the like. The present invention includes all of the mixture and the isolated form of these optical isomers.
In addition, the pharmaceutically acceptable prodrugs of the compound of the formula (I) are also included in the present invention. The pharmaceutically acceptable prodrug refers to a compound having a group which can be converted into an amino group, OH, CO₂H, or the like, of the present invention, by solvolysis or under a physiological condition. Examples of the group for forming a prodrug include those as described in Prog. Med., 5, 2157-2161 (1985) or "Pharmaceutical Research and Development" (Hirokawa Publishing Company, 1990), vol. 7, Drug Design, 163-198.

Furthermore, the compound of the formula (I) may form an acid addition salt or salt with a base, depending on the kind of the substituents, and the salt is included in the present invention, as long as it is a pharmaceutically acceptable salt. Specifically, examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, glutamic acid, and the like, and salts with inorganic bases such as sodium, potassium, magnesium, calcium, aluminum, and the like, and organic bases such as methylamine, ethylamine, ethanolamine, lysine, ornithine, and the like, ammonium salts, and others.
Furthermore, the present invention also includes various hydrates or solvates, and polymorphic crystal substances of the compound of the formula (I) and a pharmaceutically acceptable salt thereof. Furthermore, the present invention also includes the compounds labeled with various radioactive or non-radioactive isotopes.

### (Production Processes)

The compound of the formula (I) and a pharmaceutically acceptable salt thereof can be prepared by applying various known synthesis methods, using the characteristics based on their basic skeletons or the kinds of the substituents. At this time, depending on the types of the functional groups, it is in some cases effective from the viewpoint of the preparation techniques to substitute the functional group with an appropriate protecting group (a group which is capable of being easily converted into the functional group), during the steps from starting materials to intermediates. Examples of such a functional group include an amino group, a hydroxyl group, a carboxyl group, and the like, and examples of the protecting group thereof include those as described in "Protective Groups in Organic Synthesis (4th edition, 2007)", edited by Greene and Wuts, and the like, which may be appropriately selected and used depending on the reaction conditions. In these methods, a desired compound can be obtained by introducing the protecting group to carry out the reaction, and then, if desired, removing the protecting group.
In addition, the prodrug of the compound of the formula (I) can be prepared by introducing a specific group during the steps from starting materials to intermediates, in the same manner as for the above protecting groups, or by carrying out the reaction using the compound of the formula (I) obtained. The reaction can be carried out by applying a method known by a person skilled in the art, such as general esterification, amidation, dehydration, and the like.
Hereinbelow, the representative production processes for the compound of the formula (I) will be explained. Each of the production processes may also be carried out with reference to the References appended in the present description. Further, the production processes of the present invention are not limited to the examples as shown below.

### (Production Process 1)

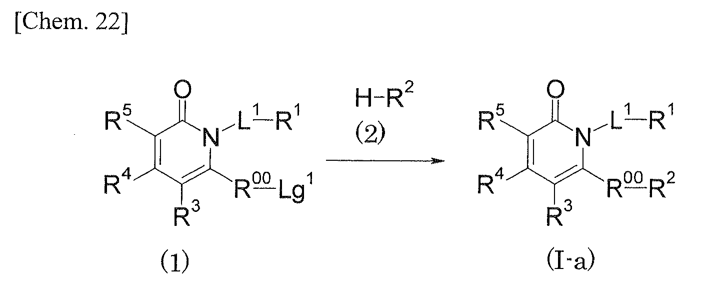

(In the formula, Lg¹ means a leaving group, for example Br. Further, R⁰⁰ means lower alkylene. The same shall apply hereinafter.)
The compound (I-a) of the present invention can be prepared by the reaction of a compound (1) with a compound (2). Herein, examples of Lg¹ include halogen, a methanesulfonyloxy group, a p-toluenesulfonlyoxy group, and the like.
In this reaction, the compound (1) and the compound (2) are used in an equivalent amount or in an excessive amount of either thereof, and the mixture thereof is stirred from under cooling to under heating and reflux, preferably at 0°C to 80°C, usually for 0.1 hour to 5 days, in a solvent which is inert to the reaction or without a solvent. Examples of the solvent as used herein are not particularly limited, but include aromatic hydrocarbons such as benzene, toluene, xylene, and the like, ethers such as diethyl ether, tetrahydrofuran (THF), dioxane, dimethoxyethane (DME), and the like, halogenated hydrocarbons such as dichloromethane (DCM), 1,2-dichloroethane (DCE), chloroform, and the like, N,N-dimethylformamide (DMF), dimethylsulfoxide (DMSO), N-methyl-2-pyrrolidinone (NMP), ethyl acetate, acetonitrile, acetone, methylethylketone, and a mixture thereof. It may be advantageous in some cases for the smooth progress of the reaction to carry out the reaction in the presence of an organic base such as triethylamine (TEA), N,N-diisopropylethylamine (DIPEA), or N-methylmorpholine (NMM), and the like, or an inorganic base such as potassium carbonate, sodium carbonate, potassium hydroxide, and the like. In addition, it may be advantageous in some cases to carry out the reaction in the presence of sodium iodide or the like, if necessary.
Furthermore, the present reaction can also be carried out using a microwave device.

### (Production Process 2)

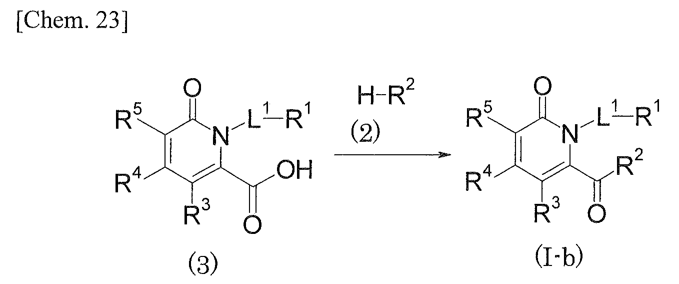

The compound (I-b) of the present invention can be prepared by the reaction of a compound (3) with the compound (2).
In the present reaction, the compound (3) and the compound (2) are used in an equivalent amount or in an excessive amount of either thereof, and the mixture thereof is stirred from under cooling to under heating, preferably at -20°C to 60°C, usually for 0.1 hour to 5 days in a solvent which is inert to the reaction in the presence of a condensing agent. Examples of the solvent as used herein are not particularly limited, but include aromatic hydrocarbons such as benzene, toluene, xylene, and the like, halogenated hydrocarbons such as DCM, DCE, chloroform, and the like, ethers such as diethyl ether, THF, dioxane, DME, and the like, DMF, DMSO, ethyl acetate, acetonitrile, or water, and a mixture thereof. Examples of the condensing agent include 1-(3-dimethylaminopropyl)-3-ethylcarbodiimide (WSC), dicyclohexylcarbodiimide (DCC), 1,1'-carbonyldiimidazole (CDI), diphenylphosphoryl azide (DPPA), and phosphorous oxychloride, but are not limited to these. It may be preferable in some cases for the reaction to use an additive (for example, 1-hydroxybenzotriazole (HOBt)). It may be advantageous in some cases for the smooth progress of the reaction to carry out the reaction in the presence of an organic base such as TEA, DIPEA, NMM, and the like, or an inorganic base such as potassium carbonate, sodium carbonate, potassium hydroxide, and the like.
Furthermore, a method in which the compound (3) is modified into a reactive derivative thereof, and then reacted with the compound (2) can also be used. Examples of the reactive derivative of the compound (3) include acid halides obtained by the reaction with a halogenating agent such as phosphorus oxychloride, thionyl chloride, and the like, mixed acid anhydrides obtained by the reaction with isobutyl chloroformate or the like, various active esters obtained by the condensation with CDI, HOBt, or the like, and others. The reaction of such a reactive derivative with the compound (2) can be carried out from under cooling to under heating, preferably at -20°C to 60°C in a solvent which is inert to the reaction, such as halogenated hydrocarbons, aromatic hydrocarbons, ethers, and the like.
Furthermore, the present reaction can also be carried out using a microwave device.

### (Production Process 3)

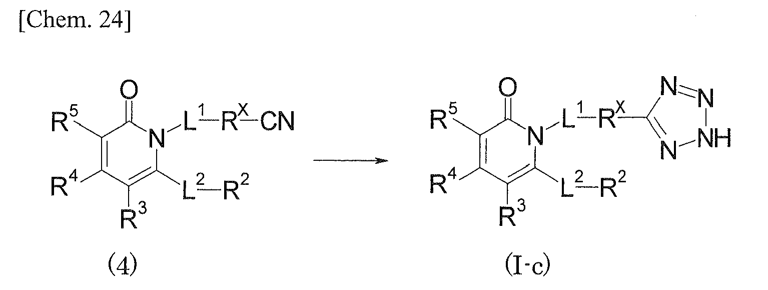

(In the formula, R^{x} means a single bond or 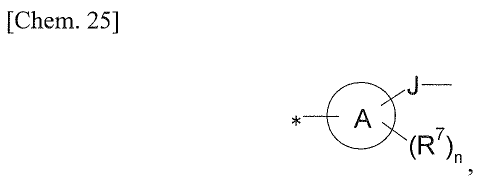 in which * means a bond to L¹. The same shall apply hereinafter.)

The compound (I-c) of the present invention can be prepared by tetrazolylation of a compound (4).
The present reaction can be carried out from at room temperature to under heating, using the compound (4) and an azide compound such as sodium azide, ammonium azide, tri-n-butyltin azide, and the like in an equivalent amount or in an excessive amount of either thereof, in a solvent such as ethers, aromatic hydrocarbons, DMF, and the like. According to the compounds, it may be advantageous in some cases for the progress of the reaction to carry out the reaction in the presence of ammonium chloride, trimethylamine hydrochloride, or the like.

### (Production Process 4)

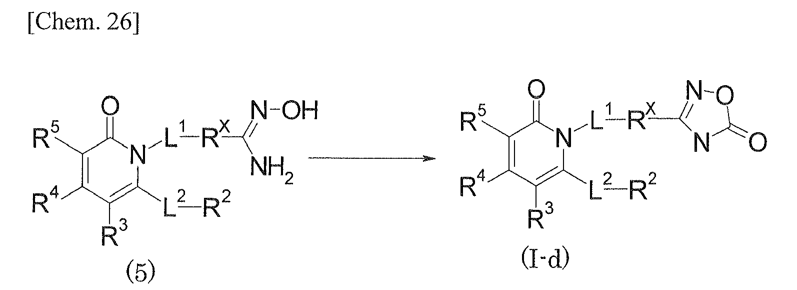

The compound (I-d) of the present invention can be prepared by reacting a compound (5) with a carbonyl compound such as CDI, phosgene, ethyl chloroformate, and the like.
The present reaction can be carried out from at room temperature to under heating, using the compound (5) and the carbonyl compound in an equivalent amount or in an excessive amount of either thereof, in a solvent such as ethers, aromatic hydrocarbons, halogenated hydrocarbons, DMF, and the like. According to the compounds, it may be advantageous in some cases for the progress of the reaction to carry out the reaction in the presence of an organic base such as triethylamine, N,N-diisopropylethylamine, pyridine, 1,8-diazabicyclo[5,4,0]undec-7-ene, and the like, or an inorganic base such as sodium carbonate, potassium carbonate, and the like.

### (Production Process 5)

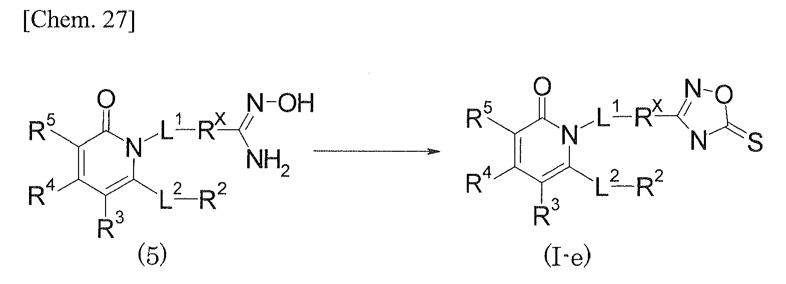

The compound (I-e) of the present invention can be prepared by reacting the compound (5) with a thiocarbonyl compound such as 1,1'-carbonothiobis(1H-imidazole) and the like.
The present reaction can be carried out from at room temperature to under heating, using the compound (5) and the thiocarbonyl compound in an equivalent amount or in an excessive amount of either thereof, in a solvent such as ethers, aromatic hydrocarbons, halogenated hydrocarbons, DMF, and the like. According to the compounds, it may be advantageous in some cases for the progress of the reaction to carry out the reaction in the presence of an organic base such as triethylamine, N,N-diisopropylethylamine, pyridine, 1,8-diazabicyclo[5,4,0]undec-7-ene, and the like, or an inorganic base such as sodium carbonate, potassium carbonate, and the like.

### (Production Process 6)

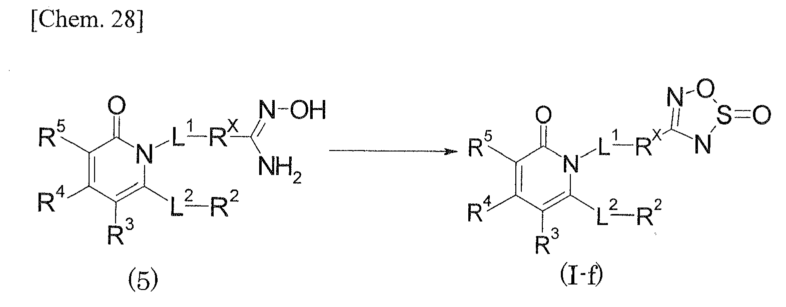

The compound (I-f) of the present invention can be prepared by reacting the compound (5) with thionyl chloride.
The present reaction can be carried out from at room temperature to under heating, using the compound (5) and thionyl chloride in an equivalent amount or in an excessive amount of either thereof, in a solvent such as ethers, aromatic hydrocarbons, halogenated hydrocarbons, DMF, and the like. According to the compounds, it may be advantageous in some cases for the progress of the reaction to carry out the reaction in the presence of an organic base such as triethylamine, N,N-diisopropylethylamine, pyridine, 1,8-diazabicyclo[5,4,0]undec-7-ene, and the like, or an inorganic base such as sodium carbonate, potassium carbonate, and the like.

### (Production Process 7)

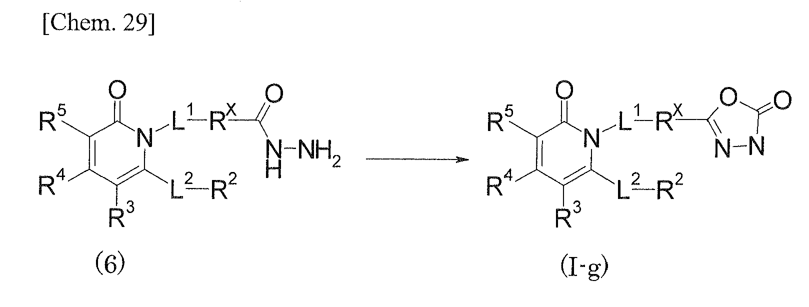

The compound (I-g) of the present invention can be prepared by reacting a compound (6) with a carbonyl compound such as CDI, phosgene, ethyl chloroformate, and the like.
The present reaction can be carried out from at room temperature to under heating, using the compound (6) and the carbonyl compound in an equivalent amount or in an excessive amount of either thereof, in a solvent such as ethers, aromatic hydrocarbons, halogenated hydrocarbons, DMF, and the like. According to the compounds, it may be advantageous in some cases for the progress of the reaction to carry out the reaction in the presence of an organic base such as triethylamine, N,N-diisopropylethylamine, pyridine, 1,8-diazabicyclo[5,4,0]undec-7-ene, and the like, or an inorganic base such as sodium carbonate, potassium carbonate, and the like.

References regarding the reactions of Production Processes 1 to 7 include the following.
"Organic Functional Group Preparations", written by S. R. Sandler and W. Karo, 2nd Edition, Vol. 1, Academic Press Inc., 1991, and "Jikken Kagaku Koza (Courses in Experimental Chemistry) (5th Edition)", edited by The Chemical Society of Japan, Vol. 14 (2005) (Maruzen)

Moreover, several compounds that can be obtained from the formula (I) can be prepared from the compound obtained as described above, by any combination of known processes that can be usually employed by a person skilled in the art, such as alkylation, amidation, a substitution reaction, oxidation, reduction, hydrolysis, and the like.
For the compounds of the formulae (I-a) and (I-b), obtained by Production Processes 1 to 2 above, when R¹ is a carboxylic ester, the compound of the formula (I) in which R¹ is -CO₂H can be prepared by carrying out a hydrolysis reaction.

The starting materials used in the preparation of the compound of the formula (I) can be prepared, for example, using the methods for starting material synthesis below, the methods described in Production Examples below, known methods, or methods apparent to a person skilled in the art, or modified methods thereof.

### (Starting Material Synthesis 1)

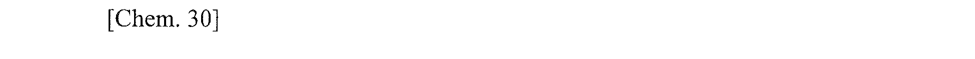

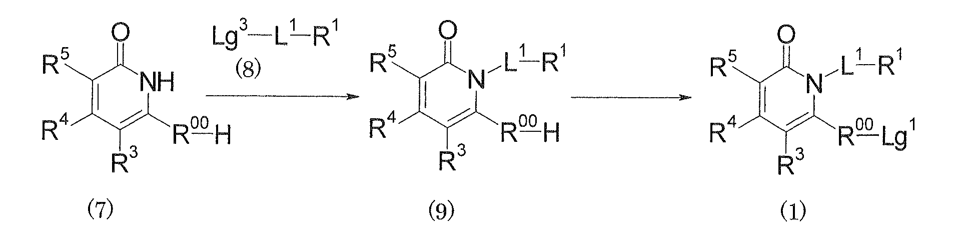

(In the formula, Lg³ represents a leaving group).
A starting compound (1) can be prepared by the introduction of a leaving group, Lg¹, for example, by bromination, to a compound (9) obtained by an N-alkylation reaction of a compound (7) with a compound (8).
The N-alkylation reaction can be carried out, for example, in the same manner as in Production Process 1.
The bromination can be carried out, for example, by the reaction with N-bromosuccinimide in the presence of a radical initiator such as 2,2'-azobis(isobutyronitrile), benzoyl peroxide, and the like, or by the reaction with N-bromosuccinimide or bromine from at room temperature to under heating in a solvent such as acetic acid, DMF, and the like,.

### (Starting Material Synthesis 2)

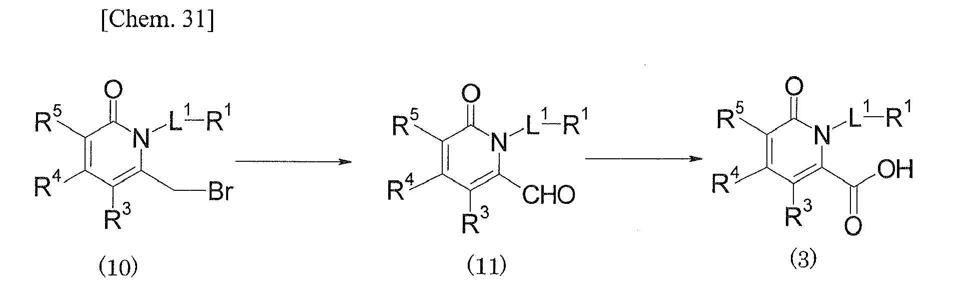

A starting compound (3) can be prepared by converting a compound (10) to a compound (11), and further, carrying out an oxidation reaction.
The conversion of the compound (10) to the compound (11) can be carried out, for example, by carrying out oxidation using trimethylamine-N-oxide, or by carrying out substitution with an acetoxy group, hydrolysis, and then oxidation.

### (Starting Material Synthesis 3)

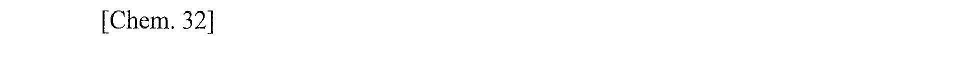

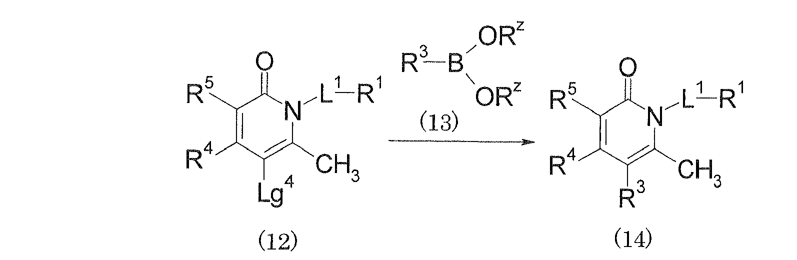

(In the formula, Lg⁴ represents a leaving group such as halogen, a trifluoromethanesulfonyloxy group, and the like, and R^{z} represents -H or lower alkyl, or two R^{z}s are combined with each other to represent lower alkylene. The same shall apply hereinafter.)
A compound (14) can be prepared by carrying out a coupling reaction of a compound (12) with a compound (13) in the presence of a base and a palladium catalyst. As the base, for example, sodium carbonate, potassium carbonate, cesium carbonate, or tripotassium phosphate is suitably used, and as the palladium catalyst, for example, tetrakistriphenylphosphine palladium or a catalyst prepared from palladium acetate with tricyclohexylphosphine or dicyclohexyl(2',6'-dimethoxybiphenyl-2-yl)phosphine is suitably used.

### (Starting Material Synthesis 4)

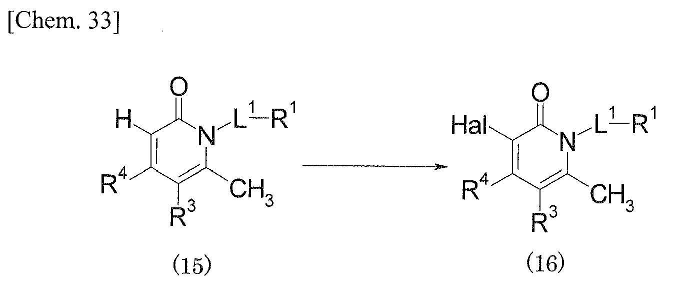

(In the formula, Hal represents halogen. The same shall apply hereinafter.)
A compound (16) can be prepared by halogenation of a compound (15).
As the halogenating agent, N-chlorosuccinimide, N-bromosuccinimide, N-iodosuccinimide, trichloroisocyanuric acid, bromine, iodine, or the like can be used.

Furthermore, the compound (4) can be prepared in the same manner as in Production Processes 1 and 2 above.
The compound (5) can be prepared by the reaction of the compound (4) with hydroxyamine.
The compound (6) can be prepared by amidation of the compound of the present invention having a corresponding carboxylic acid with hydrazine.

The compound of the formula (I) is isolated and purified as its free compound, pharmaceutically acceptable salts, hydrates, solvates, or polymorphic crystal substances thereof. The pharmaceutically acceptable salt of the compound of the formula (I) can also be prepared in accordance with a conventional method for a salt formation reaction.
Isolation and purification are carried out by employing general chemical operations such as extraction, fractional crystallization, various types of fractional chromatography, and the like.
Various isomers can be separated by selecting an appropriate starting compound or by making use of the difference in the physicochemical properties between isomers. For example, the optical isomer can be lead into a stereochemically pure isomer by means of general optical resolution methods (for example, fractional crystallization for inducing diastereomer salts with optically active bases or acids, chromatography using a chiral column, etc., and the like). In addition, the isomers can also be prepared from an appropriate optically active starting compound.

The pharmacological activity of the compound of the formula (I) was confirmed by the following test.

### Test Example 1 Evaluation Test on EP4 Receptor Affinity in Rat

### (1) Cell culture and transfection

A rat EP4 receptor cDNA was subcloned into an expression vector (pCDNA3.1-V5-His-topo, manufactured by Invitrogen Corporation) to prepare a rat EP4 expression vector. HEK293 cells were cultured in a collagen type 1-treated 15 cm dish (manufactured by Asahi Techno Glass Co,. Ltd.) to a confluence of 70%. An Opti-MEM culture medium at 1.2 mL/dish and a transfection reagent (Lipofectamine 2000, manufactured by Invitrogen Corporation) at 60 µL/dish were mixed, followed by being left to stand at room temperature for 5 minutes. Then, the rat EP4 receptor expression vector at 15 µg/dish was added thereto, followed by being left to stand at room temperature for 30 minutes. The liquid mixture of the transfection reagent was added into the dish, followed by culturing for 20 to 24 hours. The cell culture was carried out in a CO₂ incubator (37°C, 5% CO₂).

### (2) Preparation of Membrane Fraction

The culture medium was removed by suction, 10 mL of cooled PBS was added thereto per 15 cm dish, and the cells were scraped using a cell scraper. After washed with cooled PBS (1,200 rpm, 4°C, 5 minutes), suspended in 6 mL/dish of cooled 20 mM Tris-HCl (pH 7.4; manufactured by Nacalai Tesque Inc., 5 mM EDTA included) and homogenized using a Polytron, the homogenate was centrifuged (26,000 rpm, 20 minutes, 4°C). The obtained precipitate was resuspended in cooled 20 mM Tris-HCl and homogenized again using a Polytron, and the homogenate was centrifuged (26,000 rpm, 20 minutes, 4°C). The obtained precipitate was resuspended in 50 mM HEPES (pH 7.5; manufactured by Dojindo Laboratories) at 1 mL per dish, homogenized using a Polytron, and freeze-stored at -80°C as a membrane fraction. At this time, a part thereof was used for the measurement of the protein concentration. Measurement of the protein concentration was carried out using a Protein assay stain (manufactured by Bio-Rad Laboratories) in accordance with a standard Protocol as appended in duplicate.

### (3) Receptor Binding Test

[³H]PGE2 50 µL (final concentration 0.3 nM; manufactured by Perkin Elmer Co., Ltd.), 100 µL (20 µg/well) of the membrane fraction prepared from the rat EP4 expression cell, and 50 µL of a test compound were mixed in a 96-well microplate (manufactured by Sumitomo Bakelite Co., Ltd.), incubated at room temperature for 1 hour, then filtered by suction on a microplate (UniFilter-96 GF/B, manufactured by Perkin Elmer Co., Ltd.) using a cell harvester (FilterMate Harvester, manufactured by Perkin Elmer Co., Ltd.), and washed three times with 300 µL/well of a cooled assay buffer (50 mM HEPES, 10 mM MgCl₂). Dilution of [³H]PGE2 and the membrane fraction was carried out using the assay buffer, and dilution of the test compound and the unlabeled PGE2 was carried out using dimethyl sulfoxide and the assay buffer. The UniFilter-96 GF/B was treated by preliminarily washing twice with 200 µL/well of the cooled assay buffer. The UniFilter-96 GF/B after filtration was dried in a dryer overnight, 50 µL/well of a liquid scintillation cocktail (MicroScint20, manufactured by Perkin Elmer Co., Ltd.) was added thereto, and the radioactivity was then measured using a liquid scintillation counter for a microplate (TopCount, manufactured by Perkin Elmer Co., Ltd.). For measurement of the non-specific binding, an unlabeled PGE2 (final concentration 1 µM; manufactured by Cayman Chemical Company) was added. All of the measurements were carried out in duplicate, and the specific binding amount was determined by subtracting the non-specific binding amount from the total binding amount.
According to the test method as described above, the rat EP4 receptor affinity (Ki) of the compound of the formula (I) was measured. The Ki values of the representative Example Compounds of the present invention are shown below. In this connection, Ex means Example Compound number.

**[Table 1]**

| Ex | Ki (nM) |
|---|---|
| 5 | 4.7 |
| 6 | 11 |
| 9 | 11 |
| 10 | 3.2 |
| 13 | 24 |
| 15 | 3.6 |
| 16 | 13 |
| 17 | 11 |
| 18 | 4.4 |
| 19 | 5.4 |
| 27 | 25 |
| 42 | 0.52 |
| 91 | 9.8 |
| 97 | 11 |
| 99 | 25 |
| 106 | 28 |
| 107 | 10 |
| 122 | 16 |
| 123 | 21 |
| 124 | 6.2 |
| 125 | 29 |
| 130 | 4.1 |
| 138 | 7.7 |
| 139 | 12 |
| 143 | 11 |
| 145 | 19 |
| 146 | 39 |
| 149 | 9.5 |
| 150 | 15 |
| 152 | 5.1 |
| 153 | 4.9 |
| 158 | 8.6 |
| 159 | 3.3 |
| 160 | 11 |
| 208 | 2.9 |
| 345 | 2.2 |
| 346 | 2.8 |

### Test Example 2 EP4 Receptor Agonistic Action in Rat

The agonistic activity was evaluated by the cAMP increasing action in the rat EP4 receptor expression cells. The rat EP4 receptor expression vector was introduced into CHO-K1 cells (American Type Culture Collection) to prepare a rat EP4 receptor stable expression cell line. These cells were seeded onto a 96-well microplate at 2×10⁴ cell/well, and used for the experiment the next day. The culture medium of each well was removed by suction, 100 µL/well of an assay culture medium (2 µM indomethacin, 0.1% bovine serum albumin-containing α-MEM) was added thereto, and incubated at 37°C for 1 hour. The culture medium was removed again by suction, and replaced with 100 µL/well of an assay culture medium comprising a test compound and 1 mM IBMX (3-isobutyl-1-methylxanthine). After incubating at 37°C for 30 minutes, the culture medium was removed by suction, 100 µL/well of a cell lysate (0.2% Triton-X100-containing phosphate buffer physiological saline) was put thereinto, and the plate was shaken for 10 minutes. Using a cAMP femto 2 kit (manufactured by Cis Bio International), the cAMP concentration in the cell lysate was measured. Further, the cAMP increasing action (%) was calculated with the cAMP increasing action by 1 pM PGE₂ was taken as 100%.
The cAMP increasing actions (%) of the representative Example compounds of the present invention at 10 µM are shown in Table 2.

**[Table 2]**

| Ex | cAMP increasing action (%) at 10 µM |
|---|---|
| 9 | 65 |
| 17 | 58 |
| 42 | 78 |
| 107 | 76 |
| 160 | 79 |
| 208 | 83 |

### Test Example 3 Evaluation on Selectivity: Rat Prostaglandin EP Receptor Agonistic Action/Antagonistic Action

### (1) Rat EP1 and Rat EP3 Receptor Agonistic Action/Antagonistic Action

Using rat EP1 or rat EP3β receptor stable expression cells, the intracellular Ca²⁺ concentration was measured using a fluorescent imaging plate reader (FLIPR manufactured by Molecular Devices Corporation). The agonistic activity was evaluated by the intracellular Ca²⁺ increasing action of the test compound, and the antagonistic activity was evaluated by the inhibiting action of the test compound on the intracellular Ca²⁺ increasing action by PGE2.
The cDNA of the rat EP1 or EP3β receptor was subcloned into an expression vector (pCDNA3.1-V5-His-topo, manufactured by Invitrogen Corporation). This expression vector was introduced into HEK293 cells (American Type Culture Collection) to prepare a rat EP1 or EP3β receptor stable expression cell line. These cells were seeded onto a 96-well poly-D-lysin treated black wall clear bottom plate (manufactured by Becton, Dickinson and Company) at 2 to 3×10⁴ cell/well, and used for the experiment the next day. The measurement of the intracellular Ca²⁺ concentration was carried out by an FLIPR calcium 3 assay kit (manufactured by Molecular Devices Corporation). The culture medium of each well was removed by suction and replaced with a loading buffer (Hank's balanced salt solution containing 20 mM HEPES-NaOH (pH 7.4), 2.5 mM Probenecid, 0.1% bovine serum albumin, and a color), followed by incubating at room temperature for 3 hours and loading a color. For the evaluation of the agonistic action, the change in the intracellular Ca²⁺ concentration was determined from the difference in the maximum value of the intracellular Ca²⁺ concentration after the addition of the test compound and the value before the addition of the test compound. For the evaluation of the antagonistic action, after incubating the test compound for 5 minutes, PGE2 was added thereto to determine the change in the intracellular Ca²⁺ concentration by PGE2.
The measurement results of the representative Example compounds of the present invention are shown in Table 3. Further, NE means that the action has no significance.

**[Table 3]**

| Ex | Rat EP1 | | RatEP3 | |
|---|---|---|---|---|
| | Agonistic Action 10 µM | Antagonistic Action IC₅₀(nM) | Agonistic Action 10 µM | Antagonistic Action IC₅₀ (nM) |
| 9 | NE | 1000 | NE | >10000 |
| 17 | NE | 2100 | NE | >10000 |
| 107 | NE | >10000 | NE | >10000 |
| 160 | NE | 2700 | NE | 10000 |

### (2) Rat EP2 Receptor Agonistic Action/Antagonistic Action

For a rat EP2 receptor, stable expression cells were used to carry out a cAMP assay. The agonistic activity was evaluated by the cAMP increasing action by the test compound, and the antagonistic activity was evaluated by the inhibiting action of the test compound on the cAMP increasing action by PGE2.
The rat EP2 receptor cDNA was subcloned into an expression vector (pCDNA3.1-V5-His-topo, manufactured by Invitrogen Corporation). This expression vector was introduced into CHO-K1 cells (American Type Culture Collection) to prepare a rat EP2 receptor stable expression cell line. These cells were seeded onto a 96-well microplate at 0.5×10⁴ cell/well, and used for the experiment the next day. The culture medium of each well was removed by suction, 100 µL/well of an assay culture medium (α-MEM containing 2 µM indomethacin and 0.1% bovine serum albumin) was added thereto, and incubated at 37°C for 1 hour. The culture medium was removed again by suction, and replaced with 100 µL/well of an assay culture medium containing a test compound and 1 mM IBMX. After incubating at 37°C for 30 minutes, the culture medium was removed by suction, 100 µL/well of a cell lysate (0.2% Triton-X100-containing phosphate buffer physiological saline) was added thereto, and the plate was shaken for 10 minutes. Using a cAMP femto 2 kit (manufactured by Cis Bio International), the cAMP concentration in the cell lysate was measured. In this connection, the cAMP increasing action (%) was calculated with the cAMP increasing action by 1 µM PGE₂ was taken as 100%.

The results of the representative Example compounds of the present invention are shown in Table 4.

**[Table 4]**

| Ex | Rat EP2 | |
|---|---|---|
| | Agonistic Action 10 *µ*M | Antagonistic Action IC50 (nM) |
| 9 | NE | NE |
| 17 | 14% | NE |
| 107 | NE | NE |
| 160 | 19% | NE |

### Text Example 4 Inhibitory Action on LPS-Induced TNF-α Production in THP-1 Cells

Human monocytic cell line THP-1 cells were suspended in an assay medium (PRMI-1640 containing 10% fetal bovine serum, 100 unit/mL penicillin G sodium, and 100 µg/mL streptomycin sulfate), and seeded onto a 96-well plate at 1×10⁵ cells/well. 50 µL/well of an assay medium containing a test compound was added thereto, followed by incubation at 37°C for 30 minutes. Further, 50 µL/well of an assay medium containing 1 to 5 µg/mL of LPS was added thereto, and the TNF-α concentration in the assay medium of each well after 3 hours was measured. The measurement was carried out by means of a standard ELISA method. A 96-well plate which had been coated overnight with an anti-human TNF monoclonal antibody (clone: MAb1) (manufactured by Becton, Dickinson and Company) as a capture antibody was washed with a wash buffer (PBS containing 0.05% Tween-20), and PBS containing 10% fetal bovine serum was incubated at room temperature for 1 hour to perform blocking. After washing with a wash buffer, 100 µL/well of the assay medium to be measured was incubated at 4°C overnight. As the standard material to be measured, a recombinant human TNF (manufactured by Becton, Dickinson and Company) was used. After washing with a wash buffer, 100 µL of a biotinylated anti-human TNF monoclonal antibody (clone: MAb 11) as a detection antibody was treated at room temperature for 1 hour. After washing with a wash buffer, it was treated with 100 µL/well of an HRP-labeled Streptavidin (manufactured by Zymed Laboratories, Inc.) at room temperature for 30 minutes, and washed again. Treatment was conducted using 100 µL/well of a TMB (3,3',5,5'-tetramethylbenzidine) substrate liquid at room temperature for 20 minutes in a dark room. A 2 M sulfuric acid was added thereto at 50 µL/well to stop the reaction, and an absorbance at 450 nm/570 nm was measured by means of a plate reader SPECRA max (manufactured by Molecular Devices Inc.).

The IC₅₀ values of the representative Example compounds of the present invention are shown in Table 5.

**[Table 5]**

| Ex | IC₅₀(nM) |
|---|---|
| 9 | 11 |
| 13 | 7.4 |
| 17 | 9.4 |
| 26 | 12 |
| 27 | 12 |
| 42 | 15 |
| 107 | 40 |
| 149 | 2.8 |
| 159 | 2.7 |
| 160 | 29 |
| 345 | 13 |

### Test Example 5 In Vivo TNF-α Production Inhibiting Action in Rat

LPS (10 µg/kg) was administered to caudal veins of SD male rats, and after 90 minutes from the administration, the heparin blood was collected from the abdominal vena cava to prepare a plasma. The test compound was orally administered 1 hour before the administration of LPS. The amount of TNF-α in the plasma was measured using a BD OptEIA rat TNF ELISA set (manufactured by Becton, Dickinson and Company) according to the attached method. The inhibitory rate by the test compound was determined from the amount of TNF-α in plasma in a control group (administered with a solvent).
The representative ED₅₀ values of the Example compounds of the present invention are shown in Table 6.

**[Table 6]**

| Ex | ED₅₀(mg/kg) |
|---|---|
| 9 | 4.3 |
| 17 | 0.15 |
| 42 | 0.076 |
| 107 | 1.0 |
| 160 | 2.3 |
| 345 | 1.9 |
| 346 | 2.0 |

### Test Example 6 Hindlimb Blood Flow Increasing Action in Anesthetized Rat

Wistar male rats were used. The test compound was orally administered, and after 2 hours, the hindlimb blood was measured using a laser blood flow imaging apparatus (PIM II, manufactured by Integral Corporation). At 20 minutes before the measurement, 60 mg/kg of pentobarbital was intraperitoneally administered to conduct anesthesia.
When the doses shown in Table 7 were orally administered, the representative Example compounds of the present invention showed the following blood flow increasing action, when the blood flow of the group administered with the solvent was set as 100%.

**[Table 7]**

| Ex | Dose (mg/kg) | Blood flow relative to the group administered with the solvent |
|---|---|---|
| 10 | 0.3 | 124% |
| 27 | 1 | 117% |
| 42 | 0.3 | 138% |
| 106 | 0.3 | 126% |
| 159 | 0.1 | 122% |
| 207 | 1 | 140% |

As a result of each of the tests above, it was confirmed that the compound of the formula (I) has an EP4 receptor agonistic action, and exhibits an anti-inflammatory action and a blood flow increasing action. Based on this, the compound can be used as an agent for treating peripheral arterial occlusive disease such as arteriosclerosis obliterans, thromboangiitis obliterans, and the like, various symptoms based on peripheral circulatory disorders (intermittent claudication/numbness in lower extremities due to lumbar spinal stenosis, Raynaud's syndrome, erectile dysfunction, and the like), inflammatory diseases such as ulcerative colitis, Crohn's disease, and the like, renal diseases such as nephritis, renal failure, and the like, bone diseases such as osteoporosis and the like, and eye diseases such as glaucoma, ocular hypertension, and the like.

A preparation comprising one or two or more kinds of the compound of the formula (I) or a pharmaceutically acceptable salt thereof as an active ingredient can be prepared in accordance with a generally used method, using a pharmaceutical carrier, excipient, or the like, that is usually used in the art.
The administration can be carried out in any mode of oral administration via tablets, pills, capsules, granules, powders, liquid preparations, or the like, or parenteral administration via injections such as intraarticular, intravenous, intramuscular, or others, suppositories, eye drops, eye ointments, percutaneous liquid preparations, ointments, percutaneous patches, transmucosal liquid preparations, transmucosal patches, inhalations, and the like.

Regarding the solid composition for oral administration according to the present invention, tablets, powders, granules, or the like are used. In such a solid composition, one or two or more kinds of active ingredients are mixed with at least one inert excipient, for example, lactose, mannitol, glucose, hydroxypropylcellulose, microcrystalline cellulose, starch, polyvinyl pyrrolidone, and/or magnesium aluminometasilicate, or the like. According to a conventional method, the composition may contain inert additives for example, a lubricant such as magnesium stearate, a disintegrator such as carboxymethylstarch sodium, a stabilizing agent, and a solubilizing aid. As occasion demands, the tablets or the pills may be coated with a sugar coating, or a film of a gastric or enteric coating agent.
The liquid composition for oral administration includes pharmaceutically acceptable emulsions, soluble liquid preparations, suspensions, syrups, elixirs, or the like, and contains a generally used inert diluent such as purified water or ethanol. In addition to the inert diluent, this liquid composition may contain an adjuvant such as a solubilizing agent, a moistening agent, and a suspending agent, a sweetener, a flavor, an aroma, and an antiseptic.
Injections for parenteral administration contain sterile aqueous or non-aqueous soluble liquid preparations, suspensions and emulsions. The aqueous solvent includes, for example, distilled water for injection or physiological saline. Examples of the non-aqueous solvent include propylene glycol, polyethylene glycol, plant oils such as olive oil, alcohols such as ethanol, Polysorbate 80 (Japanese Pharmacopeia), and the like. Such a composition may further contain a tonicity agent, an antiseptic, a moistening agent, an emulsifying agent, a dispersing agent, a stabilizing agent, or a solubilizing agent These are sterilized, for example, by filtration through a bacteria retaining filter, blending of a bactericide, or irradiation. In addition, these can also be used by preparing a sterile solid composition, and dissolving or suspending it in sterile water or a sterile solvent for injection prior to its use.

The agent for external use includes ointments, plasters, creams, jellies, cataplasms, sprays, lotions, eye drops, eye ointments, and the like. The agents contain generally used ointment bases, lotion bases, aqueous or non-aqueous liquid preparations, suspensions, emulsions, and the like. Examples of the ointment bases or the lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, sorbitan sesquioleate, and the like.
Regarding the transmucosal agents such as an inhalation, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device, and the like. The dry powder inhaler or the like may be for single or multiple administration use, and a dry powder or a powder-containing capsule may be used. Alternatively, this may be in a form such as a pressurized aerosol spray which uses an appropriate propellant, for example, a suitable gas such as chlorofluoroalkane, hydrofluoroalkane, carbon dioxide, and the like, or other forms.

Generally, in the case of oral administration, the daily dose is from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably administered from about 0.0001 to 10 mg/kg per body weight, once a day or two or more times a day. In addition, a transmucosal agent is administered at a dose from about 0.001 to 100 mg/kg per body weight, once a day or two or more times a day. The dose is appropriately decided in response to the individual case by taking the symptoms, the age, the gender, and the like into consideration.

The compound of the formula (I) can be used in combination with various agents for treating or preventing the above diseases for which the compound of the formula (I) is considered to be effective. The combined preparation may be administered simultaneously, or separately and continuously or at a desired time interval. The preparations to be co-administered may be a combination drug, or may be prepared individually.

### Examples

Hereinbelow, the production processes for the compound of the formula (I) are described with reference to Examples in more detail. The present invention is not limited to the compounds as described in Examples below. In addition, the production processes for the starting compounds are shown in Production Examples. Further, the production processes for the compound of the formula (I) are not limited to the production methods of specific Examples as shown below, but the compound of the formula (I) can be prepared by the combination of these production processes therefor or the methods obvious to a skilled person in the art.

In addition, the following abbreviations are used in Production Examples, Examples, and Tables below. Pre: Production Example number, Ex: Example number, No: Compound number, Str: Structural Formula (The description of HCl, TFA, and fum in the structural formula means that the compounds are hydrochloride, trifluoroacetate, and fumarate, respectively), Syn: Production Process (The number shows that it was prepared using a corresponding starting material in the same manner as the Example compound having the number as the Example number. For example, Example Compound 55 each shows that it was prepared in the same manner as for Example Compound 1). PSyn: Production Process (The number shows that it was prepared using a corresponding starting material in the same manner as the compound having the number as the Production Example number. For example, Production Example Compound 57 each shows that it was prepared in the same manner as for Production Example Compound 2.), Dat: Physicochemical Data (NMR: δ (ppm) in 1H NMR in DMSO-d₆, FAB+: FAB-MS (cation) (which means (M+H)⁺ unless otherwise specified), FAB-: FAB-MS (anion) (which means (M-H)⁻ unless otherwise specified), ESI+: ESI-MS (cation) (which means (M+H)⁺ unless otherwise specified), ESI-: ESI-MS (anion) (which means (M-H)⁻ unless otherwise specified), EI: EI-MS (which means (M)⁺ unless otherwise specified), CI+: CI-MS (cation) (which means (M+H)⁺, unless otherwise specified), APCI+: APCI-MS (cation) (which means (M+H)⁺ unless otherwise specified), APCI/ESI+: meaning the simultaneous measurement of APCI+ and ESI+, Elemental Analysis: Analysis of Elements, Found: Found Values (%), calc: Calculated Values (%)).

### Production Example 1

To a solution of 1.0 g of 3,5-dichloro-6-methylpyridin-2(1H)-one in 15 ml of DME was added 777 mg of potassium carbonate at room temperature, followed by stirring at 80°C for 30 minutes, and then 1.63 g of methyl 4-(2-iodoethyl)benzoate was added thereto, followed by heating and reflux for 12 hours. Further, 777 mg of potassium carbonate and 1.63 g of methyl 4-(2-iodoethyl)benzoate were added thereto, followed by stirring at the same temperature for 12 hours. Again, the same operations were repeated. Under ice-cooling, ethyl acetate and 1 M hydrochloric acid were added thereto to carry out a liquid separation operation. The organic layer was washed with saturated brine and then dried over anhydrous sodium sulfate, and the solvent was evaporated under reduced pressure. The obtained solid was washed with a mixed solvent of ethyl acetate and n-hexane to collect 317 mg of 3,5-dichloro-6-methylpyridin-2(1H)-one. On the other hand, after the mother liquid was concentrated under reduced pressure, the residue was purified by silica gel column chromatography to obtain 538 mg of methyl 4-[2-(3,5-dichloro-6-methyl-2-oxopyridin-1(2H)-yl)ethyl]benzoate.

### Production Example 2

To a solution of 444 mg of tert-butyl 4-[2-(3,5-dichloro-6-methyl-2-oxopyridin-1(2H)-yl)ethyl]benzoate in 10 ml of carbon tetrachloride were added 210 mg of N-bromosuccinimide and 19 mg of 2,2'-azobis(isobutyronitrile), followed by heating and reflux for 1 hour. After leaving it to be cooled at room temperature, chloroform and saturated aqueous sodium bicarbonate were added thereto to carry out a liquid separation operation. The organic layer was washed with saturated brine and dried over anhydrous sodium sulfate, and the solvent was evaporated under reduced pressure. The residue was purified by silica gel column chromatography to obtain 393 mg of tert-butyl 4-{2-[6-(bromomethyl)-3,5-dichloro-2-oxopyridin-1(2H)-yl]ethyl}benzoate.

### Production Example 3

To a solution of 390 mg of tert-butyl 4-{2-[6-(bromomethyl)-3,5-dichloro-2-oxopyridin-1(2H)-yl]ethyl}benzoate in 10 ml of acetone was added 347 mg of sodium acetate, followed by stirring at 70°C for 6 hours. After leaving it to be cooled at room temperature, a saturated aqueous ammonium chloride solution and ethyl acetate were added thereto to carry out a liquid separation operation. The organic layer was washed with saturated brine, dried over anhydrous sodium sulfate, and concentrated under reduced pressure to obtain 369 mg of tert-butyl 4-{2-[6-(acetoxymethyl)-3,5-dichloro-2-oxopyridin-1(2H)-yl]ethyl}benzoate.

### Production Example 4

To a solution of 365 mg of tert-butyl 4-{2-[6-(acetoxymethyl)-3,5-dichloro-2-oxopyridin-1(2H)-yl]ethyl}benzoate in 10 ml of methanol was added 343 mg of potassium carbonate at room temperature, followed by stirring for 30 minutes. Ethyl acetate and a saturated aqueous ammonium chloride solution were added thereto to carry out a liquid separation operation. The organic layer was washed with saturated brine, dried over anhydrous sodium sulfate, and concentrated under reduced pressure. The obtained solid was washed with a mixed solvent of ethyl acetate and n-hexane to obtain 251 mg of tert-butyl 4-{2-[3,5-dichloro-6-(hydroxymethyl)-2-oxopyridin-1(2H)-yl]ethyl}benzoate. On the other hand, the mother liquid was concentrated under reduced pressure, and the residue was purified by silica gel column chromatography to obtain 54 mg of tert-butyl 4-{2-[3,5-dichloro-6-(hydroxymethyl)-2-oxopyridin-1(2H)-yl]ethyl}benzoate.

### Production Example 5

To a solution of 22.8 g of methyl 4-{2-[6-(acetoxymethyl)-3,5-dichloro-2-oxopyridin-1(2H)-yl]ethyl}benzoate in 250 ml of methanol was slowly added 25 ml of concentrated sulfuric acid at room temperature, followed by stirring for 10 minutes and then heating and reflux for 2 hours. After leaving it to be cooled at room temperature, water was added thereto, and the precipitated solid was collected by filtration. The obtained solid was purified by silica gel column chromatography to obtain 16.8 g of methyl 4-{2-[3,5-dichloro-6-(hydroxymethyl)-2-oxopyridin-1(2H)-yl]ethyl}benzoate.

### Production Example 6

To a solution of 301 mg of tert-butyl 4-{2-[3,5-dichloro-6-(hydroxymethyl)-2-oxopyridin-1(2H)-yl]ethyl}benzoate in 8 ml of chloroform was added 1.3 g of manganese dioxide at room temperature, followed by stirring for 3 days. The reaction solution was filtered through Celite and the filtrate was concentrated under reduced pressure. The residue was purified by silica gel column chromatography to obtain 240 mg of tert-butyl 4-[2-(3,5-dichloro-6-formyl-2-oxopyridin-1(2H)-yl)ethyl]benzoate.

### Production Example 7

To a solution of 2.93 g of methyl 4-{2-[6-(bromomethyl)-3,5-dichloro-2-oxopyridin-1(2H)-yl]ethyl}benzoate in 58 ml of DMF were slowly added 2.22 g of sodium carbonate and 1.55 g of trimethylamine N-oxide, and the pressure of the reaction system was reduced using an aspirator, followed by stirring at 40°C for 15 minutes. Ethyl acetate and water were added to the reaction solution to carry out a liquid separation operation, and the organic layer was washed with water and saturated brine in this order, dried over anhydrous sodium sulfate, and concentrated under reduced pressure. The residue was purified by silica gel column chromatography to obtain 1.65 g of methyl 4-[2-(3,5-dichloro-6-formyl-2-oxopyridin-1(2H)-yl)ethyl]benzoate as a pale yellow solid.

### Production Example 8

To a mixed solution of 300 mg of methyl 4-[2-(3,5-dichloro-6-formyl-2-oxopyridin-1(2H)-yl)ethyl]benzoate in 1.8 ml of tert-butylalcohol, 0.3 ml of acetonitrile, and 0.6 ml of water were added 3 83 mg of sodium chlorite, 102 mg of sodium dihydrogen phosphate, and 0.45 ml of 2-methyl-2-butene at room temperature, followed by stirring for 2 hours. To the reaction solution was added sodium hydrogen sulfite, and then ethyl acetate and saturated aqueous sodium bicarbonate were added thereto to carry out a liquid separation operation. The aqueous layer was made weakly acidic by the addition of 1 M hydrochloric acid, and ethyl acetate was added thereto to carry out a liquid separation operation again. The organic layer was dried over anhydrous sodium sulfate and concentrated under reduced pressure to obtain 182 mg of 3,5-dichloro-1-{2-[4-(methoxycarbonyl)phenyl]ethyl}-6-oxo-1,6-dihydropyridine 2-carboxylic acid.

### Production Example 9

To a suspension of 62.7 g of 4-hydroxy-6-methyl-2H-pyran-2-one in 40 ml of water was added 497 ml of a 1 M aqueous sodium hydroxide solution, followed by stirring at room temperature for 15 minutes. Then, 50 g of 4-(2-aminoethyl)benzoic acid hydrochloride was added thereto, followed by stirring at 80°C for 24 hours. After leaving it to be cooled, 249 ml of 1 M hydrochloric acid and 200 ml of methanol were added thereto, followed by stirring for 30 minutes. The precipitated solid was collected by filtration to obtain 64.6 g of 4-[2-(4-hydroxy-6-methyl-2-oxopyridin-1(2H)-yl)ethyl]benzoic acid.

### Production Example 10

To a suspension of 67.8 g of 4-[2-(4-hydroxy-6-methyl-2-oxopyridin-1(2H)-yl)ethyl]benzoic acid in 500 ml of methanol was slowly added 50 ml of concentrated sulfuric acid, followed by heating and reflux for 4 hours. After leaving it to be cooled, 1.50 L of water was added, and the precipitated solid was collected by filtration to obtain 69.2 g of methyl 4-[2-(4-hydroxy-6-methyl-2-oxopyridin-(2H)-yl]benzoate.

### Production Example 11

To a suspension of 69.2 g of methyl 4-[2-(4-hydroxy-6-methyl-2-oxopyridin-1(2H)-yl]benzoate in 500 ml of pyridine was added 60 ml of trifluoromethanesulfonic anhydride at 5°C over about 1 hour, followed by stirring at the same temperature for 2 hours. Then, 500 ml of 1 M hydrochloric acid and 500 ml of water were added thereto in this order, and the precipitated solid was collected by filtration to obtain a pale brown solid. Ethyl acetate and water were added to the obtained solid to carry out a liquid separation operation, the organic layer was washed with saturated brine and dried over anhydrous sodium sulfate, and the solvent was evaporated under reduced pressure to obtain 84.0 g of methyl 4-{2-[6-methyl-2-oxo-4-{[(trifluoromethyl)sulfonly]oxy}pyridin-1(2H)-yl]ethyl}benzoate. Further, ethyl acetate and water were added to the mother liquid to carry out a liquid separation operation, and the same operations were carried out to obtain 20.2 g of methyl 4-{2-[6-methyl-2-oxo-4-{[(trifluoromethyl)sulfonly]oxy}pyridin-1(2H)-yl]ethyl}benzoate.

### Production Example 12

To a solution of 56 g of methyl 4-{2-[6-methyl-2-oxo-4-{[(trifluoromethyl)sulfonly]oxy}pyridin-1(2H)-yl]ethyl}benzoate in 300 ml of ethyl acetate were added 28 ml of N-ethyl-N-isopropylpropan-2-amine and 2.8 g of 10% palladium-carbon (water-containing product), followed by stirring at room temperature for 4 hours under a hydrogen atmosphere. The reaction solution was filtered through Celite, and water and ethyl acetate were added to the filtrate to carry out a liquid separation operation. The organic layer was washed with saturated brine and then dried over anhydrous sodium sulfate, and the solvent was evaporated under reduced pressure. The obtained residue was made into powders with ethyl acetate to obtain 21.3 g of methyl 4-[2-(6-methyl-2-oxopyridin-1(2H)-yl)ethyl]benzoate as a white solid. On the other hand, the mother liquid was concentrated under reduced pressure, and then the residue was purified by silica gel column chromatography to obtain 12.3 g of methyl 4-[2-(6-methyl-2-oxopyridin-1(2H)-yl)ethyl]benzoate as a pale brown solid.

### Production Example 13

To a mixed liquid of 65.0 g of methyl 4-[2-(6-methyl-2-oxopyridin-1(2H)-yl)ethyl]benzoate, 500 ml of acetic acid, and 300 ml of water was added 64 g of N-chlorosuccinimide, followed by stirring at room temperature for 30 minutes, and then at 70°C overnight. After leaving it to be cooled at room temperature, 1 L of water was added thereto and the precipitated solid was collected by filtration. The obtained solid was washed under heating with a mixed solvent of ethyl acetate and hexane to obtain 58.8 g of methyl 4-[2-(3,5-dichloro-6-methyl-2-oxopyridin-1(2H)-yl)ethyl]benzoate.

### Production Example 14

To a solution of 3.0 g of methyl 4-[2-(6-methyl-2-oxopyridin-(2H)-yl)ethyl]benzoate in 60 ml of acetic acid was added 2.0 g of N-bromosuccinimide, followed by stirring at room temperature for 3 hours. Water and ethyl acetate were added thereto to carry out a liquid separation operation, and the organic layer was washed with saturated brine, then dried over anhydrous sodium sulfate, and concentrated under reduced pressure. The residue was purified by silica gel column chromatography to obtain 2.2 g of methyl 4-[2-(5-bromo-6-methyl-2-oxopyridin-1(2H)-yl)ethyl]benzoate.

### Production Example 15

To a mixed liquid of 21.5 g of methyl 4-[2-(3,5-dichloro-6-methyl-2-oxopyridin-1(2H)-yl)ethyl]benzoate in 130 ml of THF and 70 ml of methanol was added 76 ml of a 1 M aqueous sodium hydroxide solution, followed by stirring at 70°C for 4 hours. After leaving it to be cooled at room temperature, the reaction solution was neutralized with 1 M hydrochloric acid, and the precipitated solid was collected by filtration to obtain 19.6 g of 4-[2-(3,5-dichloro-6-methyl-2-oxopyridin-1(2H)-yl)ethyl]benzoic acid.

### Production Example 16

To a mixed solution of 19.6 g of 4-[2-(3,5-dichloro-6-methyl-2-oxopyridin-1(2H)-yl)ethyl]benzoic acid in 150 ml of THF and 150 ml of tert-butylalcohol were slowly added 16 g of di-tert-butyl dicarbonate and 3.7 g of N,N-dimethylpyridin-4-amine, followed by stirring at 60°C overnight. After leaving it to be cooled at room temperature, 16 g of di-tert-butyl dicarbonate and 3.7 g of N,N-dimethylpyridin-4-amine were added thereto, followed by stirring at 60°C overnight. After leaving it to be cooled at room temperature again, 7.0 g of di-tert-butyl dicarbonate was added thereto, followed by stirring at 60°C overnight. After leaving it to be cooled at room temperature, water and ethyl acetate were added thereto to carry out a liquid separation operation. The organic layer was washed with saturated brine, then dried over anhydrous sodium sulfate, and concentrated under reduced pressure. The residue was purified by silica gel column chromatography to obtain 18.8 g of tert-butyl 4-[2-(3,5-dichloro-6-methyl-2-oxopyridin-1(2H)-yl)ethyl]benzoate.

### Production Example 17

To a solution of 2.0 g of methyl 4-(2-bromoethyl)benzoate in 50 ml of acetone was added 2.6 g of sodium iodide at room temperature, followed by stirring overnight. The precipitated solid was removed by filtration, and then the filtrate was concentrated under reduced pressure. Water and ethyl acetate were added to the residue to carry out a liquid separation operation. The organic layer was washed with saturated brine, dried over anhydrous sodium sulfate, and then concentrated under reduced pressure to obtain 2.4 g of methyl 4-(2-iodoethyl)benzoate.

### Production Example 18

To a mixed solution of 1.06 g of methyl 4-[2-(3-chloro-6-methyl-2-oxopyridin-1(2H)-yl)ethyl]benzoate in 30 ml of acetic acid and 10 ml of water was added 648 mg of N-bromosuccinimide at room temperature, followed by stirring overnight. Water was added thereto and the precipitated solid was collected by filtration to obtain 1.27 g of methyl 4-[2-(5-bromo-3-chloro-6-methyl-2-oxopyridin-1(2H)-yl)ethyl]benzoate as a pale brown solid.

### Production Example 19

To a solution of 1.0 g of methyl 4-{2-[3-chloro-6-methyl-2-oxo-4-{[(trifluoromethyl)sulfonly]oxy}pyridin-1(2H)-yl]ethyl}benzoate in 20 ml of THF were added 1.23 ml of triethylamine and 170 µl of formic acid in this order under ice-cooling, followed by stirring for 5 minutes. 75 mg of palladium acetate and 174 mg of triphenylphosphine were added thereto at room temperature, followed by stirring at 60°C for 3 hours. Ethyl acetate and 1 M hydrochloric acid were added thereto under ice-cooling to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 520 mg of methyl 4-[2-(3-chloro-6-methyl-2-oxopyridin-1(2H)-yl)ethyl]benzoate as a pale yellow solid.

### Production Example 20

To a suspension of a mixture of 5.0 g of methyl 4-[2-(4-hydroxy-6-methyl-2-oxopyridin-1(2H)-yl)ethyl]benzoate in 20 ml of acetic acid and 5.0 ml of water was added 2.33 g of N-chlorosuccinimide at room temperature, followed by stirring at 80°C overnight. After cooling, water was added thereto and the precipitated solid was collected by filtration to obtain 4.48 g of methyl 4-[2-(3-chloro-4-hydroxy-6-methyl-2-oxopyridin-1(2H)-yl)ethyl]benzoate as a pale brown solid.

### Production Example 21

To a mixed solution of 170 mg of methyl 4-[2-(6-methyl-2-oxo-5-phenylpyridin-1(2H)-yl)ethyl]benzoate in 4.0 ml of acetic acid and 1.0 ml of water was added 78 mg of N-chlorosuccinimide at room temperature, followed by stirring at 100°C overnight. After cooling, water and chloroform were added thereto to carry out a liquid separation operation, the organic layer was washed with a saturated aqueous sodium chloride solution, and then dried over anhydrous sodium sulfate, and the solvent was evaporated under reduced pressure. The residue was purified by silica gel column chromatography to obtain 100 mg of methyl 4-[2-(3-chloro-6-methyl-2-oxo-5-phenylpyridin-1 (2H)-yl)ethyl]benzoate.

### Production Example 22

To a solution of 500 mg of methyl 4-[2-(5-bromo-6-methyl-2-oxopyridin-1(2H)-yl)ethyl]benzoate in 10 ml of 1,4-dioxane were added 261 mg of phenylboronic acid, 485 mg of tripotassium phosphate, and 165 mg of tetrakistriphenylphosphine palladium at room temperature, followed by stirring at 90°C overnight. After cooling, the insoluble materials were removed by filtration using Celite and the solvent was evaporated under reduced pressure. The residue was purified by silica gel column chromatography to obtain 170 mg of methyl 4-[2-(6-methyl-2-oxo-5-phenylpyridin-1(2H)-yl)ethyl]benzoate.

### Production Example 23

To a solution of 1.0 g of tert-butyl (2R)-2-(hydroxymethyl)pyrrolidine-1-carboxylate in 10 ml of DMF was added 325 mg of 55% sodium hydride (oily) under ice-cooling, and then 872 mg of 3-bromo-2-methylprop-1-ene was added thereto, followed by stirring at room temperature overnight. Water and ethyl acetate were added to the reaction solution to carry out a liquid separation operation, the organic layer was washed with a saturated aqueous sodium chloride solution and then dried over anhydrous sodium sulfate, and the solvent was evaporated under reduced pressure. The residue was purified by silica gel column chromatography to obtain 1.1 g of colorless oily tert-butyl (2R)-2-{[(2-methylprop-2-en-1-yl)oxy]methyl}pyrrolidine-1-carboxylate.

### Production Example 24

To a solution of 940 mg of tert-butyl (2R)-2-{[(2-methylprop-2-en-1-yl)oxy]methyl}pyrrolidine-1-carboxylate in 10 ml of ethanol was added 150 mg of 10% palladium carbon, followed by stirring at room temperature for 24 hours under a hydrogen atmosphere. After filtration using Celite, the solvent was evaporated under reduced pressure. The residue was purified by silica gel column chromatography to obtain 792 mg of colorless oily tert-butyl (2R)-2-(isobutoxymethyl)pyrrolidine-1-carboxylate.

### Production Example 25

To a solution of 785 mg of tert-butyl (2R)-2-(isobutoxymethyl)pyrrolidine-1-carboxylate in 5.0 ml of ethyl acetate was added 5.0 ml of 4 M hydrogen chloride-ethyl acetate at room temperature, followed by stirring overnight. The solvent was evaporated under reduced pressure to obtain 610 mg of colorless oily (2R)-2-(isobutoxymethyl)pyrrolidine hydrochloride.

### Production Example 26

A solution of 2.0 g of 1-(3-fluorophenyl)acetone and 7.39 ml of methylamine (40% methanol solution) in 20 ml of methanol was stirred at room temperature overnight. 603 mg of sodium borohydride was added thereto at room temperature, followed by stirring overnight. Water and ethyl acetate were added thereto to carry out a liquid separation operation, the organic layer was washed with a saturated aqueous sodium chloride solution and then dried over anhydrous sodium sulfate, and the solvent was evaporated under reduced pressure. The residue was purified by silica gel column chromatography to obtain 529 mg of pale brown oily 1-(3-trifluorophenyl)-N-methylpropan-2-amine.

### Production Example 27

(1) To a suspension of 1.46 g of magnesium and a catalytic amount of iodide in 4.0 ml of THF was slowly added a solution of 4.47 g of (bromomethyl)cyclobutane in 27 ml of THF, followed by heating, to prepare a Grignard reagent. The Grignard reagent prepared was added dropwise to a solution of 4.0 g of 4-chlorobutanoyl chloride and 150 mg of iron (III) acetylacetonate in 40 ml of THF at -78°C under an argon atmosphere, followed by stirring at the same temperature for 30 minutes. A saturated aqueous ammonium chloride solution and ethyl acetate were added to the reaction solution to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 4.49 g of 5-chloro-1-cyclobutylpentan-2-one as a colorless oily substance.
(2) A mixture of 4.49 g of the colorless oily substance obtained, 3.88 g of (2S)-2-amino-2-phenylethanol, 4.48 ml of N-ethyl-N-isopropylpropan-2-amine, and 2.25 ml of chloroform were stirred at room temperature for 7 days. To the reaction product was added diethyl ether, and the supernatant was removed. These operations were repeated three times, followed by concentration under reduced pressure. The residue was purified by silica gel column chromatography to obtain 5.7 g of a pale yellow oily substance including rel-(3R,7aR)-7a-(cyclobutylmethyl)-3-phenylhexahydropyrrolo[2,1-b][1,3]oxazole. ESI+: 258
(3) To a solution of 5.7 g of the pale yellow oily substance including rel-(3R,7aR)-7a-(cyclobutylmethyl)-3-phenylhexahydropyrrolo[2,1-b][1,3]oxazole in 171 ml of THF was added dropwise 30.5 ml of lithium triethylborohydride (1.09 M solution in THF) under ice-cooling under an argon atmosphere, followed by stirring at 4°C overnight. To the reaction solution was added water, and then ethyl acetate was added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 4.48 g of yellow oily (2S)-2-[(2R)-2-(cyclobutylmethyl)pyrrolidon-1-yl]-2-phenylethanol.

### Production Example 28

To a solution of 4.48 g of (2S)-2-[(2R)-2-(cyclobutylmethyl)pyrrolidon-1-yl]-2-phenylethanol in 90 ml of methanol was added 900 mg of 10% palladium carbon, followed by stirring at room temperature for 4 days under a hydrogen atmosphere of 3 atm. After filtration using Celite, the solvent was evaporated under reduced pressure. To the residue was added a 4 M hydrogen chloride-ethyl acetate solution to make it into hydrochloride, followed by addition of n-hexane, and the supernatant was removed. These operations were repeated three times, and then the remaining material was made into powders from a mixed solvent of diethyl ether and ethyl acetate to obtain 1.5 g of (2R)-2-(cyclobutylmethyl)pyrrolidine hydrochloride as a white solid.

### Production Example 29

To a solution of 3.25 g of tert-butyl 4-[2-(5-bromo-6-methyl-2-oxopyridin-1(2H)-yl)ethyl]benzoate in 60 ml of toluene was added 3.0 ml of water, and then 1.0 g of cyclopropylboronic acid, 7.0 g of tripotassium phosphate, 186 mg of palladium acetate, and 464 mg of tricyclohexylphosphine were added thereto in this order at room temperature, followed by heating and reflux overnight. After cooling, water and ethyl acetate were added thereto, the insoluble materials were removed using Celite and then a liquid separation operation was carried out. The organic layer was washed with 0.5 M hydrochloric acid, a saturated aqueous sodium hydrogen carbonate solution, and a saturated aqueous sodium chloride solution in this order, and dried over anhydrous sodium sulfate, and the solvent was evaporated under reduced pressure. The residue was purified by silica gel column chromatography to obtain 2.17 g of tert-butyl 4-[2-(5-cyclopropyl-6-methyl-2-oxopyridin-1(2H)-yl)ethyl]benzoate as a pale yellow solid.

### Production Example 30

To a solution of 353 mg of tert-butyl 4-[2-(5-cyclopropyl-6-methyl-2-oxopyridin-1(2H)-yl)ethyl]benzoate in 8.0 ml of DMF was added a solution of 93 mg of trichloroisocyanuric acid in 2.0 ml of DMF at -5°C under cooling, followed by stirring at the same temperature for 1 hour. Water and ethyl acetate were added thereto to carry out a liquid separation operation, the organic layer was washed with a saturated aqueous sodium chloride solution and then dried over anhydrous sodium sulfate, and the solvent was evaporated under reduced pressure. The residue was purified by silica gel column chromatography to obtain 231 mg of tert-butyl 4-[2-(3-chloro-5-cyclopropyl-6-methyl-2-oxopyridin-1(2H)-yl)ethyl]benzoate as a pale yellow solid.

### Production Example 31

To a solution of 1.0 g of (1S,2S)-2-(benzyloxy)cyclopentanamine in 20 ml of THF were added 659 mg of sodium hydrogen carbonate and 1.37 g of di-tert-butyl dicarbonate at room temperature, followed by stirring for 12 hours. Water and ethyl acetate were added thereto to carry out a liquid separation operation, the organic layer was washed with 1 M hydrochloric acid and a saturated aqueous sodium chloride solution in this order, and then dried over anhydrous sodium sulfate, and the solvent was evaporated under reduced pressure. The residue was purified by silica gel column chromatography to obtain 1.51 g of tert-butyl [(1S,2S)-2-(benzyloxy)cyclopentyl]carbamate as a white solid.

### Production Example 32

To a solution of 626 mg of tert-butyl [(1S,2S)-2-(benzyloxy)cyclopentyl]carbamate in 13 ml of DMF was added 141 mg of 55% sodium hydride (oily) under ice-cooling, followed by stirring at room temperature for 10 minutes. At the same temperature, 0.4 ml of methyl iodide was added thereto, followed by stirring at 55°C overnight. Water and ethyl acetate were added thereto under ice-cooling to carry out a liquid separation operation, followed by washing sequentially with a saturated aqueous sodium chloride solution and then drying over anhydrous sodium sulfate, and the solvent was evaporated under reduced pressure. The residue was purified by silica gel column chromatography to obtain 423 mg of colorless oily tert-butyl [(1S,2S)-2-(benzyloxy)cyclopentyl]methylcarbamate.

### Production Example 33

To a solution of 589 mg of tert-butyl [(1S,2S)-2-(benzyloxy)cyclopentyl]methylcarbamate in 5.0 ml of ethanol was added 100 mg of 10% palladium hydroxide, followed by stirring at room temperature for 3 hours under a hydrogen atmosphere. After filtration using Celite, the solvent was evaporated under reduced pressure to obtain 436 mg of colorless oily tert-butyl [(1S,2S)-2-hydroxycyclopentyl]methylcarbamate.

### Production Example 34

To a solution of 280 mg of tert-butyl [(1S,2S)-2-hydroxycyclopentyl]methylcarbamate in 5.0 ml of DMF was added 85 mg of 55% sodium hydride (oily) under ice-cooling, followed by stirring at room temperature for 10 minutes. At the same temperature, 0.11 ml of methyl iodide was added thereto, followed by stirring overnight. Water and ethyl acetate were added thereto under ice-cooling to carry out a liquid separation operation, followed by washing with a saturated aqueous sodium chloride solution and then drying over anhydrous sodium sulfate, and the solvent was evaporated under reduced pressure. The residue was purified by silica gel column chromatography to obtain 253 mg of colorless oily tert-butyl [(1S,2S)-2-methoxycyclopentyl]methylcarbamate.

### Production Example 35

To a solution of 250 mg of tert-butyl [(1S,2S)-2-methoxycyclopentyl]methylcarbamate in 2.0 ml of ethyl acetate was added 2.0 ml of 4 M hydrogen chloride-ethyl acetate at room temperature, followed by stirring for 3 hours. The solvent was evaporated under reduced pressure to obtain 182 mg of colorless oily (1S,2S)-2-methoxy-N-methylcyclopentanamine hydrochloride.

### Production Example 36

To a suspension of 1.8 g of (methoxymethyl)(triphenyl)phosphonium chloride in 10 ml of THF was added 586 mg of potassium tert-butoxide under ice-cooling, followed by stirring for 10 minutes. At the same temperature, a solution of 1.0 g of methyl 4-[2-(3,5-dichloro-6-formyl-2-oxopyridin-1(2H)-yl)ethyl]benzoate in 10 ml of THF was added dropwise thereto, followed by stirring for 10 minutes. Separately, a reagent prepared from a solution of 484 mg of (methoxymethyl)(triphenyl)phosphonium chloride in 10 ml of THF, and 158 mg of potassium tert-butoxide was added to the reaction system under ice-cooling, followed by stirring for 10 minutes. Water and ethyl acetate were added to the reaction solution to carry out a liquid separation operation, the organic layer was washed with a saturated aqueous sodium chloride solution and then dried over anhydrous sodium sulfate, and the solvent was evaporated under reduced pressure. The residue was purified by silica gel column chromatography to obtain 534 mg of yellow oily methyl 4-{2-[3,5-dichloro-6-(2-methoxyvinyl)-2-oxopyridin-1(2H)-yl)ethyl]benzoate.

### Production Example 37

To a solution of 530 mg of methyl 4-{2-[3,5-dichloro-6-(2-methoxyvinyl)-2-oxopyridin-1(2H)-yl)ethyl]benzoate in 5.0 ml of DCM was added 10 ml of formic acid at room temperature, followed by stirring at 70°C for 5 hours. After cooling, the reaction solution was evaporated under reduced pressure, and then the residue was purified by silica gel column chromatography to obtain 422 mg of yellow oily methyl 4-{2-[3,5-dichloro-2-oxo-6-(2-oxoethyl)pyridin-1(2H)-yl]ethyl}benzoate.

### Production Example 38

To a solution of 719 mg of tert-butyl [(1R,2R)-2-hydroxycyclopentyl]methylcarbamate in 4.0 ml of pyridine was added 2.0 ml of acetic anhydride under ice-cooling, followed by stirring at room temperature for 19 hours. After concentration under reduced pressure, the residue was purified by silica gel column chromatography to obtain 800 mg of colorless oily (1R,2R)-2-[(tert-butoxycarbonyl)(methyl)amino]cyclopentyl acetate.

### Production Example 39

To a solution of 255 mg of tert-butyl [3-(trifluoromethoxy)phenyl]carbamate in 6.0 ml of DMF was added 40 mg of 55% sodium hydride (oily) under ice-cooling, followed by stirring at room temperature for 1 hour. At the same temperature, 300 mg of methyl 4-{2-[6-(bromomethyl)-3-chloro-5-cyclopropyl-2-oxopyridin-1(2H)-yl]ethyl}benzoate was added, followed by stirring overnight. To the reaction solution was added water, and the precipitated solid was collected by filtration. The obtained solid was purified by silica gel column chromatography to obtain 400 mg of methyl 4-{2-[6-({(tert-butoxycarbonyl)[3-(trifluoromethoxy)phenyl]amino}methyl)-3-chloro-5-cyclopropyl-2-oxopyridin-1(2H)-yl]ethyl}benzoate.

### Production Example 40

To a mixture of 400 mg of methyl 4-{2-[6-({(tert-butoxycarbonyl)[3-(trifluoromethoxy)phenyl]amino}methyl)-3-chloro-5-cyclopropyl-2-oxopyridin-1(2H)-yl]ethyl}benzoate in 2.0 ml of methanol and 4.0 ml of THF was added 2.0 ml of a 1 M aqueous sodium hydroxide solution at room temperature, followed by stirring at 65°C for 2 hours. After cooling, the reaction system was neutralized by the addition of 1 M hydrochloric acid. Water was added thereto, and the precipitated solid was collected by filtration to obtain 300 mg of 4-{2-[6-({(tert-butoxycarbonyl)[3-(trifluoromethoxy)phenyl]amino}methyl)-3-chloro-5-cyclopropyl-2-oxopyridin-1(2H)-yl]ethyl}benzoic acid.

### Production Example 41

To a solution of 1.0 g of tert-butyl 4-[2-(5-cyclopropyl-6-methyl-2-oxopyridin-1(2H)-yl)ethyl]benzoate in 15 ml of acetic acid was added 600 mg of N-bromosuccinimide at room temperature, followed by stirring overnight. Water was added thereto and the precipitated solid was collected by filtration to obtain 1.2 g of tert-butyl 4-[2-(3-bromo-5-cyclopropyl-6-methyl-2-oxopyridin-1(2H)-yl)ethyl]benzoate.

### Production Example 42

To a solution of 50 mg of methyl 4-[2-(3,5,6-trimethyl-2-oxopyridin-1(2H)-yl)ethyl]benzoate in 3.0 mg of acetic acid was added 33 mg ofN-bromosuccinimide at room temperature, followed by stirring at 60°C for 4 hours. After cooling, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 43 mg of methyl 4-{2-[6-(bromomethyl)-3,5-dimethyl-2-oxopyridin-1(2H)-yl]ethyl}benzoate.

### Production Example 43

To a solution of 1.0 g of methyl 4-[2-(3,5-dibromo-6-methyl-2-oxopyridin-1(2H)-yl)ethyl]benzoate in 20 ml of toluene was added 2.0 ml of water, and then 841 mg of a 2,4,6-trivinylcyclotriboroxane/pyridine complex, 2.47 g of tripotassium phosphate, 52 mg of palladium acetate, and 191 mg of dicyclohexyl(2',6'-dimethoxybiphenyl-2-yl)phosphine were added thereto in this order at room temperature, followed by heating and reflux for 1.5 hours. After cooling, filtration was conducted using silica gel, and the effluent including the obtained methyl 4-[2-(6-methyl-2-oxo-3,5-divinylpyridin-1(2H)-yl)ethyl]benzoate in ethyl acetate was used in the next reaction as it was. To the obtained effluent was added 200 mg of 10% palladium carbon at room temperature, followed by thoroughly stirring overnight under a hydrogen atmosphere. After filtration using Celite, the solvent was evaporated under reduced pressure. The residue was purified by silica gel column chromatography to obtain 403 mg of yellow oily methyl 4-[2-(3,5-diethyl-6-methyl-2-oxopyridin-1(2H)-yl)ethyl]benzoate.

### Production Example 44

To a solution of 530 mg of methyl 4-[2-(3-chloro-6-methyl-2-oxo-5-vinylpyridin-1(2H)-yl)ethyl]benzoate in 11 ml of ethyl acetate was added 170 mg of 10% palladium carbon, followed by stirring for 4 hours under a hydrogen atmosphere. After filtration using Celite, the solvent was evaporated under reduced pressure to obtain 500 mg of methyl 4-[2-(3-chloro-5-ethyl-6-methyl-2-oxopyridin-1(2H)-yl)ethyl]benzoate.

### Production Example 45

To a solution of 220 mg of methyl 4-[2-(5-bromo-3-chloro-6-methyl-2-oxopyridin-1(2H)-yl)ethyl]benzoate in 10 ml of toluene was added 500 µl of water at room temperature, and then 288 mg of 2-isopropenyl-4,4,5,5-tetramethyl-1,3,2-dioxaborane, 364 mg of tripotassium phosphate, 19 mg of palladium acetate, and 48 mg of tricyclohexylphosphine were added thereto, followed by stirring at 80°C overnight. After cooling, water and ethyl acetate were added thereto, followed by filtration using Celite, and a liquid separation operation was carried out. The organic layer was washed with a saturated aqueous sodium chloride solution and then dried over anhydrous sodium sulfate, and the solvent was evaporated under reduced pressure. The residue was purified by silica gel column chromatography to obtain 143 mg of pale yellow oily methyl 4-[2-(3-chloro-5-isopropenyl-6-methyl-2-oxopyridin-1(2H)-yl)ethyl]benzoate.

### Production Example 46

To a solution of 1.0 g of methyl 4-[2-(5-bromo-3-chloro-6-methyl-2-oxopyridin-1(2H)-yl)ethyl]benzoate in 18 ml of toluene was added 1.6 ml of water at room temperature, and then 600 mg of a 2,4,6-trivinylcyclotriboroxane/pyridine complex, 1.66 g of tripotassium phosphate, 58 mg of palladium acetate, and 213 mg of dicyclohexyl(2',6'-dimethoxybiphenyl-2-yl)phosphine were added thereto, followed by stirring at 90°C overnight. After cooling, water and ethyl acetate were added thereto, followed by filtration using Celite, and a liquid separation operation was carried out. The organic layer was washed with a saturated aqueous sodium chloride solution and then dried over anhydrous sodium sulfate, and the solvent was evaporated under reduced pressure. The residue was purified by silica gel column chromatography to obtain 300 mg of methyl 4-[2-(3-chloro-6-methyl-2-oxo-5-vinylpyridin-1(2H)-yl)ethyl]benzoate.

### Production Example 47

To a solution of 530 mg of methyl 4-[2-(5-cyclopropyl-3,6-dimethyl-2-oxopyridin-1(2H)-yl)ethyl]benzoate in 11 ml of carbon tetrachloride was added 100 µl of bromine at room temperature, followed by stirring at 90°C overnight. After cooling, a saturated aqueous sodium hydrogen carbonate solution and chloroform were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 350 mg of methyl 4-{2-[6-(bromomethyl)-5-cyclopropyl-3-methyl-2-oxopyridin-1(2H)-yl]ethyl}benzoate.

### Production Example 48

To a solution of 200 mg of methyl 4-[2-(3-bromo-5-cyclopropyl-6-methyl-2-oxopyridin-1(2H)-yl)ethyl]benzoate in 4.0 ml of toluene was added 1.0 ml of water at room temperature, and then 61 mg of methylboronic acid, 326 mg of tripotassium phosphate, 6.0 mg of palladium acetate, and 21 mg of dicyclohexyl(2',6'-dimethoxybiphenyl-2-yl)phosphine were added thereto, followed by stirring at 90°C overnight. After cooling, water and ethyl acetate were added thereto, filtration was conducted using Celite, and a liquid separation operation was carried out. The organic layer was washed with a saturated aqueous sodium chloride solution and then dried over anhydrous sodium sulfate, and the solvent was evaporated under reduced pressure. The residue was purified by silica gel column chromatography to obtain 120 mg of pale yellow oily methyl 4-[2-(5-cyclopropyl-3,6-dimethyl-2-oxopyridin-1(2H)-yl)ethyl]benzoate.

### Production Example 49

To a solution of 5.57 g of methyl 4-[2-(5-chloro-6-methyl-2-oxopyridin-1(2H)-yl)ethyl]benzoate in 100 ml of toluene was added 5.0 ml of water at room temperature, and then 3.8 g of cyclopropylboronic monohydrate, 15.5 g of tripotassium phosphate, 409 mg of palladium acetate, and 1.5 g of dicyclohexyl(2',6'-dimethoxybiphenyl-2-yl)phosphine were added thereto, followed by heating and reflux for 4 hours. After cooling, 1 M hydrochloric acid and ethyl acetate were added thereto, filtration was conducted using Celite, and a liquid separation operation was carried out. The organic layer was washed with a saturated aqueous sodium chloride solution and then dried over anhydrous sodium sulfate, and the solvent was evaporated under reduced pressure. The residue was purified by silica gel column chromatography to obtain 4.68 g of methyl 4-[2-(5-cyclopropyl-6-methyl-2-oxopyridin-1(2H)-yl)ethyl]benzoate as a pale yellow solid.

### Production Example 50

To a solution of 1.0 g of methyl 4-[2-(3-bromo-5-chloro-6-methyl-2-oxopyridin-1(2H)-yl)ethyl]benzoate in 20 ml of 1,4-dioxane were added 233 mg of methylboronic acid, 883 mg of tripotassium phosphate, and 601 mg of tetrakistriphenylphosphine palladium at room temperature, followed by stirring at 90°C for 5 days. After cooling, the insoluble materials were removed by filtration using Celite, and the solvent was evaporated under reduced pressure. The residue was purified by silica gel column chromatography to obtain 425 mg of methyl 4-[2-(5-chloro-3,6-dimethyl-2-oxopyridin-1(2H)-yl)ethyl]benzoate.

### Production Example 51

To a solution of 231 mg of tert-butyl 4-[2-(3-chloro-5-cyclopropyl-6-methyl-2-oxopyridin-1(2H)-yl)ethyl]benzoate in 5.0 ml of carbon tetrachloride were added 111 mg of N-bromosuccinimide and 5 mg of 2,2'-azobis(isobutyronitrile), followed by heating and reflux overnight. After cooling, chloroform and water were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 189 mg of tert-butyl 4-{2-[6-(bromomethyl)-3-chloro-5-cydopropyl-2-oxopyridin-1(2H)-yl]ethyl}benzoate in a pale yellow amorphous state.

### Production Example 52

To a solution of 2.0 g of 4-{2-[6-(bromomethyl)-3,5-dichloro-2-oxopyridin-1(2H)-yl]ethyl}benzonitrile in 40 ml of DMF were added 430 mg of potassium carbonate and 466 mg of (2R)-2-isobutylpyrrolidine hydrochloride at room temperature, followed by stirring overnight. Water and ethyl acetate were added thereto to carry out a liquid separation operation, the organic layer was washed with a saturated aqueous sodium chloride solution and then dried over anhydrous sodium sulfate, and the solvent was evaporated under reduced pressure. The residue was purified by silica gel column chromatography to obtain 400 mg of 4-{2-[3,5-dichloro-2-oxo-6-{[(2R)-2-isobutylpyrpyrrolidin-1-yl]methyl}pyridin-1(2H)-yl]ethyl}benzonitrile as a white solid.

### Production Example 53

To a solution of 170 mg of 4-{2-[3,5-dichloro-2-oxo-6-{[(2S)-2-propylpyrrolidin-1-yl]methyl}pyridin-1(2H)-yl]ethyl}benzonitrile in 4.0 ml of DMSO were added 170 µl of triethylamine and 85 mg of hydroxylamine hydrochloride at room temperature, followed by stirring at 100°C overnight. After cooling, water was added thereto, and the precipitated solid was collected by filtration to obtain 180 mg of 4-{2-[3,5-dichloro-2-oxo-6-{[(2S)-2-propylpyrrolidin-1-yl]methyl}pyridin-1(2H)-yl]ethyl}-N'-hydroxybenzenecarboxyimidamide.

### Production Example 54

To a solution of 500 mg of 4-{2-[3,5-dichloro-2-oxo-6-{[(2R)-2-isobutylpyrrolidin-1-yl]methyl}pyridin-1(2H)-yl]ethyl}benzoic acid trifluoroacete in 10 ml of DMF were added 148 µl of triethylamine, 206 mg of 1-(3-dimethylaminopropyl)-3-ethylcarbodiimide hydrochloride, 191 mg of HOBt, and 58 mg of hydrazine monohydrate in this order at room temperature, followed by stirring at the same temperature overnight. Water and ethyl acetate were added to the reaction solution to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 400 mg of 4-{2-[3,5-dichloro-2-oxo-6-{[(2R)-2-isobutylpyrrolidin-1-yl]methyl}pyridin-1(2H)-yl]ethyl}benzohydrazine.

### Production Example 55

To a solution of 4.0 g of methyl 4-[2-(5-cyclopropyl-3,6-dimethyl-2-oxopyridin-1(2H)-yl)ethyl]benzoate in 150 ml of acetic acid was added 1.0 g of trichloroisocyanuric acid at room temperature, followed by stirring at the same temperature for 4 hours. Water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 4.4 g of methyl 4-[2-[6-(bromomethyl)-5-cyclopropyl-3-methyl-2-oxopyridin-1(2H)-yl]ethyl]benzoate in a colorless amorphous state.

### Production Example 56

To a solution of 1.3 g of methyl 4-[2-(3,6-dimethyl-2-oxopyridin-1(2H)-yl)ethyl]benzoate in 26 ml of acetic acid was added 1.8 g of N-bromosuccinimide at room temperature, followed by stirring at 90°C overnight. After cooling, water and chloroform were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 1.8 g of methyl 4-{2-[5-bromo-6-(bromomethyl)-3-methyl-2-oxopyridin-1(2H)-yl]ethyl}benzoate.

The Production Example Compounds 57 to 99 were prepared in the same manner as the methods of Production Examples 1 to 56 above using the respective corresponding starting materials. The structures, the production processes, and the physicochemical data of the Production Example Compounds are shown in Tables 8 to 24.

### Example 1

To a solution of 500 mg of methyl 4-{2-[6-(bromomethyl)-3,5-dichloro-2-oxopyridin-1(2H)-yl]ethyl}benzoate in 10 ml of DMF were added 200 mg of potassium carbonate and 200 mg of 3-isopropyl-N-methylbenzenamine, followed by stirring at 60°C overnight. After leaving it to be cooled at room temperature, water and ethyl acetate were added thereto to carry out a liquid separation operation. The organic layer was washed with saturated brine, then dried over anhydrous sodium sulfate, and concentrated under reduced pressure. The residue was purified by silica gel column chromatography to obtain 420 mg of methyl 4-{2-[3,5-dichloro-6-{[(3-isopropylphenyl)(methyl)amino]methyl}-2-oxopyridin-1(2H)-yl]ethyl}benzoate.

### Example 2

To a solution of 173 mg of tert-butyl (4-ethylpyridin-2-yl)carbamate in 3.0 ml of DMF was added 28 mg of 55% sodium hydride (oily), followed by stirring for 30 minutes, and then 300 mg of tert-butyl 4-{2-[6-(bromomethyl)-3,5-dichloro-2-oxopyridin-1(2H)-yl]ethyl}benzoate was added thereto, followed by stirring at the same temperature as room temperature overnight. Ethyl acetate and water were added to the reaction solution to carry out a liquid separation operation. The organic layer was washed with water and saturated brine in this order, then dried over anhydrous sodium sulfate, and concentrated under reduced pressure. The residue was purified by silica gel column chromatography to obtain 300 mg of tert-butyl 4-{2-[6-{[(tert-butoxycarbonyl)(4-ethylpyridin-2-yl)amino]methyl}-3,5-dichloro-2-oxopyridin-1(2H)-yl]ethyl}benzoate.

### Example 3

A solution of 254 mg of methyl 4-{2-[5-bromo-6-(bromomethyl)-2-oxopyridin-1(2H)-yl]ethyl}benzoate and 157 mg of 6-methylindoline in 2.5 ml of NMP was stirred at 140°C for 10 minutes using a microwave device (INITIATOR 60 manufactured by Biotage Japan Ltd.). After leaving it to be cooled at room temperature, ethyl acetate and water were added to the reaction solution to carry out a liquid separation operation. The organic layer was washed with water and saturated brine, then dried over anhydrous sodium sulfate, and concentrated under reduced pressure. The residue was purified by silica gel column chromatography to obtain 170 mg of methyl 4-(2-{5-bromo-6-[(6-methyl-2,3-dihydro-1H-indol-1-yl)methyl]-2-oxopyridin-1(2H)-yl} ethyl)benzoate.

### Example 4

To a solution of 200 mg of tert-butyl 4-{2-[6-(bromomethyl)-3,5-dichloro-2-oxopyridin-1(2H)-yl]ethyl}benzoate in 2.0 ml of DMF were added 55 mg of sodium carbonate and 66 mg of 4-propylpiperidine, followed by stirring at room temperature overnight. Ethyl acetate and water were added to the reaction solution to carry out a liquid separation operation. The organic layer was washed with water and saturated brine in this order, then dried over anhydrous sodium sulfate, and concentrated under reduced pressure. The residue was purified by silica gel column chromatography to obtain 215 mg of tert-butyl 4-{2-[3,5-dichloro-2-oxo-6-[(4-propylpiperidin-1-yl)methyl]pyridin-1(2H)-yl]ethyl}benzoate.

### Example 5

To a solution of 420 mg of methyl 4-{2-[3,5-dichloro-6-{[(3-isopropylphenyl)(methyl)amino]methyl}-2-oxopyridin-1(2H)-yl]ethyl}benzoate in 8.0 ml of 1,4-dioxane was added 1.0 ml of 6 M hydrochloric acid, followed by stirring at 90°C overnight. After leaving it to be cooled at room temperature, chloroform and water were added thereto to carry out a liquid separation operation. The organic layer was washed with saturated brine, then dried over anhydrous sodium sulfate, and concentrated under reduced pressure. The residue was dissolved in a 1 M aqueous sodium hydroxide solution, followed by addition of 1 M hydrochloric acid, and the precipitated solid was collected by filtration. The solid was dried and then reprecipitated with ethanol to obtain 177 mg of 4-{2-[3,5-dichloro-6-{[(3-isopropylphenyl)(methyl)amino]methyl}-2-oxopyridin-1(2H)-yl]ethyl}benzoic acid as a colorless solid.

### Example 6

To a solution of 100 mg of tert-butyl 4-{2-[3,5-dichloro-6-{[methyl(3-methylphenyl)amino]methyl}-2-oxopyridin-1(2H)-yl]ethyl}benzoate in 2.0 ml of DCM was added 500 µL of trifluoroacetic acid, followed by stirring at room temperature for 2 hours. The reaction solution was concentrated under reduced pressure, and then to the residue were added a 1 M aqueous sodium hydroxide solution and water, followed by dissolution. The solution was neutralized by the addition of 1 M hydrochloric acid, and the precipitated solid was collected by filtration to obtain 62 mg of 4-{2-[3,5-dichloro-6-{[methyl(3-methylphenyl)amino]methyl}-2-oxopyridin-1(2H)-yl]ethyl} benzoic acid.

### Example 7

To a solution of 295 mg of tert-butyl 4-{2-[6-{[(tert-butoxycarbonyl)(4-ethylpyridin-2-yl)amino]methyl}-3,5-dichloro-2-oxopyridin-1(2H)-yl]ethyl}benzoate in 2.9 ml of DCM was added 2.9 ml of trifluoroacetic acid, followed by stirring at room temperature for 4 hours. The reaction solution was concentrated under reduced pressure, and then the residue was washed with ethyl acetate to obtain 265 mg of 4-{2-[3,5-dichloro-6-{[(4-ethylpyridin-2-yl)amino]methyl}-2-oxopyridin-1(2H)-yl]ethyl}benzoic acid trifluoroacetate.

### Example 8

To a solution of 20 mg of 3,5-dichloro-1-{2-[4-(methoxycarbonyl)phenyl]ethyl}-6-oxo-1,6-dihydropyridine-2-carboxylic acid in 1 ml of THF were added 11 µL of oxalyl dichloride and 4 µl of DMF, followed by stirring at room temperature for 1 hour. The reaction solution was concentrated under reduced pressure. The obtained residue was added to a separately prepared solution of 20 mg of 3-ethylaniline and 11 mg of triethylamine in 1 ml of THF, followed by stirring at room temperature for 2 hours. Water and ethyl acetate were added thereto to carry out a liquid separation operation, the organic layer was washed with saturated brine and then dried over anhydrous sodium sulfate, and the solvent was evaporated under reduced pressure. The residue was purified by silica gel column chromatography to obtain 13 mg of methyl 4-(2-{3,5-dichloro-6-[(3-ethylphenyl)carbamoyl]-2-oxopyridin-1(2H)-yl}ethyl)benzoate. To a solution of the present compound in 2.0 ml of 1,4-dioxane was added 2.0 ml of 6 M hydrochloric acid, followed by heating and reflux overnight. After leaving it to be cooled at room temperature, water and ethyl acetate were added thereto to carry out a liquid separation operation. The organic layer was washed with saturated brine and then dried over anhydrous sodium sulfate, and the solvent was evaporated under reduced pressure. The residue was purified by silica gel column chromatography, and then made into powders with a mixed solution of diisopropylether, ethyl acetate, and n-hexane to obtain 6 mg of 4-(2-{3,5-dichloro-6-[(3-ethylphenyl)carbamoyl]-2-oxopyridin-1(2H)-yl}ethyl)benzoic acid.

### Example 9

To a solution of 250 mg of methyl 4-{2-[6-(bromomethyl)-3,5-dichloro-2-oxopyridin-1(2H)-yl]ethyl}benzoate in 2.5 ml of DMF were added 99 mg of potassium carbonate and 90 mg of 6-fluoroindoline, followed by stirring at 60°C overnight. After leaving it to be cooled at room temperature, water and ethyl acetate were added to the reaction solution to carry out a liquid separation operation. The organic layer was washed with saturated brine, then dried over anhydrous sodium sulfate, and concentrated under reduced pressure. The residue was purified by silica gel column chromatography to obtain methyl 4-(2-{3,5-dichloro-6-[(6-fluoro-2,3-dihydro-1H-indol-1-yl)methyl]-2-oxopyridin-1(2H)-yl}ethyl)benzoate. To a solution of the present compound in 2.5 ml of 1,4-dioxane was added 2.5 ml of 6 M hydrochloric acid, followed by heating and reflux overnight under an argon atmosphere. After leaving it to be cooled at room temperature, the solvent was evaporated under reduced pressure, and the precipitated solid was collected by filtration to obtain 130 mg of 4-(2-{3,5-dichloro-6-[(6-fluoro-2,3-dihydro-1H-indol-1-yl)methyl]-2-oxopyridin-1(2H)-yl}ethyl)benzoic acid.

### Example 10

To a solution of 250 mg of tert-butyl 4-{2-[6-(bromomethyl)-3,5-dichloro-2-oxopyridin-1(2H)-yl]ethyl}benzoate in 2.5 ml of DMF were added 187 mg of potassium carbonate and 119 mg of 6-ethoxyindoline hydrochloride, followed by stirring at 60°C overnight. After leaving it to be cooled at room temperature, water and ethyl acetate were added to the reaction solution to carry out a liquid separation operation. The organic layer was washed with saturated brine, then dried over anhydrous sodium sulfate, and concentrated under reduced pressure. The residue was purified by silica gel column chromatography to obtain tert-butyl 4-(2-{3,5-dichloro-6-[(6-ethoxy-2,3-dihydro-1H-indol-1-yl)methyl]-2-oxopyridin-1(2H)-yl}ethyl)benzoate. To a solution of the present compound in 2.5 ml of DCM was added 2.5 ml of trifluoroacetic acid, followed by stirring at room temperature for 2 hours. The solvent was evaporated under reduced pressure, a mixed solution of ethyl acetate and hexane were added thereto, and the precipitated solid was collected by filtration to obtain 159 mg of 4-(2-{3,5-dichloro-6-[(6-ethoxy-2,3-dihydro-1H-indol-1-yl)methyl]-2-oxopyridin-1(2H)-yl}ethyl)benzoic acid.

### Example 11

To a solution of 150 mg of methyl 4-{2-[6-(bromomethyl)-3,5-dichloro-2-oxopyridin-1(2H)-yl]ethyl}benzoate in 3.0 ml of DMF were added 75 mg of potassium carbonate and 67 µl of N,2-dimethylaniline, followed by stirring at 60°C overnight. After leaving it to be cooled at room temperature, water and ethyl acetate were added thereto to carry out a liquid separation operation. The organic layer was washed with saturated brine, then dried over anhydrous sodium sulfate, and concentrated under reduced pressure. The residue was purified by silica gel column chromatography to obtain methyl 4-{2-[3,5-dichloro-6-{[methyl(2-methylphenyl)amino]methyl}-2-oxopyridin-1(2H)-yl]ethyl}benzoate. To a mixed solution of the present compound in 1.0 ml of methanol and 1.0 ml of THF was added 400 µl of a 1 M aqueous sodium hydroxide solution, followed by stirring at 60°C overnight. After leaving it to be cooled at room temperature, the reaction solution was neutralized by the addition of 1 M hydrochloric acid, and the precipitated insoluble materials were collected by filtration. The obtained insoluble materials were purified by silica gel column chromatography and dissolved in a 1 M aqueous sodium hydroxide solution, followed by addition of 1 M hydrochloric acid, and the precipitated solid was collected by filtration to obtain 25 mg of 4-{2-[3,5-dichloro-6-{[methyl(2-methylphenyl)amino]methyl}-2-oxopyridin-1(2H)-yl]ethyl}benzoic acid.

### Example 12

91 mg of N-phenylaniline in 2.0 ml of DMF was added 16 mg of 55% sodium hydride (oily) under ice-cooling, followed by stirring for 30 minutes, and then 150 mg of methyl 4-{2-[6-(bromomethyl)-3,5-dichloro-2-oxopyridin-1(2H)-yl]ethyl}benzoate was added thereto, followed by stirring at room temperature overnight. A saturated aqueous ammonium chloride solution and ethyl acetate were added to the reaction solution to carry out a liquid separation operation. The organic layer was washed with saturated brine, then dried over anhydrous sodium sulfate, and concentrated under reduced pressure. The residue was purified by silica gel column chromatography to obtain methyl 4-(2-[3,5-dichloro-6-[(diphenylamino)methyl]-2-oxopyridin-1(2H)-yl]ethyl)benzoate. To a solution of the present compound in 2.0 ml of 1,4-dioxane was added 2.0 ml of 6 M hydrochloric acid, followed by heating and reflux overnight. After leaving it to be cooled at room temperature, ethyl acetate and water were added thereto to carry out a liquid separation operation. The organic layer was washed with saturated brine, then dried over anhydrous sodium sulfate, and concentrated under reduced pressure. The residue was purified by silica gel column chromatography to obtain 17 mg of 4-(2-[3,5-dichloro-6-[(diphenylamino)methyl]-2-oxopyridin-1(2H)-yl]ethyl)benzoic acid.

### Example 13

A solution of 250 mg of methyl 4-{2-[5-bromo-6-(bromomethyl)-2-oxopyridin-1(2H)-yl]ethyl}benzoate and 176 mg of 7-ethylindoline in 2.5 ml of NMP was stirred at 140°C for 10 minutes using a microwave device. After leaving it to be cooled at room temperature, ethyl acetate and water were added thereto to carry out a liquid separation operation. The organic layer was washed with water and saturated brine, then dried over anhydrous sodium sulfate, and concentrated under reduced pressure. The residue was purified by silica gel column chromatography to obtain methyl 4-(2-{5-bromo-6-[(7-ethyl-2,3-dihydro-1H-indol-1-yl)methyl]-2-oxopyridin-1(2H)-yl}ethyl)benzoate. To a solution of the present compound in 2.5 ml of 1,4-dioxane was added 2.5 ml of 6 M hydrochloric acid at room temperature, followed by heating and reflux overnight under an argon atmosphere. After leaving it to be cooled at room temperature, the solvent was evaporated under reduced pressure and the precipitated solid was washed with ethyl acetate to obtain 237 mg of 4-(2-{5-bromo-6-[(7-ethyl-2,3-dihydro-1H-indol-1-yl)methyl]-2-oxopyridin-1(2H)-yl}ethyl)benzoic acid.

### Example 14

A solution of 250 mg of methyl 4-{2-[5-bromo-6-(bromomethyl)-2-oxopyridin-1(2H)-yl]ethyl}benzoate and 240 mg of 7-(trifluoromethyl)-1,2,3,4-tetrahydroquinoline in 2.5 ml of NMP was stirred at 140°C for 10 minutes using a microwave device. After leaving it to be cooled at room temperature, ethyl acetate and water were added thereto to carry out a liquid separation operation. The organic layer was washed with water and saturated brine, then dried over anhydrous sodium sulfate, and concentrated under reduced pressure. The residue was purified by silica gel column chromatography to obtain methyl 4-{2-[3,5-dichloro-2-oxo-6-{[7-(trifluoromethyl)-3,4-dihydroquinolin-1(2H)-yl]ethyl}benzoate. To a solution of the present compound in 2.5 ml of THF was added 2.0 ml of a 1 M aqueous sodium hydroxide solution, followed by heating and reflux for 2 days under an argon atmosphere. After leaving it to be cooled at room temperature, the reaction solution was neutralized by the addition of 1 M hydrochloric acid. After the solvent was evaporated under reduced pressure, the precipitated solid was collected by filtration to obtain 145 mg of 4-{2-[3,5-dichloro-2-oxo-6-{[7-(trifluoromethyl)-3,4-dihydroquinolin-1(2H)-yl]ethyl}benzoic acid.

### Example 15

To a solution of 250 mg of methyl 4-{2-[6-(bromomethyl)-3,5-dichloro-2-oxopyridin-1(2H)-yl]ethyl}benzoate in 2.5 ml of NMP were added 247 mg of potassium carbonate and 120 mg of 6-ethylindoline hydrochloride, followed by stirring at 60°C overnight. After leaving it to be cooled at room temperature, water and ethyl acetate were added to the reaction solution to carry out a liquid separation operation. The organic layer was washed with saturated brine, then dried over anhydrous sodium sulfate, and concentrated under reduced pressure. The residue was purified by silica gel column chromatography to obtain methyl 4-(2-{3,5-dichloro-6-[(6-ethyl-2,3-dihydro-1H-indol-1-yl)methyl]-2-oxopyridin-1(2H)-yl}ethyl)benzoate. To a solution of the present compound in 2.5 ml of 1,4-dioxane was added 2.5 ml of 6 M hydrochloric acid at room temperature, followed by heating and reflux overnight under an argon atmosphere. After leaving it to be cooled at room temperature, the solvent was evaporated under reduced pressure, and the precipitated solid was collected by filtration to obtain 196 mg of 4-(2-{3,5-dichloro-6-[(6-ethyl-2,3-dihydro-1H-indol-1-yl)methyl]-2-oxopyridin-1(2H)-yl}ethyl)benzoic acid.

### Example 16

To a solution of 77 mg of indoline in 2.0 ml of DMF was added 58 mg of potassium tert-butoxide, followed by stirring at room temperature for 30 minutes, and then 200 mg of tert-butyl 4-{2-[6-(bromomethyl)-3,5-dichloro-2-oxopyridin-1(2H)-yl]ethyl}benzoate was added thereto, followed by stirring at the same temperature overnight. Ethyl acetate and water were added to the reaction solution to carry out a liquid separation operation, and the organic layer was washed with water and saturated brine in this order, then dried over anhydrous sodium sulfate, and concentrated under reduced pressure. The residue was purified by silica gel column chromatography to obtain 180 mg of tert-butyl 4-{2-[3,5-dichloro-6-(2,3-dihydro-1H-indol-1-ylmethyl)-2-oxopyridin-1(2H)-yl]ethyl}benzoate. To a solution of the present compound in 1.8 ml of DCM was added 1.8 ml of trifluoroacetic acid, followed by stirring at room temperature for 4 hours. The solvent was evaporated under reduced pressure and the precipitated solid was washed with ethyl acetate to obtain 70 mg of 4-{2-[3,5-dichloro-6-(2,3-dihydro-1H-indol-1-ylmethyl)-2-oxopyridin-1(2H)-yl]ethyl}benzoic acid.

### Example 17

A solution of 600 mg of tert-butyl 4-{2-[6-(bromomethyl)-3,5-dichloro-2-oxopyridin-1(2H)-yl]ethyl}benzoate and 1.0 g of (2S)-2-propylpyrrolidine in 6.0 ml of NMP was stirred at 140°C for 10 minutes using a microwave device. After leaving it to be cooled at room temperature, ethyl acetate and water were added thereto to carry out a liquid separation operation. The organic layer was washed with saturated brine, then dried over anhydrous sodium sulfate, and concentrated under reduced pressure. The residue was purified by silica gel column chromatography. To a solution of the obtained compound in 6.0 ml of DCM was added 6.0 ml of trifluoroacetic acid, followed by stirring at room temperature for 4 hours. The solvent was evaporated under reduced pressure and the precipitated solid was washed with ethyl acetate to obtain 628 mg of 4-{2-[3,5-dichloro-2-oxo-6-{[(2S)-2-propylpyrrolidin-1-yl]methyl}pyridin-1(2H)-yl]ethyl}benzoic acid trifluoroacetate.

### Example 18

To a solution of 600 mg of tert-butyl 4-{2-[6-(bromomethyl)-3,5-dichloro-2-oxopyridin-1(2H)-yl]ethyl}benzoate in 6.0 ml of NMP were added 539 mg of potassium carbonate and 255 mg of (2R)-2-isobutylpyrrolidine hydrochloride, followed by stirring at 60°C overnight. After leaving it to be cooled at room temperature, ethyl acetate and water were added thereto to carry out a liquid separation operation. The organic layer was washed with saturated brine and then dried over anhydrous sodium sulfate, and the solvent was evaporated under reduced pressure. The residue was purified by silica gel column chromatography, and to a solution of the obtained compound in 6.0 ml of DCM was added 6.0 ml of trifluoroacetic acid, followed by stirring at room temperature for 4 hours. After the solvent was evaporated under reduced pressure, the precipitated solid was washed with ethyl acetate to obtain 518 mg of 4-{2-[3,5-dichloro-6-{[(2R)-2-isobutylpyrrolidin-1-yl]methyl}-2-oxopyridin-1(2H)-yl]ethyl}benzoic acid trifluoroacetate.

### Example 19

A solution of 100 mg of tert-butyl 4-{2-[6-(bromomethyl)-3,5-dichloro-2-oxopyridin-1(2H)-yl]ethyl}benzoate and 84 mg ofN-methyl-3-(trifluoromethoxy)aniline in 2.0 ml of NMP was stirred at 140°C for 10 minutes using a microwave device. After leaving it to be cooled at room temperature, ethyl acetate and water were added to the reaction solution to carry out a liquid separation operation. The organic layer was washed with saturated brine and then dried over anhydrous sodium sulfate, and the solvent was evaporated under reduced pressure. The residue was purified by silica gel column chromatography to obtain 53 mg of 4-{2-[3,5-dichloro-6-({methyl[3-(trifluoromethoxy)phenyl]amino}methyl)-2-oxopyridin-1(2H)-yl]ethyl}benzoic acid.

### Example 20

To a solution of 200 mg of tert-butyl 4-{2-[6-(bromomethyl)-3,5-dichloro-2-oxopyridin-1(2H)-yl]ethyl}benzoate in 6.0 ml of DMF were added 96 mg of potassium carbonate and 76 mg of N-methylcyclopentanamine hydrochloride at room temperature, followed by stirring overnight. Water and ethyl acetate were added to the reaction solution to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 203 mg of tert-butyl 4-{2-[3,5-dichloro-6-{[cyclopentyl(methyl)amino]methyl}-2-oxopyridin-1(2H)-yl]ethyl}benzoate as a white solid.

### Example 21

A mixed solution of 150 mg of tert-butyl 4-{2-[6-(bromomethyl)-3,5-dichloro-2-oxopyridin-1(2H)-yl]ethyl}benzoate, 45 mg of potassium carbonate, 91 mg ofN,5-dimethyl-1,3-thiazol-2-amine, and 5.0 ml of NMP was stirred at 110°C for 10 minutes using a microwave device. After cooling, ethyl acetate and water were added to the reaction solution to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 139 mg of tert-butyl 4-{2-[3,5-dichloro-6-{[methyl(5-methyl-1,3-thiazol-2-yl)amino]methyl}-2-oxopyridin-1(2H)-yl]ethyl}benzoate as a white solid.

### Example 22

A solution of 20 mg of methyl 4-{2-[6-(bromomethyl)-3-chloro-5-cyclopropyl-2-oxopyridin-1(2H)-yl]ethyl}benzoate, 120 mg of N-methyl-3-(trifluoromethyl)aniline hydrochloride, and 200 µl of N-ethyl-N-isopropylpropan-2-amine in 4.0 ml of NMP was stirred at 140°C for 10 minutes using a microwave device. After cooling, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 240 mg of methyl 4-{2-[3-chloro-5-cyclopropyl-6-({methyl[3-(trifluoromethyl)phenyl]amino}methyl)-2-oxopyridin-1(2H)-yl]ethyl}benzoate.

### Example 23

To a solution of 450 mg of tert-butyl 4-{2-[3-bromo-6-(bromomethyl)-5-cyclopropyl-2-oxopyridin-1(2H)-yl]ethyl}benzoate in 9.0 ml of DMF were added 220 µl of N-ethyl-N-isopropylpropan-2-amine and 190 mg of (2R)-2-isobutylpyrrolidine hydrochloride at room temperature, followed by stirring overnight. Water and ethyl acetate were added to the reaction solution to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 350 mg of tert-butyl 4-{2-[3-bromo-5-cyclopropyl-6-{[(2R)-2-isobutylpyrrolidin-1-yl]methyl}-2-oxopyridin-1(2H)-yl]ethyl}benzoate as a white solid.

### Example 24

A solution of 120 mg of methyl 4-{2-[6-(bromomethyl)-5-cyclopropyl-3-methyl-2-oxopyridin-1(2H)-yl]ethyl}benzoate, 75 mg ofN-methyl-3-(trifluoromethyl)aniline hydrochloride, and 90 mg of potassium carbonate in 2.4 ml of NMP was stirred at 140°C for 15 minutes using a microwave device. After cooling, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 120 mg of methyl 4-{2-[5-cyclopropyl-3-methyl-6-({methyl[3-(trifluoromethyl)phenyl]amino}methyl)-2-oxopyridin-(2H)-yl]ethyl}benzoate.

### Example 25

To a solution of 100 mg of tert-butyl 4-{2-[6-(bromomethyl)-3,5-dichloro-2-oxopyridin-1(2H)-yl]ethyl}benzoate in 3.0 ml of DMF were added 83 mg of 3-chloro-N,2-dimethylaniline hydrochloride and 70 mg ofN-ethyl-N-isopropylpropan-2-amine at room temperature, followed by stirring at 70°C overnight. 50 mg of potassium carbonate was added thereto at the same temperature, followed by stirring for 5 hours. After cooling, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 10 mg of colorless oily tert-butyl 4-{2-[3,5-dichloro-6-{[(3-chloro-2-methylphenyl)(methyl)amino]methyl}-2-oxopyridin-1(2H)-yl]ethyl}benzoate.

### Example 26

To a solution of 200 mg of tert-butyl 4-{2-[3,5-dichloro-6-{[cyclopentyl(methyl)amino]methyl}-2-oxopyridin-1(2H)-yl]ethyl}benzoate in 2.0 ml of DCM was added 2.0 ml of trifluoroacetic acid at room temperature, followed by stirring at the same temperature for 4 hours. After the solvent was evaporated under reduced pressure, the residue was made into powders by the addition of ethyl acetate to obtain 191 mg of 4-{2-[3,5-dichloro-6-{[cyclopentyl(methyl)amino]methyl}-2-oxopyridin-1(2H)-yl]ethyl}benzoic acid trifluoroacetate as a white solid.

### Example 27

To a solution of 80 mg of tert-butyl 4-{2-[5-cyclopropyl-6-{[(2R)-2-isobutylpyrrolidin-l-yl]methyl}-3-methyl-2-oxopyridin-1(2H)-yl]ethyl}benzoate in 1.6 ml of DCM was added 500 µl of trifluoroacetic acid at room temperature, followed by stirring overnight. After the solvent was evaporated under reduced pressure, to the residue were added a 1 M aqueous sodium hydroxide solution and water, followed by dissolution. The solution was neutralized by the addition of 1 M hydrochloric acid, and then chloroform was added thereto to carry out a liquid separation operation. The organic layer was dried over anhydrous sodium sulfate and the solvent was evaporated under reduced pressure. To a solution of the residue in 1.7 ml of ethyl acetate was added 500 µl of a 4 M hydrogen chloride-ethyl acetate solution under ice-cooling, followed by stirring at room temperature for 3 hours. After the solvent was evaporated under reduced pressure, the obtained hydrochloride was made into powders with a mixed solvent of ethyl acetate and n-hexane to obtain 26 mg of 4-{2-[5-cyclopropyl-6-{[(2R)-2-isobutylpyrrolidin-1-yl]methyl}-3-methyl-2-oxopyridin-1(2H)-yl]ethyl}benzoic acid as a colorless solid.

### Example 28

To a solution of 100 mg of methyl 4-{2-[5-bromo-3-chloro-6-{[(2R)-2-isobutylpyrrolidin-1-yl]methyl}-2-oxopyridin-1(2H)-yl]ethyl}benzoate in 4.0 ml of 1,4-dioxane was added 330 µl of 6 M hydrochloric acid at room temperature, followed by stirring at 90°C overnight. After cooling, the reaction solution was neutralized by the addition of a 1 M aqueous sodium hydroxide solution, then water was added thereto, and the precipitated solid was collected by filtration to obtain 58 mg of 4-{2-[5-bromo-3-chloro-6-{[(2R)-2-isobutylpyrrolidin-1-yl]methyl}-2-oxopyridin-1(2H)-yl]ethyl}benzoic acid as a colorless solid.

### Example 29

To a mixture of 190 mg of methyl 4-{2-[3-chloro-5-cyclopropyl-2-oxo-6-{[(2S)-2-propylpyrrolidin-1-yl]methyl}pyridin-1(2H)-yl]ethyl}benzoate in 6.0 ml of THF and 1.0 ml of methanol was added 1.25 ml of a 1 M aqueous sodium hydroxide solution at room temperature, followed by stirring at 60°C for 4 hours. After cooling, the reaction solution was neutralized by the addition of 1 M hydrochloric acid, and then ethyl acetate was added thereto to carry out a liquid separation operation. The organic layer was dried over anhydrous sodium sulfate and the solvent was evaporated under reduced pressure. To a solution of the residue in 5.0 ml of ethyl acetate was added 1.0 ml of a 4 M hydrogen chloride-ethyl acetate solution under ice-cooling, followed by stirring at room temperature for 30 minutes. After the solvent was evaporated under reduced pressure, the obtained hydrochloride was made into powders with a mixed solvent of methanol and ethyl acetate to obtain 171 mg of 4-{2-[3-chloro-5-cyclopropyl-2-oxo-6-{[(2S)-2-propylpyrrolidin-1-yl]methyl}pyridin-1(2H)-yl]ethyl}benzoic acid as a white solid.

### Example 30

To a solution of 205 mg of tert-butyl 4-{2-[6-{[butyl(methyl)amino]methyl}-3,5-dichloro-2-oxopyridin-1(2H)-yl]ethyl}benzoate in 2.0 ml of ethyl acetate was added 2.0 ml of a 4 M hydrogen chloride-ethyl acetate solution at room temperature, followed by stirring for 6 hours. After the solvent was evaporated under reduced pressure, to the residue was added water, followed by dissolution. The reaction solution was neutralized by the addition of a saturated aqueous sodium hydrogen carbonate solution, and chloroform was added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 37 mg of 4-{2-[6-{[butyl(methyl)amino]methyl}-3,5-dichloro-2-oxopyridin-1(2H)-yl]ethyl} benzoic acid as a white solid.

### Example 31

To a mixture of 143 mg of methyl 4-{2-[6-{[(2R)-2-isobutylpyrrolidin-1-yl]methyl}-3,5-dimethyl-2-oxopyridin-1(2H)-yl]ethyl}benzoate in 2.0 ml of THF and 2.0 ml of methanol was added 1.0 ml of a 1 M aqueous sodium hydroxide solution at room temperature, followed by stirring for 22 hours. The reaction solution was neutralized by the addition of 1 M hydrochloric acid, and then chloroform was added thereto to carry out a liquid separation operation. The organic layer was dried over anhydrous sodium sulfate and the solvent was evaporated under reduced pressure. To 2.0 ml of a solution of the residue in 1,4-dioxane was added 168 µl of a 4 M hydrogen chloride-1,4-dioxane solution under ice-cooling, followed by stirring at room temperature for 30 minutes. After the solvent was evaporated under reduced pressure, the obtained hydrochloride was made into powders from ethyl acetate to obtain 96 mg of 4-{2-[6-{[(2R)-2-isobutylpyrrolidin-1-yl]methyl}-3,5-dimethyl-2-oxopyridin-1(2H)-yl]ethyl}benzoic acid hydrochloride as a white solid.

### Example 32

A solution of 201 mg of tert-butyl 4-{2-[5-bromo-6-(bromomethyl)-2-oxopyridin-1(2H)-yl]ethyl}benzoate, 214 mg of N-methyl-3-(trifluoromethyl)aniline hydrochloride, and 0.14 ml of triethylamine in 2.0 ml of NMP was stirred at 140°C for 30 minutes using a microwave device. After cooling, ethyl acetate and water were added to the reaction solution to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography, and then the obtained solid was made into powders with diethylether to obtain 47 mg of 4-{2-[5-bromo-6-({methyl[3-(trifluoromethoxy)phenyl]amino}methyl)-2-oxopyridin-1(2H)-yl]ethyl}benzoic acid as a white solid.

### Example 33

To a solution of 100 mg of tert-butyl 4-{2-[6-(bromomethyl)-3,5-dichloro-2-oxopyridin-1(2H)-yl]ethyl}benzoate in 2.0 ml of DMF were added 39 mg of potassium carbonate and 42 mg of 3-methoxy-N-methylbenzylamine at room temperature, followed by stirring overnight. After cooling, water and ethyl acetate were added to the reaction solution to carry out a liquid separation operation. The organic layer was washed with a saturated aqueous sodium chloride solution and then dried over anhydrous sodium sulfate. The solvent was evaporated under reduced pressure, and to a solution of the residue in 2.0 ml of DCM was added 500 µl of trifluoroacetic acid at room temperature, followed by stirring overnight. The solvent was evaporated under reduced pressure and the residue was made into powders from a mixed solvent of ethyl acetate and n-hexane to obtain 77 mg of 4-{2-[3,5-dichloro-6-{[(3-methoxybenzyl)(methyl)amino]methyl}-2-oxopyridin-1(2H)-yl]ethyl}benzoic acid trifluoroacetate as a white solid.

### Example 34

A solution of 200 mg of methyl 4-{2-[6-(bromomethyl)-3-chloro-5-cyclopropyl-2-oxopyridin-1(2H)-yl]ethyl}benzoate, 77 mg of 3-methoxy-N-methylaniline, and 80 mg of potassium carbonate in 4.0 ml of DMF was stirred at 140°C for 10 minutes using a microwave device. After cooling, ethyl acetate and water were added to the reaction solution to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 200 mg of methyl 4-{2-[3-chloro-5-cyclopropyl-6-{[(3-methoxyphenyl)(methyl)amino]methyl}-2-oxopyridin-1(2H)-yl]ethyl}benzoate.

To a mixture of 200 mg of the obtained methyl 4-{2-[3-chloro-5-cyclopropyl-6-{[(3-methoxyphenyl)(methyl)amino]methyl}-2-oxopyridin-(2H)-yl]ethyl}benzoate in 4.0 ml of THF and 2.0 ml of methanol was added 1.25 ml of a 1 M aqueous sodium hydroxide solution at room temperature, followed by stirring at 65°C for 2 hours. After cooling, the reaction solution was neutralized by the addition of 1 M hydrochloric acid, then water was added thereto, and the precipitated solid was collected by filtration. The obtained solid was purified by silica gel column chromatography to obtain 65 mg of 4-{2-[3-chloro-5-cyclopropyl-6-{[(3-methoxyphenyl)(methyl)amino]methyl}-2-oxopyridin-1(2H)-yl]ethyl}benzoic acid.

### Example 35

To a solution of 120 mg of 4-{2-[3,5-dichloro-2-oxo-6-{[(2S)-2-propylpyrrolidin-1-yl]methyl}pyridin-1(2H)-yl]ethyl}benzoic acid in 4.0 ml of 1,4-dioxane was added 70 mg of CDI at room temperature, followed by stirring overnight. It was poured into 4.0 ml of 28% aqueous ammonia at the same temperature, followed by stirring for 1 hour. Water and chloroform were added to the reaction solution to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 40 mg of 4-{2-[3,5-dichloro-2-oxo-6-{[(2S)-2-propylpyrrolidin-1-yl]methyl}pyridin-1(2H)-yl]ethyl}benzamide.

### Example 36

To a solution of 800 mg of methyl 4-{2-[5-bromo-3-chloro-6-{[(2R)-2-isobutylpyrrolidin-1-yl]methyl}-2-oxopyridin-1(2H)-yl]ethyl}benzoate in 16 ml of 1,4-dioxane were added 122 mg of methylboronic acid, 466 mg of tripotassium phosphate, and 181 mg of tetrakistriphenylphosphine palladium at room temperature, followed by stirring at 90°C overnight. After cooling, water and ethyl acetate were added thereto, and then the insoluble materials were removed by filtration using Celite to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 200 mg of methyl 4-{2-[3-chloro-6-{[(2R)-2-isobutylpyrrolidin-1-yl]methyl}-5-methyl-2-oxopyridin-1(2H)-yl]ethyl}benzoate.

### Example 37

To a solution of 270 mg of methyl 4-{2-[3,5-dichloro-2-oxo-6-(2-oxoethyl)pyridin-1(2H)-yl]ethyl}benzoate in 15 ml of chloroform were added 143 mg of (2S)-2-propylpyrrolidine hydrochloride, 78 mg of sodium acetate, and 249 mg of sodium triacetoxyborohydride at room temperature, followed by stirring for 1 hour. Saturated sodium hydrogen carbonate and chloroform were added to the reaction solution to carry out a liquid separation operation. The organic layer was dried over anhydrous sodium sulfate and the solvent was evaporated under reduced pressure. The residue was purified by silica gel column chromatography to obtain 270 mg of yellow oily methyl 4-{2-[3,5-dichloro-2-oxo-6-{2-[(2S)-2-propylpyrrolidin-1-yl]ethyl}pyridin-1(2H)-yl]ethyl}benzoate.

### Example 38

A mixture of 130 mg of 4-{2-[6-({[(1R,2R)-2-acetoxycyclopentyl](methyl)amino}methyl)-3,5-dichloro-2-oxopyridin-1(2H)-yl]ethyl}benzoic acid trifluoroacetate, 50 mg of potassium carbonate, and 3.0 ml of methanol was stirred at room temperature overnight, and then stirred at 60°C for 3 hours. After cooling, the mixture was neutralized (pH=7) by the slow addition of 1 M hydrochloric acid, followed by addition of ethyl acetate to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography. To a solution of the obtained compound in 3.0 ml of ethyl acetate solution was added 500 µl of 4 M hydrogen chloride-ethyl acetate solution under ice-cooling, followed by stirring at room temperature for 30 minutes. After the solvent was evaporated under reduced pressure, the obtained hydrochloride was made into powders with a mixed solvent of ethyl acetate and n-hexane to obtain 10 mg of 4-{2-[3,5-dichloro-6-({[(1R,2R)-2-hydroxycydopentyl](methyl)amino}methyl)-2-oxopyridin-1(2H)-yl]ethyl}benzoic acid hydrochloride as a white solid.

### Example 39

To a solution of 250 mg of 4-{2-[6-({(tert-butoxycarbonyl)[3-(trifluoromethoxy)phenyl]amino}methyl)-3-chloro-5-cyclopropyl-2-oxopyridin-1(2H)-yl]ethyl}benzoic acid in 2.0 ml of ethyl acetate was added 0.1 ml of 4 M hydrogen chloride-ethyl acetate solution at room temperature, followed by stirring overnight. The precipitated solid was collected by filtration to obtain 200 mg of 4-{2-[3-chloro-5-cyclopropyl-2-oxo-6-({[3-(trifluoromethoxy)phenyl]amino}methyl)pyridin-1(2H)-yl]ethyl}benzoic acid.

### Example 40

To a solution of 350 mg of tert-butyl 4-{2-[3-bromo-5-cyclopropyl-6-{[(2R)-2-isobutylpyrrolidin-1-yl]methyl}-2-oxopyridin-1(2H)-yl]ethyl}benzoate in 3.5 ml of 1,4-dioxane were added 100 mg of methylboronic acid, 470 mg of tripotassium phosphate, and 75 mg of tetrakistriphenylphosphine palladium at room temperature, followed by stirring at 90°C for 3 days. After cooling, water and ethyl acetate were added thereto, and then the insoluble materials were removed by filtration using Celite to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 80 mg oftert-butyl 4-{2-[5-cyclopropyl-6-{[(2R)-2-isobutylpyrrolidin-1-yl]methyl}-3-methyl-2-oxopyridin-1(2H)-yl]ethyl}benzoate.

### Example 41

To a solution of 200 mg of methyl 4-[2-(5-bromo-3-chloro-6-methyl-2-oxopyridine-1(2H)-ethyl)benzoate in 2.0 ml of toluene was added 400 µl of water, and then 428 mg of 2-(trifluoromethoxy)phenylboronic acid, 552 mg oftripotassium phosphate, 12 mg of palladium acetate, and 43 mg of dicyclohexyl(2',6'-dimethoxybiphenyl-2-yl)phosphine were added in this order at room temperature, followed by reflux at 90°C overnight. After cooling, water and ethyl acetate were added thereto, and then the insoluble materials were removed by filtration using Celite to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain a preparation of 240 mg of methyl 4-(2-{6-methyl-2-oxy-3,5-bis[2-(trifluoromethoxy)phenyl]pyridin-1(2H)-yl}ethyl)benzoate.
To a solution of 240 mg of the obtained methyl 4-(2-{6-methyl-2-oxy-3,5-bis[2-(trifluoromethoxy)phenyl]pyridin-1(2H)-yl}ethyl)benzoate in 4.8 ml of carbon tetrachloride were added 96 mg of N-bromosuccinimide and 4.2 mg of 2,2'-azobis(isobutyronitrile), followed by heating and reflux overnight. After cooling, chloroform and water were added thereto to carry out a liquid separation operation. The organic layer was washed with a saturated aqueous sodium chloride solution and then dried over anhydrous sodium sulfate, and the solvent was evaporated under reduced pressure to obtain 240 mg of methyl 4-{2-[6-(bromomethyl)-2-oxo-3,5-bis[2-(trifluoromethoxy)phenyl]pyridin-1(2H)-yl]ethyl}benzoate.
To a solution of 140 mg of the crude material of the obtained methyl 4-{2-[6-(bromomethyl)-2-oxo-3,5-bis[2-(trifluoromethoxy)phenyl]pyridin-1(2H)-yl]ethyl}benzoate in DMF were added 85 mg of potassium carbonate and 50 mg of (2R)-2-isobutylpyrrolidine hydrochloride at room temperature, followed by stirring overnight. Water was added thereto, and the precipitated solid was collected by filtration to obtain 150 mg of methyl 4-{2-[6-{[(2R)-2-isobutylpyrrolidin-1-yl]methyl}-2-oxo-3,5-bis[2-(trifluoromethoxy)phenyl]pyridin-1(2H)-yl]ethyl}benzoate.
To a mixed solution of 150 mg of the obtained methyl 4-{2-[6-{[(2R)-2-isobutylpyrrolidin-1-yl]methyl}-2-oxo-3,5-bis[2-(trifluoromethoxy)phenyl]pyridin-1(2H)-yl]ethyl}benzoate in 3.0 ml of THF and 1.5 ml of methanol were added 1.0 ml of a 1 M aqueous sodium hydroxide solution, followed by stirring at 65°C for 3 hours. After cooling, the reaction system was neutralized by the addition of 1 M hydrochloric acid, and chloroform was added thereto to carry out a liquid separation operation. The organic layer was dried over anhydrous sodium sulfate and the solvent was evaporated under reduced pressure. To a solution of the residue in 2.0 ml of ethyl acetate was added 500 µl of a 4 M hydrogen chloride-ethyl acetate solution under ice-cooling, followed by stirring at room temperature for 30 minutes. After the solvent was evaporated under reduced pressure, the obtained hydrochloride was made into powders with a mixed solvent of ethyl acetate and n-hexane to obtain 60 mg of 4-{2-[6-{[(2R)-2-isobutylpyrrolidin-1-yl]methyl}-2-oxo-3,5-bis[2-(trifluoromethoxy)phenyl]pyridin-1(2H)-yl]ethyl}benzoic acid hydrochloride.

### Example 42

To a suspension of 100 mg of 4-{2-[3,5-dichloro-2-oxo-6-{[(2S)-2-propylpyrrolidin-1-yl]methyl}pyridin-1(2H)-yl]ethyl}benzonitrile in 2.0 ml of toluene were added 80 mg of sodium azide and 170 mg of triethylamine hydrochloride at room temperature, followed by stirring at 100°C for 3 days. After cooling, water and ethyl acetate were added thereto to carry out a liquid separation operation. The organic layer was dried over anhydrous sodium sulfate and the solvent was evaporated under reduced pressure. The residue was made into powders with a mixed solvent of ethyl acetate-n-hexane to obtain 51 mg of 3,5-dichloro-6-{[(2S)-2-propylpyrrolidin-1-yl]methyl}-1-{2-[4-(1H-tetrazol-5-yl)phenyl]ethyl}pyridin-2(1H)-one as a white solid.

### Example 43

To a solution of 50 mg of 4-{2-[3,5-dichloro-2-oxo-6-{[(2S)-2-propylpyrrolidin-1-yl]methyl}pyridin-1(2H)-yl]ethyl}-N'-hydroxybenzenecarboxyimidamide in 1.0 ml of DMF was added 30 mg of CDI at room temperature, followed by stirring at 80°C overnight. After cooling, water was added thereto, and the precipitated solid was collected by filtration to obtain 6.0 mg of 3,5-dichloro-1-{2-[4-(5-oxo-4,5-dihydro-1,2,4-oxadiazol-3-yl)phenyl]ethyl}-6-{[(2S)-2-propylpyrrolidin-1-yl]methyl}pyridin-2(1H)-one.

### Example 44

To a solution of 50 mg of 4-{2-[3,5-dichloro-2-oxo-6-{[(2S)-2-propylpyrrolidin-1-yl]methyl}pyridin-1(2H)-yl]ethyl}-N'-hydroxybenzenecarboxyimidamide in 1.0 ml of 1,4-dioxane were added 29 mg of 1,8-diazabicyclo[5,4,0]undec-7-ene and 33 mg of 1,1'-carbonothiobis(1H-imidazole) at room temperature, followed by stirring at 80°C overnight. After cooling, water was added thereto and the precipitated solid was collected by filtration to obtain 36 mg of 3,5-dichloro-6-{[(2S)-2-propylpyrrolidin-1-yl]methyl}-1-{2-[4-(5-thioxo-4,5-dihydro-1,2,4-oxadiazol-3-yl)phenyl]ethyl}pyridin-2(1H)-one.

### Example 45

To 50 mg of 4-{2-[3,5-dichloro-2-oxo-6-{[(2S)-2-propylpyrrolidin-1-yl]methyl}pyridin-1(2H)-yl]ethyl}-N'-hydroxybenzenecarboxyimidamide were added 1.0 ml of DCM, 15 µl of pyridine, and 13 µl of thionyl chloride in this order at room temperature, and the present mixture was stirred at the same temperature overnight. 1 M hydrochloric acid and chloroform were added to the reaction solution to carry out a liquid separation operation. The organic layer was dried over anhydrous sodium sulfate and the solvent was evaporated under reduced pressure. To a solution of the residue in 2.0 ml of ethyl acetate was added 500 µl of a 4 M hydrogen chloride-ethyl acetate solution under ice-cooling, followed by stirring at room temperature for 30 minutes. After the solvent was evaporated under reduced pressure, the obtained hydrochloride was made into powders with a mixed solvent of ethyl acetate and n-hexane to obtain 8.0 mg of 3,5-dichloro-1-{2-[4-(2-oxido-3H-1,2,3,5-oxathiazol-4-yl)phenyl]ethyl}-6-{[(2S)-2-propylpyrrolidin-1-yl]methyl}pyridin-2(1H)-one hydrochloride.

### Example 46

To a mixture of 120 mg of 3-{[(4-{2-[3,5-dichloro-2-oxo-6-{[(2S)-2-propylpyrrolidin-1-yl]methyl}pyridin-1(2H)-yl]ethyl}benzoyl)amino]sulfonly}propyl acetate in 2.0 ml of THF and 1.0 ml of methanol was added 1.0 ml of a 1 M aqueous sodium hydroxide solution at room temperature, followed by stirring for 3 hours. After cooling, the reaction solution was neutralized by the addition of 1 M hydrochloric acid, then water was added thereto, and the precipitated solid was collected by filtration. The obtained solid was purified by silica gel column chromatography to obtain 13 mg of 4-{2-[3,5-dichloro-2-oxo-6-{[(2S)-2-propylpyrrolidin-1-yl]methyl}pyridin-1(2H)-yl]ethyl}-N-[(3-hydroxypropyl)sulfonly]benzamide.

### Example 47

To a solution of 400 mg of 4-{2-[3,5-dichloro-2-oxo-6-{[(2R)-2-isobutylpyrrolidin-1-yl]methyl}pyridin-1(2H)-yl]ethyl}benzohydrazine in 8.0 ml of 1,4-dioxane was added 209 mg of N,N'-carbonyldiimidazole at room temperature, followed by stirring at 65°C overnight. After cooling, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 400 mg of 3,5-dichloro-1-{2-[4-(5-oxo-4,5-dihydro-1,3,4-oxadiazol-2-yl)phenyl]ethyl}-6-{[(2R)-2-isobutylpyrrolidin-1-yl]methyl}pyridin-2(1H)-one. To a solution of the obtained 400 mg of 3,5-dichloro-1-{2-[4-(5-oxo-4,5-dihydro-1,3,4-oxadiazol-2-yl)phenyl]ethyl}-6-{[(2R)-2-isobutylpyrrolidin-1-yl]methyl}pyridin-2(1H)-one in 8.0 ml of ethyl acetate was added 2.0 ml of a 4 M hydrogen chloride-ethyl acetate solution under ice-cooling, followed by stirring at room temperature for 30 minutes. After the solvent was evaporated under reduced pressure, it was made into powders with ethanol to obtain 195 mg of 3,5-dichloro-1-{2-[4-(5-oxo-4,5-dihydro-1,3,4-oxadiazol-2-yl)phenyl]ethyl}-6-{[(2R)-2-isobutylpyrrolidin-1-yl]methyl}pyridin-2(1H)-one hydrochloride as a white solid.

### Example 48

To a solution of 100 mg of 4-{2-[3,5-dichloro-2-oxo-6-{[(2S)-2-propylpyrrolidin-1-yl]methyl}pyridin-1(2H)-yl]ethyl}benzohydrazine in 2.0 ml of ethanol were added 19 mg of potassium hydroxide and 51 mg of carbon disulfide at room temperature, followed by stirring at 65°C overnight. After cooling, the reaction solution was acidified by the addition of 1 M hydrochloric acid, then water was added thereto, and the precipitated solid was collected by filtration to obtain 78 mg of 3,5-dichloro-6-{[(2S)-2-propylpyrrolidin-1-yl]methyl}-1-{2-[4-(5-thioxo-4,5-dihydro-1,3,4-oxadiazol-2-yl)phenyl]ethyl}pyridin-2(1H)-one.

### Example 49

To a solution of 100 mg of 4-{2-[3,5-dichloro-6-({methyl[3-(trifluoromethyl)phenyl]amino}methyl)-2-oxopyridin-1(2H)-yl]ethyl}benzoic acid in 2.0 ml of DMF were added 47 mg of 1-(3-dimethylaminopropyl)-3-ethylcarbodiimide hydrochloride, 43 mg of HOBt, and 14 mg of hydrazine monohydrate in this order at room temperature, followed by stirring at the same temperature overnight. Water and ethyl acetate were added to the reaction solution to carry out a liquid separation operation. The organic layer was washed with a saturated aqueous sodium chloride solution and then dried over anhydrous sodium sulfate, and the solvent was evaporated under reduced pressure to obtain a crude material of 4-{2-[3,5-dichloro-6-({methyl[3-(trifluoromethyl)phenyl]amino}methyl)-2-oxopyridin-1(2H)-yl]ethyl}benzohydrazine.
To a solution of the crude material of the obtained 4-{2-[3,5-dichloro-6-({methyl[3-(trifluoromethyl)phenyl]amino}methyl)-2-oxopyridin-1(2H)-yl]ethyl}benzohydrazine in 1.8 ml of 1,4-dioxane were added 40 µl oftriethylamine and 35 mg of N,N'-carbonyldiimidazole at room temperature, followed by stirring at 65°C overnight. After cooling, water was added thereto and the precipitated solid was collected by filtration to obtain 70 mg of 3,5-dichloro-6-({methyl[3-(trifluoromethyl)phenyl]amino}methyl)-1-{2-[4-(5-oxo-4,5-dihydro-1,3,4-oxazol-2-yl)phenyl] ethyl}pyridin-1(2H)-one.

### Example 50

To a solution of 150 mg of 4-{2-[3,5-dichloro-2-oxo-6-{[(2R)-2-isobutylpyrrolidin-1-yl]methyl}pyridin-1(2H)-yl]ethyl}-N' - hydroxybenzenecarboxyimidamide in 2.3 ml of THF was added 69 mg of 1,1'-carbonothiobis(1H-imidazole) at room temperature, followed by stirring at the same temperature for 2 hours. Water and ethyl acetate were added to the reaction solution to carry out a liquid separation operation. The organic layer was washed with a saturated sodium chloride and then dried over anhydrous sodium sulfate, and the solvent was evaporated under reduced pressure. To a solution of the residue in 2.3 ml of THF was added 204 µl of a boron trifluoride/ethyl ether complex at room temperature, followed by stirring at the same temperature for 1 hour. Water was added thereto and the precipitated solid was collected by filtration to obtain 72 mg of 3,5-dichloro-6-{[(2R)-2-isobutylpyrrolidon-1-yl]methyl}-1-{2-[4-(5-oxo-4,5-dihydro-1,2,4-thiazol-3-yl)phenyl]ethyl}pyridin-2(1H)-one.

### Example 51

To a solution of 200 mg of 4-{2-[3,5-dichloro-2-oxo-6-{[(2S)-2-propylpyrrolidin-1-yl]methyl}pyridin-1(2H)-yl]ethyl}benzoic acid trifluoroacetate in 2.0 ml of 1,4-dioxane was added 90 mg of CDI at room temperature, followed by stirring overnight. Separately, to a solution of 90 mg of 3-(aminosulfonly)propyl acetate in 2.0 ml of 1,4-dioxane was added 80 µL of 1,8-diazabicyclo[5,4,0]undec-7-ene, and the thus prepared reaction solution was added to the above-described reaction system at room temperature, followed by stirring at 60°C overnight. After cooling, water was added thereto and the precipitated solid was collected by filtration. The obtained solid was purified by silica gel column chromatography to obtain 120 mg of 3-{[(4-{2-[3,5-dichloro-2-oxo-6-{[(2S)-2-propylpyrrolidin-1-yl]methyl}pyridin-1(2H)-yl]ethyl}benzoyl)amino]sulfonly}propyl acetate.

### Example 52

To a solution of 800 mg of (1R,2R)-2-[(tert-butoxycarbonyl)(methyl)amino]cyclopentyl acetate in 4.0 ml of ethyl acetate was added 4.0 ml of 4 M hydrogen chloride-ethyl acetate at room temperature, followed by stirring overnight. The solvent was evaporated under reduced pressure to obtain 605 mg of (1R,2R)-2-(methylamino)cyclopentyl acetate hydrochloride as a colorless oily substance. A mixed solution of 157 mg of the obtained colorless oily substance, 150 mg oftert-butyl 4-{2-[6-(bromomethyl)-3,5-dichloro-2-oxopyridin-1(2H)-yl]ethyl}benzoate, 0.13 ml of triethylamine, and 5.0 ml of DMF was stirred at room temperature overnight. A saturated aqueous sodium hydrogen carbonate solution and ethyl acetate were added to the reaction solution to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 153 mg of pale yellow oily tert-butyl 4-{2-[6-({[(1R,2R)-2-acetoxycyclopentyl](methyl)amino}methyl)-3,5-dichloro-2-oxopyridin-1(2H)-yl]ethyl}benzoate.

### Example 53

To a solution of 150 mg of tert-butyl 4-{2-[6-({[(1R,2R)-2-acetoxycyclopentyl](methyl)amino}methyl)-3,5-dichloro-2-oxopyridin-1(2H)-yl]ethyl}benzoate in 2.0 ml of DCM was added 2.0 ml of trifluoroacetic acid at room temperature, followed by stirring for 5 hours. After the solvent was evaporated under reduced pressure, it was made into powders with ethyl acetate to obtain 20 mg of 4-{2-[6-({[(1R,2R)-2-acetoxycyclopentyl](methyl)amino}methyl)-3,5-dichloro-2-oxopyridin-1(2H)-yl]ethyl}benzoic acid trifluoroacetate as a white solid.

### Example 54

To a solution of 350 mg of methyl 4-{2-[5-bromo-6-{[(2R)-2-isobutylpyrrolidin-1-yl]methyl}-3-methyl-2-oxopyridin-1(2H)-yl]ethyl}benzoate in 7.0 ml of toluene was added 1.8 ml of water at room temperature, and then 120 mg of a 2,4,6-trivinylcyclotriboroxane/pyridine complex, 304 mg of tripotassium phosphate, 16 mg of palladium acetate, and 59 mg of dicyclohexyl(2',6'-dimethoxybiphenyl-2-yl)phosphine were added thereto, followed by stirring at 90°C overnight. After cooling, water and ethyl acetate were added thereto, filtration was conducted using Celite, and a liquid separation operation was carried out. The organic layer was washed with a saturated aqueous sodium chloride solution and then dried over anhydrous sodium sulfate, and the solvent was evaporated under reduced pressure. The residue was purified by silica gel column chromatography to obtain 60 mg of methyl 4-{2-[6-{[(2R)-2-isobutylpyrrolidin-1-yl]methyl}-3-methyl-2-oxo-5-vinylpyridin-(2H)-yl]ethyl}benzoate.

The Example Compounds 55 to 352 shown in Tables below were prepared in the same production processes as the Examples 1 to 54 using the respective corresponding starting materials. The structures, the production processes, and the physicochemical data of each of the Example Compounds are shown in Tables 25 to 100.

Furthermore, the structures of the other compounds of the present invention are shown in Table 101. These can be easily synthesized by using the preparation methods as described above, or the methods described in Examples and methods obvious to a skilled person in the art, or modified methods thereof.

**[Table 8]**

| Pre | PSyn | Str | Dat |
|---|---|---|---|
| 1 | 1 | 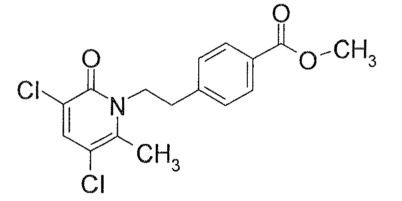 | FAB+ : 340, 342, 344 |
| 2 | 2 | 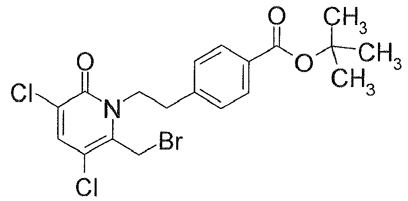 | FAB+ : 460, 462, 464, 466 |
| 3 | 3 | 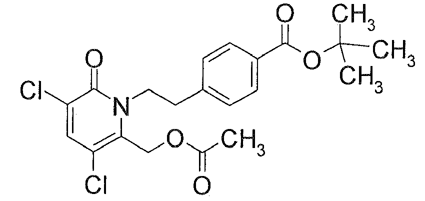 | FAB+ : 440, 442, 444 |
| 4 | 4 | 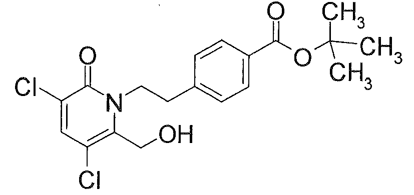 | FAB+ : 398, 400, 402 |
| 5 | 5 | 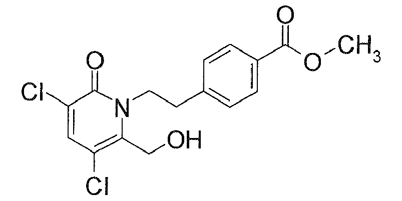 | ESI+: 356, 358, 360 |
| 6 | 6 | 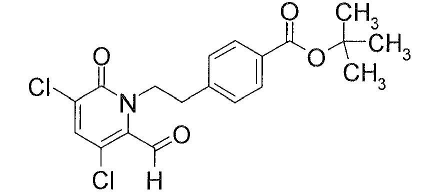 | FAB+ : 396, 398, 340 |

**[Table 9]**

| | | | |
|---|---|---|---|
| 7 | 7 | 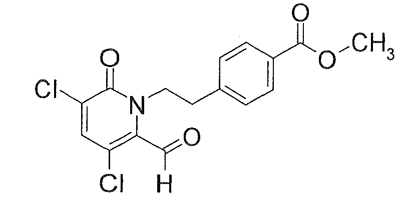 | EI : 353, 355 |
| 8 | 8 | 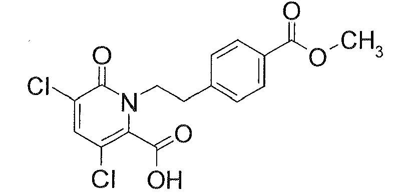 | FAB-: 368 |
| 9 | 9 | 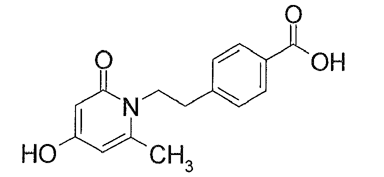 | ESI+: 274 |
| 10 | 10 | 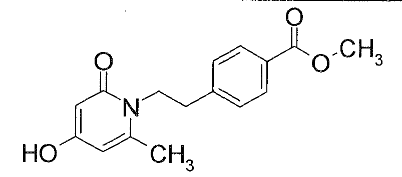 | ESI+: 288 |
| 11 | 11 | 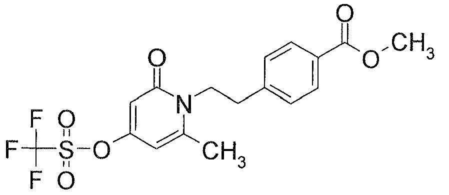 | EI: 419 |
| 12 | 12 | 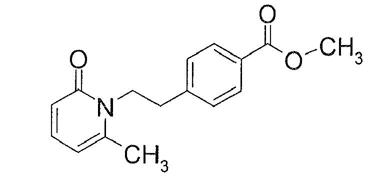 | ESI+: 272 |
| 13 | 13 | 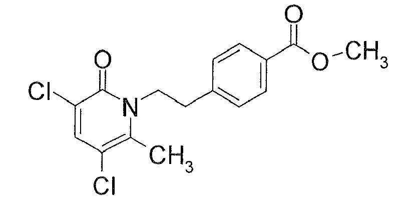 | ESI+: 340, 342, 344 |

**[Table 10]**

| | | | |
|---|---|---|---|
| 14 | 14 | 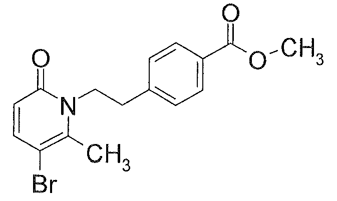 | EI : 349, 351 |
| 15 | 15 | 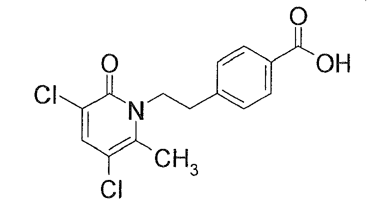 | ESI+: 326, 328, 330 |
| 16 | 16 | 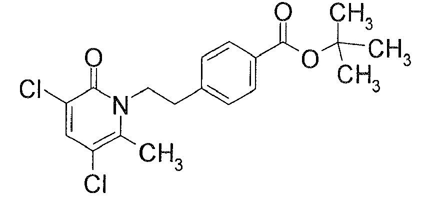 | ESI+: 382, 384, 386 |
| 17 | 17 | 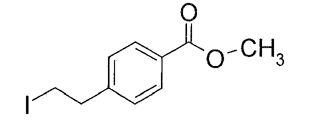 | CI+: 291 |
| 18 | 18 | 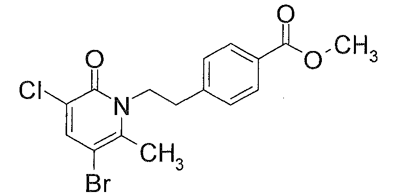 | ESI+: 384,386 |
| 19 | 19 | 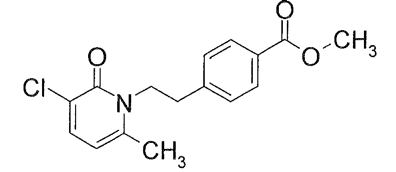 | ESI+: 306, 308 |
| 20 | 20 | 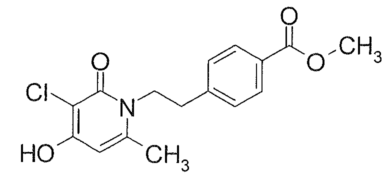 | ESI+: 322, 324 |

**[Table 11]**

| | | | |
|---|---|---|---|
| 21 | 21 | 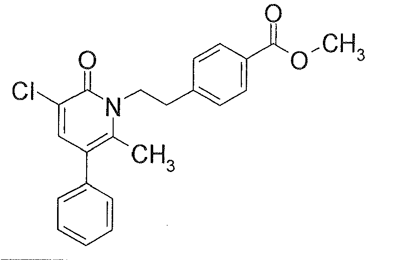 | ESI+:382,384 |
| 22 | 22 | 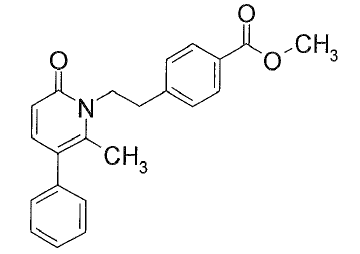 | ESI+: 348 |
| 23 | 23 | 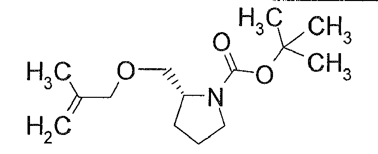 | ESI+: 256 |
| 24 | 24 | 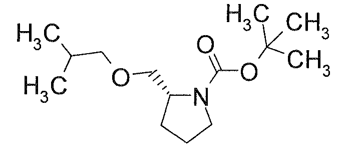 | ESI+: 258 |
| 25 | 25 | 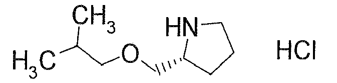 | ESI+: 158 |
| 26 | 26 | 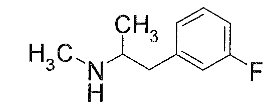 | FAB+: 168 |
| 27 | 27 | 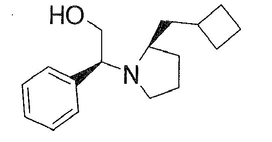 | ESI+: 260 |
| 28 | 28 | 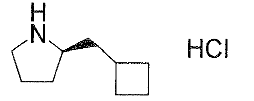 | ESI+: 140 |

**[Table 12]**

| | | | |
|---|---|---|---|
| 29 | 29 | 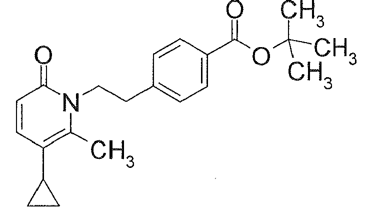 | ESI+: 354 |
| 30 | 30 | 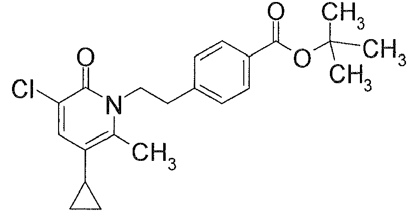 | ESI+: 388, 340 |
| 31 | 31 | 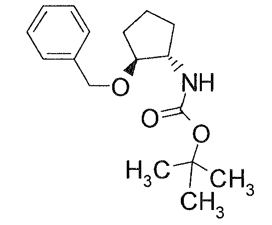 | ESI+: 292 |
| 32 | 32 | 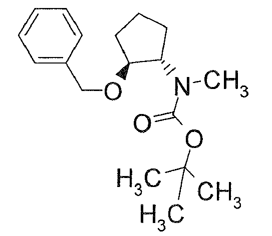 | ESI+: 306 |
| 33 | 33 | 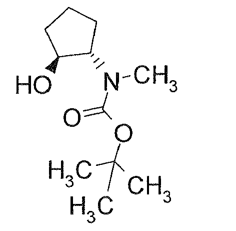 | ESI+: 216 |

**[Table 13]**

| | | | |
|---|---|---|---|
| 34 | 34 | 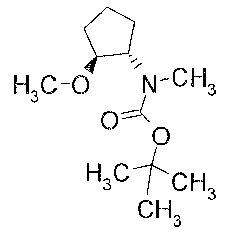 | ESI+: 230 |
| 35 | 35 | 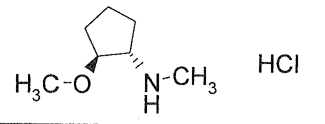 | ESI+: 130 |
| 36 | 36 | 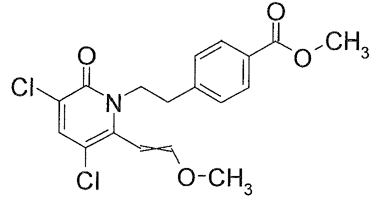 | ESI+: 382 |
| 37 | 37 | 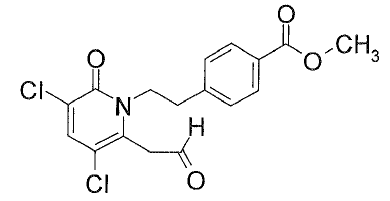 | ESI+: 368 |
| 38 | 38 | 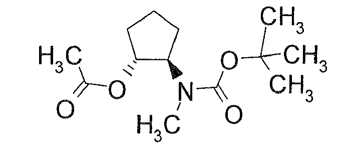 | ESI+: 258 |
| 39 | 39 | 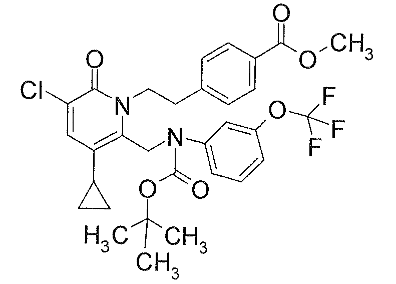 | ESI+: 621 |

**[Table 14]**

| | | | |
|---|---|---|---|
| 40 | 40 | 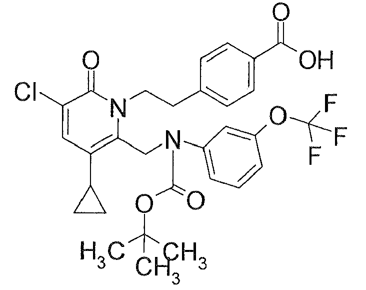 | ESI+: 607, 609 |
| 41 | 41 | 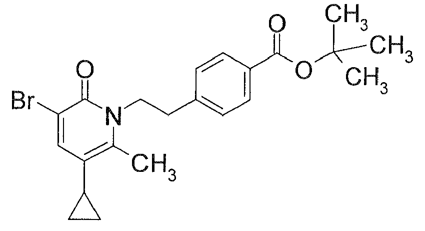 | ESI+: 432, 434 |
| 42 | 42 | 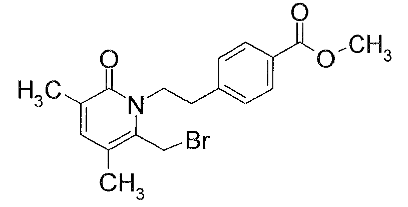 | ESI+: 378 |
| 43 | 43 | 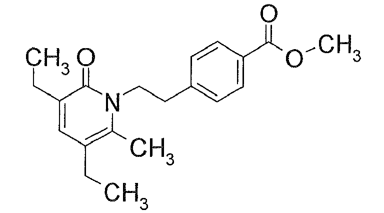 | ESI+: 328 |
| 44 | 44 | 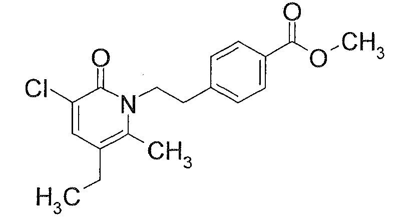 | ESI+: 334, 336 |

**[Table 15]**

| | | | |
|---|---|---|---|
| 45 | 45 | 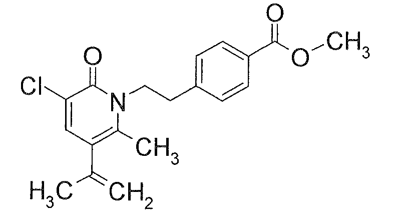 | ESI+: 346, 348 |
| 46 | 46 | 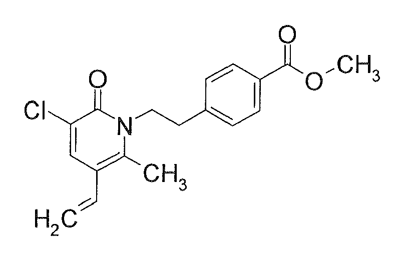 | APCI/ESI+: 332 |
| 47 | 47 | 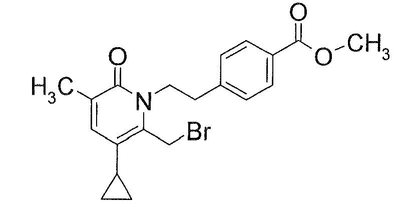 | ESI+: 404, 406 |
| 48 | 48 | 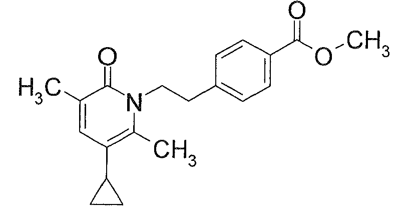 | ESI+: 326 |
| 49 | 49 | 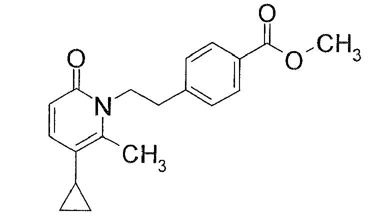 | ESI+: 312 |

**[Table 16]**

| | | | |
|---|---|---|---|
| 50 | 50 | 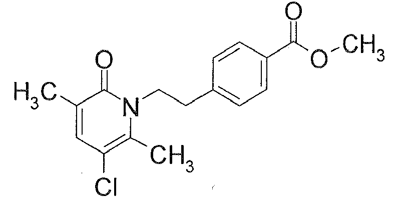 | ESI+: 320 |
| 51 | 51 | 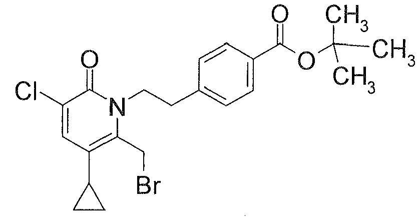 | ESI+; 466, 468 |
| 52 | 52 | 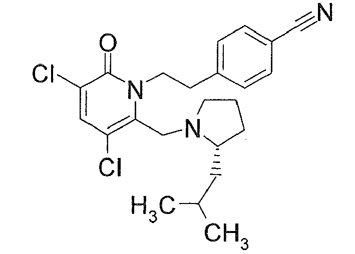 | ESI+: 432 |
| 53 | 53 | 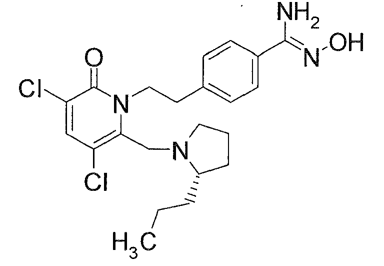 | ESI+: 451, 453 |
| 54 | 54 | 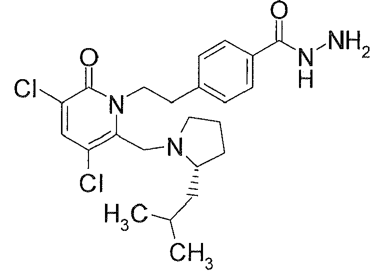 | ESI+; 465, 467 |

**[Table 17]**

| | | | |
|---|---|---|---|
| 55 | 55 | 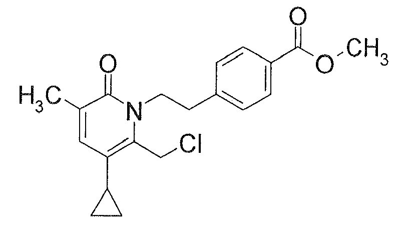 | ESI+: 360 |
| 56 | 56 | 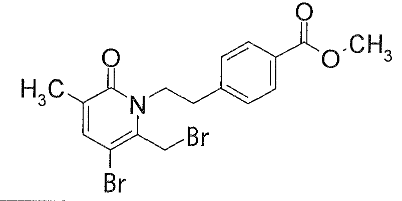 | ESI+: 442, 444, 446 |
| 57 | 2 | 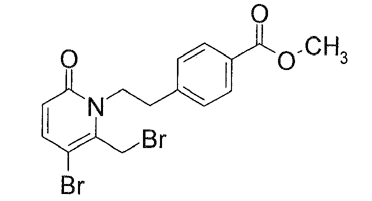 | FAB+ : 428, 430, 432 |
| 58 | 2 | 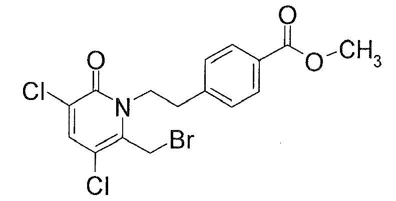 | ESI+: 418, 420, 422, 424 |
| 59 | 3 | 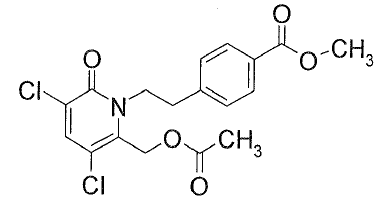 | ESI+: 398, 400, 402 |
| 60 | 6 | 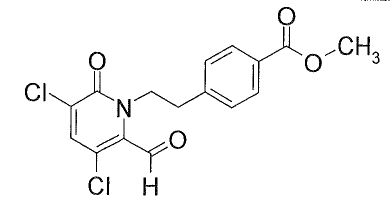 | EI: 353, 355, 357 |

**[Table 18]**

| | | | |
|---|---|---|---|
| 61 | 52 | 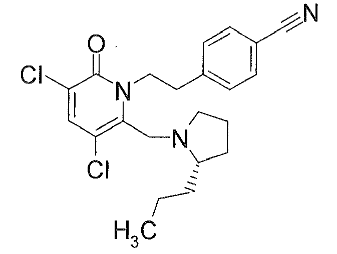 | ESI+: 418, 420, 422 |
| 62 | 51 | 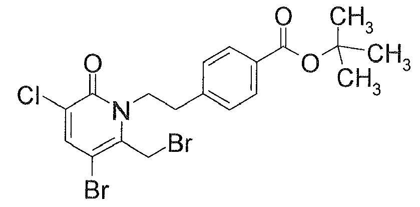 | ESI+: 504, 506, 508 |
| 63 | 51 | 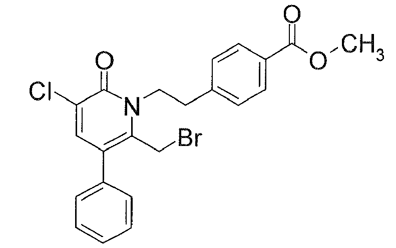 | ESI+: 460, 462 |
| 64 | 15 | 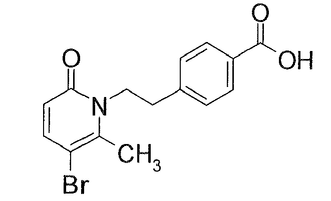 | ESI+: 336 |
| 65 | 16 | 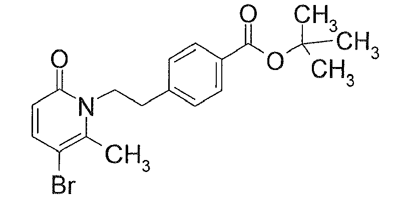 | ESI+: 392, 394 |
| 66 | 51 | 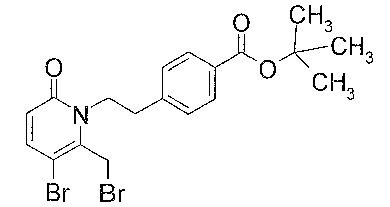 | ESI+: 472 |

**[Table 19]**

| | | | |
|---|---|---|---|
| 67 | 54 | 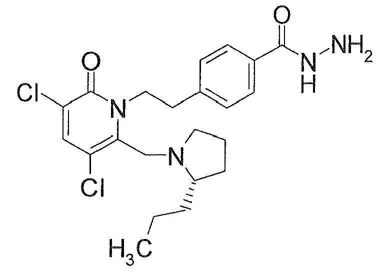 | ESI+: 451, 453 |
| 68 | 11 | 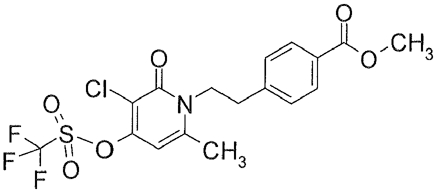 | ESI+: 454, 456 |
| 69 | 27 | 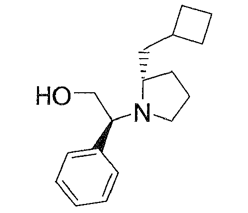 | ESI+: 260 |
| 70 | 53 | 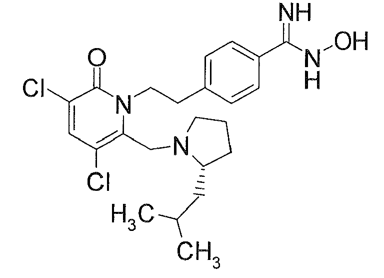 | ESI+: 465, 467 , 469 |
| 71 | 31 | 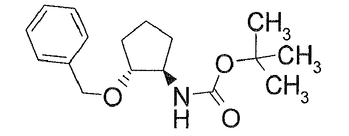 | ESI+: 292 |
| 72 | 32 | 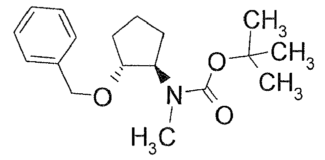 | ESI+: 306 |

**[Table 20]**

| | | | |
|---|---|---|---|
| 73 | 35 | 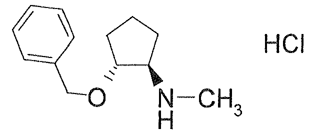 | ESI+: 206 |
| 74 | 33 | 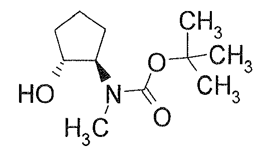 | ESI+: 216 |
| 75 | 54 | 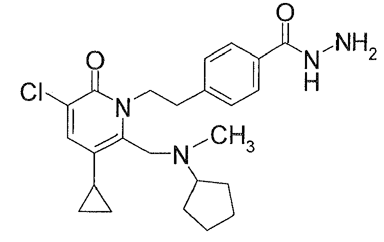 | ESI+: 443 |
| 76 | 54 | 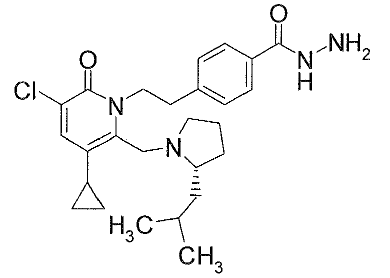 | ESI+: 471 |
| 77 | 51 | 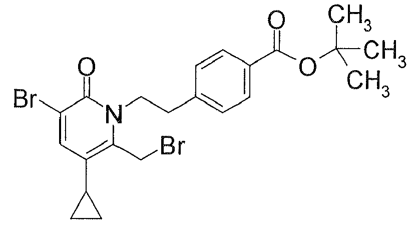 | ESI+: 510, 512, 514 |
| 78 | 51 | 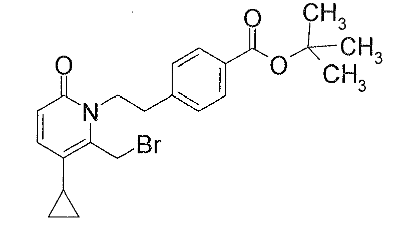 | ESI+: 432 |

**[Table 21]**

| | | | |
|---|---|---|---|
| 79 | 29 | 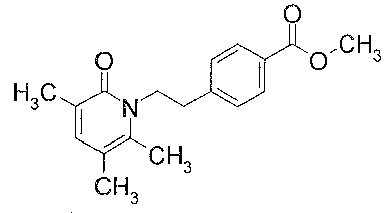 | ESI+: 300 |
| 80 | 42 | 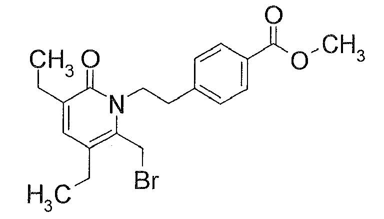 | ESI+: 406 |
| 81 | 44 | 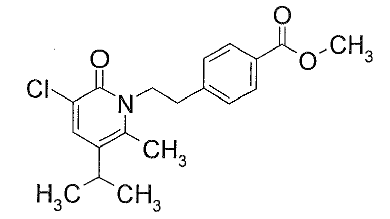 | ESI+:348,350 |
| 82 | 41 | 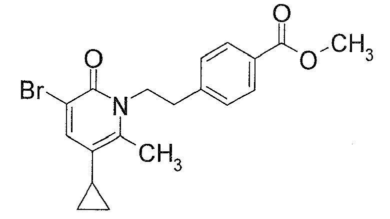 | ESI+: 390 |
| 83 | 2 | 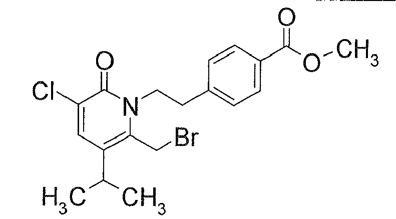 | ESI+: 426, 428, 430 |
| 84 | 31 | 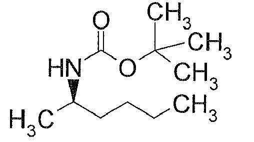 | ESI+: 202 |

**[Table 22]**

| | | | |
|---|---|---|---|
| 85 | 31 | 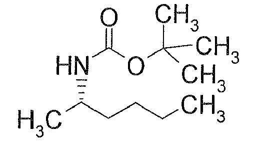 | ESI+: 202 |
| 86 | 32 | 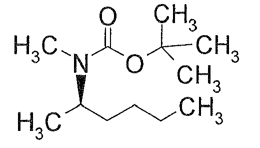 | ESI+: 216 |
| 87 | 32 | 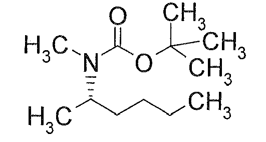 | ESI+: 216 |
| 88 | 35 | 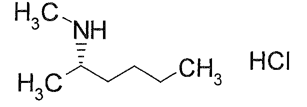 | ESI+: 116 |
| 89 | 35 | 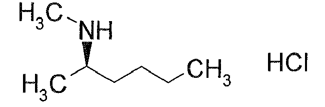 | ESI+: 116 |
| 90 | 42 | 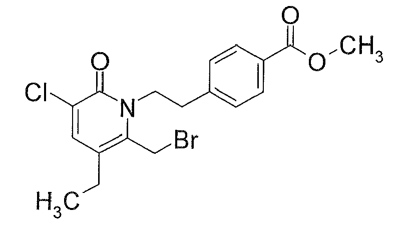 | ESI+: 414, 416 |
| 91 | 42 | 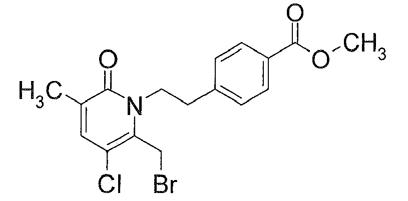 | ESI+: 398, 400 |

**[Table 23]**

| | | | |
|---|---|---|---|
| 92 | 54 | 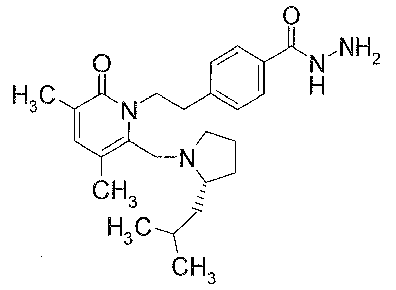 | ESI+: 425 |
| 93 | 54 | 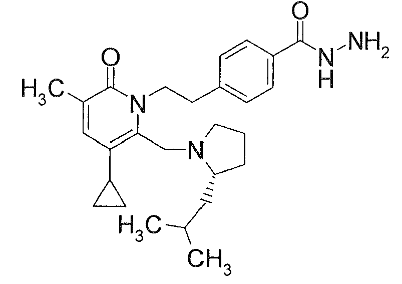 | APCI/ESI+: 451 |
| 94 | 15 | 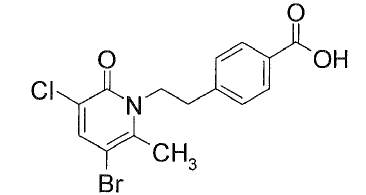 | ESI+:370,372,374 |
| 95 | 16 | 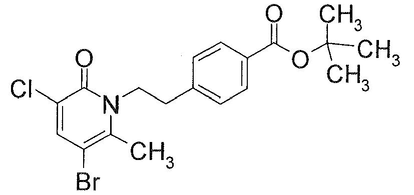 | ESI+: 426, 428, 430 |
| 96 | 9 | 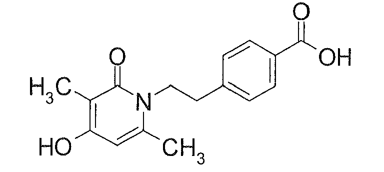 | ESI+: 288 |
| 97 | 10 | 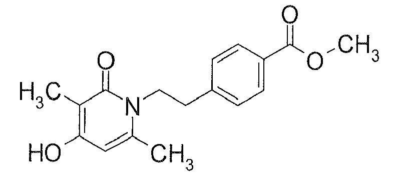 | ESI+: 302 |

**[Table 24]**

| | | | |
|---|---|---|---|
| 98 | 11 | 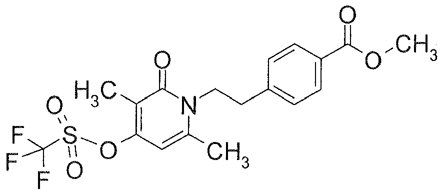 | ESI+: 434 |
| 99 | 12 | 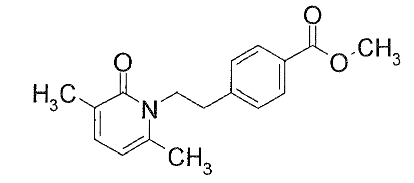 | ESI+: 286 |

**[Table 25]**

| Ex | Syn | Str | Dat |
|---|---|---|---|
| 1 | 1 | 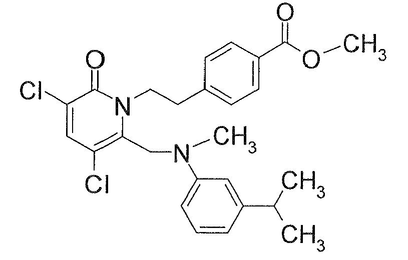 | ESI+: 487, 489, 491 |
| 2 | 2 | 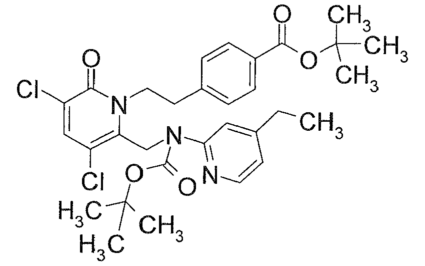 | ESI+: 602 |
| 3 | 3 | 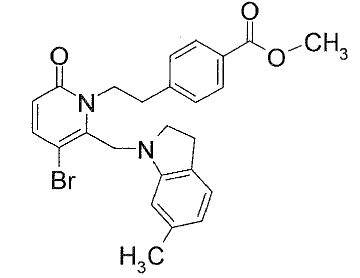 | ESI+: 481, 483 |
| 4 | 4 | 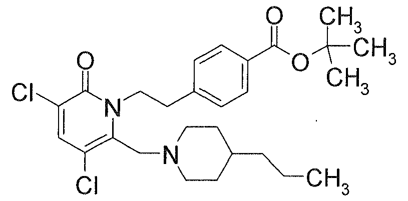 | ESI+: 507, 509 |
| 5 | 5 | 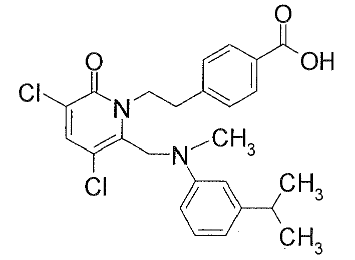 | ESI+: 473, 475, 477 |

**[Table 26]**

| | | | |
|---|---|---|---|
| 6 | 6 | 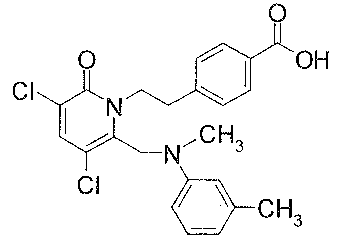 | FAB+: 445 |
| 7 | 7 | 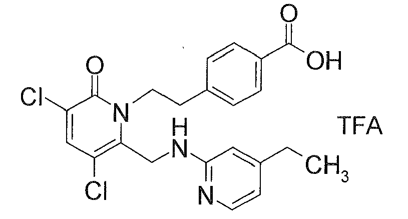 | ESI+: 446, 448 |
| 8 | 8 | 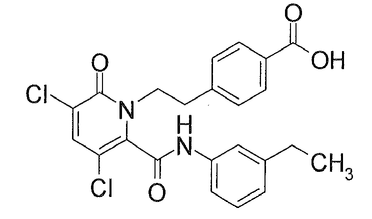 | FAB+: 459 |
| 9 | 9 | 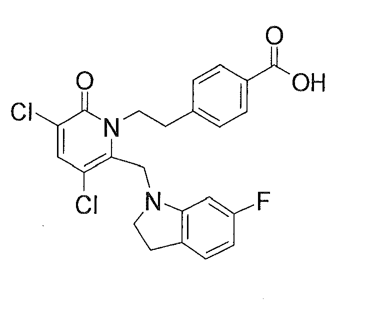 | NMR(300 MHz): 2.86 (2H, t, J = 8.1 Hz), 2.97-3.08 (2H, m), 3.34 (2H, t, J = 8.1 Hz), 4.08-4.20 (2H, m), 4.49 (2H, s), 6.46 (1H, ddd, J = 2.1, 7.8, 10.2 Hz), 6.68 (1H, dd, J = 2.1, 10.8 Hz), 7.09 (1H, dd, J =6.0, 7.8 Hz), 7.16 (2H, d, J = 8.1 Hz), 7.79 (2H, d, J = 8.1 Hz), 8.09 (1H, s), 12.88 (1H, s); ESI+: 461, 463; Elemental Analysis: Found(C:59.78, H:4.13, N:6.03, Cl:15.37, F:4.19), Calc(C:59.88, H:4.15, N:6.07, Cl:15.37, F:4.12) |

**[Table 27]**

| | | | |
|---|---|---|---|
| 10 | 10 | 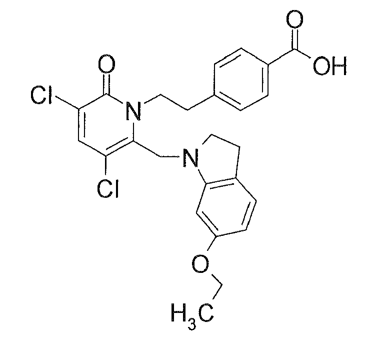 | NMR(300 MHz): 1.29 (3H, t, J = 6.9 Hz), 2.79 (2H, t, J = 8.1 Hz), 2.98-3.09 (2H, m), 3.26 (2H, t, J = 8.1 Hz), 3.92 (2H, q, J = 6.9 Hz), 4.13-4.24 (2H, m), 4.43 (2H, s), 6.24 (1H, dd, J = 2.1, 8.1 Hz), 6.40 (1H, d, J = 2.1 Hz), 6.97 (1H, d, J = 8.1 Hz), 7.19 (2H, d, J = 8.1 Hz), 7.78 (2H, d, J = 8.1 Hz), 8.09 (1H, s), 12.87 (1H, s); ESI+: 487, 489 |
| 11 | 11 | 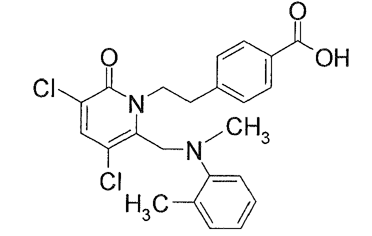 | ESI+: 445, 447 |
| 12 | 12 | 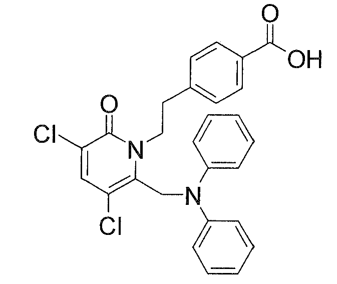 | FAB+: 493 |
| 13 | 13 | 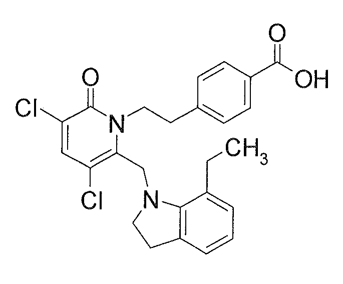 | NMR(300 MHz): 1.06 (3H, t, J = 7.5 Hz), 2.56 (2H, q, J = 7.5 Hz), 2.85 (2H, t, J = 8.1 Hz), 3.06-3.18 (4H, m), 4.42 (2H, s), 4.52-4.62 (2H, m), 6.80 (1H, dd, J = 7.2, 7.2 Hz), 6.93 (1H, d, J = 7.2 Hz), 7.01 (1H, d, J = 7.2 Hz), 7.27 (2H, d, J = 8.1 Hz), 7.84 (2H, d, J = 8.1 Hz), 8.10 (1H, s); ESI-: 469, 471, 473 |

**[Table 28]**

| | | | |
|---|---|---|---|
| 14 | 14 | 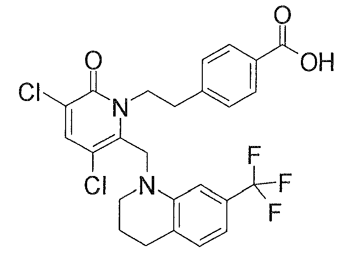 | FAB+: 525 |
| 15 | 15 | 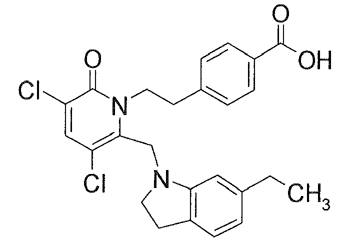 | ESI+: 471, 473, 475 |
| 16 | 16 | 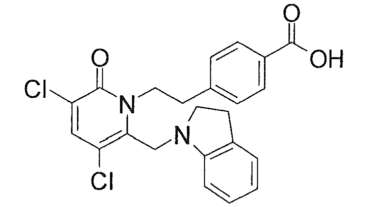 | ESI+: 443, 445, 447 |
| 17 | 17 | 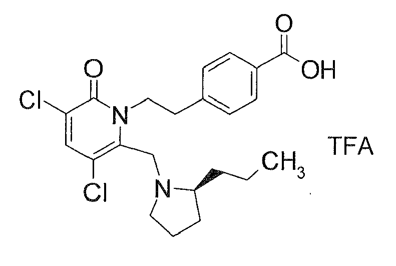 | NMR(300 MHz): 0.74-0.96 (br), 1.07-2.12 (br), 2.93-3.13 (2H, m), 3.40-4.80 (br), 7.39 (2H, d, J = 8.1 Hz), 7.91 (2H, d, J = 8.1 Hz), 8.08 (br); APCI+: 437, 439; Elemental Analysis: Found(C:51.96,H:4.82,N: 5.15,Cl:12.75,F:10.22), Calc(C:52.28,H:4.94,N:5. 08,Cl:12.86,F:10.34) |

**[Table 29]**

| | | | |
|---|---|---|---|
| 18 | 18 | 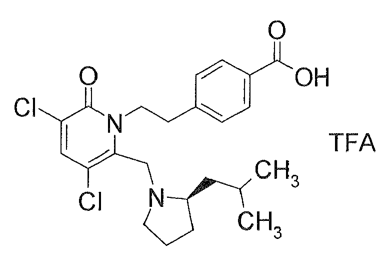 | NMR(300MHz): 0.76-0.92 (br), 1.20-2.30 (br), 2.96-3.10 (2H, m), 4.27-4.60 (br), 7.39 (2H, d, J = 8.1 Hz), 7.91 (2H, d, J = 8.1 Hz), 8.10 (br); ESI+: 451, 453; Elemental Analysis: Found(C:52.83,H:5.01,N: 4.97,Cl:12.67,F:10.24), Calc(C:53.11,H:5.17,N:4. 95,Cl:12.54,F:10.08) |
| 19 | 19 | 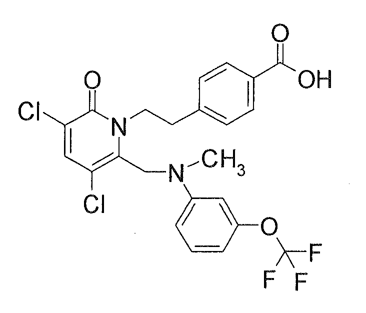 | NMR(400 MHz): 2.77 (3H, s), 2.86-2.93 (2H, m), 3.95-4.06 (2H, m), 4.67 (2H, s), 6.78 (1H, d, J = 8.0 Hz), 6.93-6.96 (1H, m), 6.98 (2H, d, J = 8.0 Hz), 7.02 (1H, dd, J = 8.0, 2.0 Hz), 7.37 (1H, dd, J = 8.0, 8.0 Hz), 7.72 (2H, d, J = 8.0 Hz), 8.11 (1H, s), 12.87 (1H, s); ESI+: 515, 517, 519 |
| 20 | 20 | 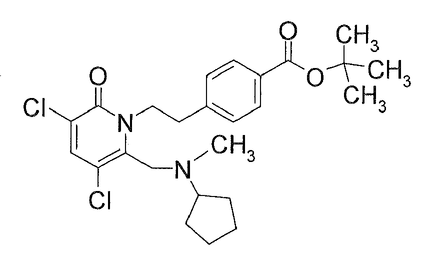 | NMR(400 MHz, CDCl3): 1.60 (9H, s), 1.38-1.73 (6H, m), 1.78-1.88 (2H, m), 2.15 (3H, s), 2.76 (1H, quintet, J=7.2 Hz), 3.05-3.14 (2H, m), 3.59 (2H, s), 4.49-4.56 (2H, m), 7.31 (2H, d, J = 8.4 Hz), 7.57 (1H, s), 7.93 (2H, d, J = 8.4 Hz) |
| 21 | 21 | 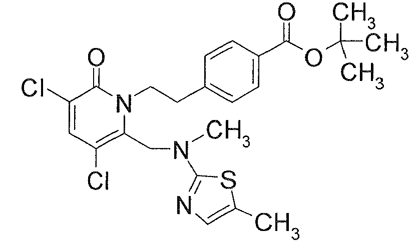 | ESI+: 508 |

**[Table 30]**

| | | | |
|---|---|---|---|
| 22 | 22 | 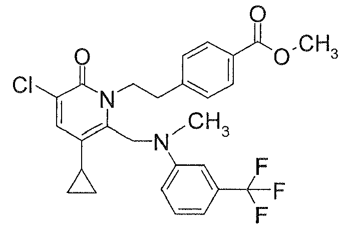 | ESI+: 519, 521 |
| 23 | 23 | 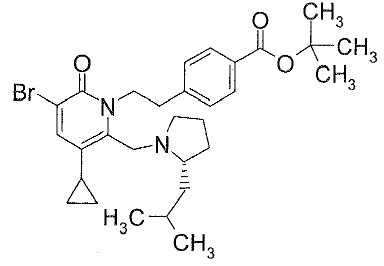 | ESI+: 557, 559 |
| 24 | 24 | 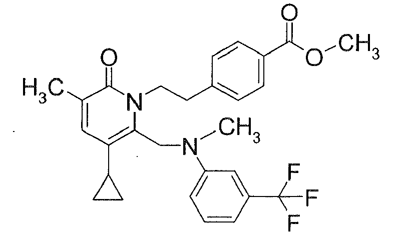 | ESI+: 499 |
| 25 | 25 | 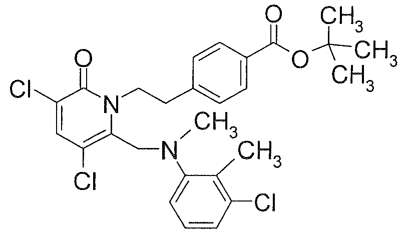 | ESI+: 535 |
| 26 | 26 | 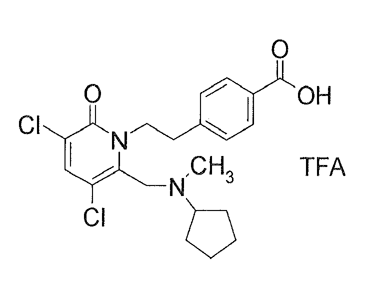 | NMR(400 MHz): 1.45-1.72 (6H, br), 1.80-2.00 (2H, br), 2.20-2.60 (2H, br), 3.02 (2H, dd, J = 7.6, 7.6 Hz), 3.60-5.20 (br), 7.36 (2H, d, J = 8.0 Hz), 7.91 (2H, d, J = 8.0 Hz), 8.07 (1H, s); ESI+: 423; Elemental Analysis: Found(C:51.23,H:4.70,N: 5.15,Cl:13.20,F:10.82), Calc(C:51.41,H:4.69,N:5.2 1,Cl:13.20,F:10.61) |

**[Table 31]**

| | | | |
|---|---|---|---|
| 27 | 27 | 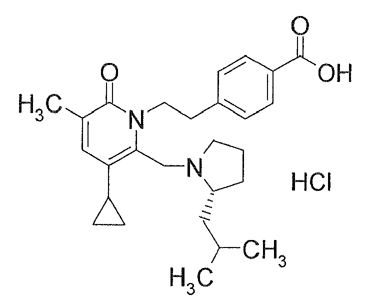 | NMR(400MHz): 0.52-0.74 (2H, m), 0.80-1.02 (8H, m),1.42-1.76 (4H, m), 1.82-2.13 (3H, m), 2.05 (3H, s), 2.16-2.30 (1H, m), 2.84-3.03 (2H, dd, J = 7.6 Hz, 7.6 Hz), 3.16-4.76 (m), 7.07 (1H, s), 7.38 (2H, d, J = 8.0 Hz), 7.89 (2H, d, J = 8.0 Hz), 10.38 (br), 12.8 (br); ESI+: 437 |
| 28 | 28 | 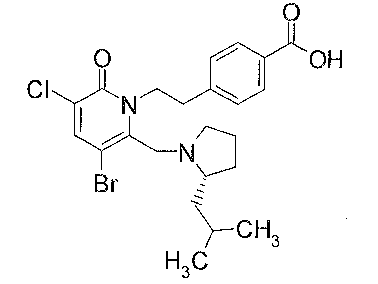 | ESI+: 495, 497 |
| 29 | 29 | 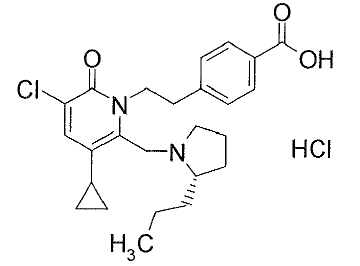 | ESI+: 443, 445 |
| 30 | 30 | 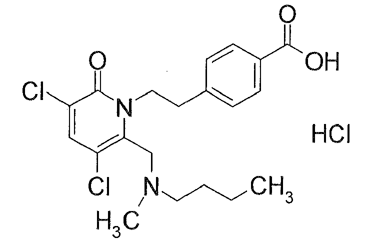 | FAB+: 411, 413, 415 |

**[Table 32]**

| | | | |
|---|---|---|---|
| 31 | 31 | 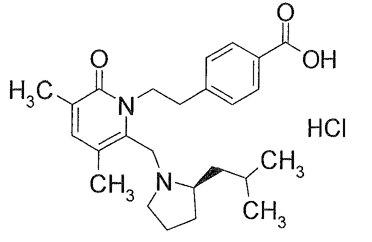 | ESI+: 411 |
| 32 | 32 | 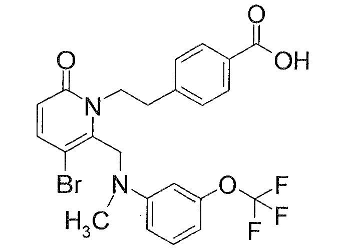 | ESI+: 525, 527 |
| 33 | 33 | 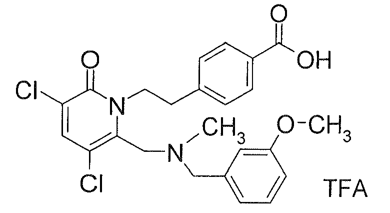 | ESI+: 475, 477, 479 |
| 34 | 34 | 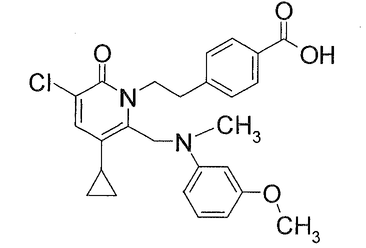 | ESI+: 467 |
| 35 | 35 | 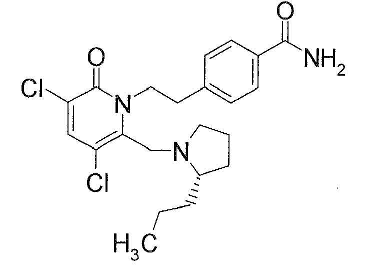 | ESI+: 436, 438 |

**[Table 33]**

| | | | |
|---|---|---|---|
| 36 | 36 | 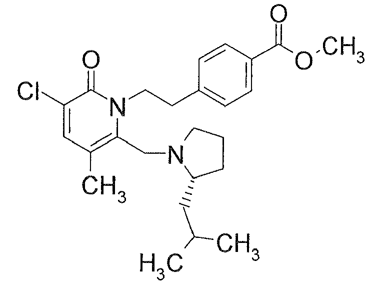 | ESI+: 445, 447 |
| 37 | 37 | 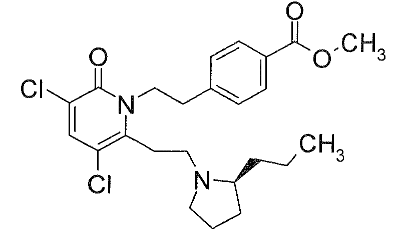 | ESI+: 465 |
| 38 | 38 | 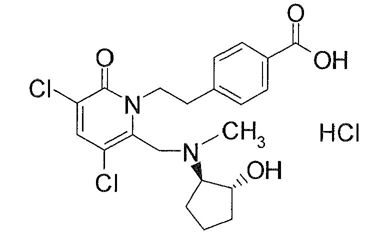 | ESI+: 439 |
| 39 | 39 | 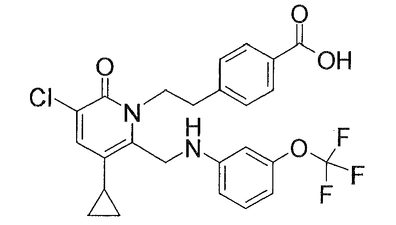 | ESI+: 507, 509 |
| 40 | 40 | 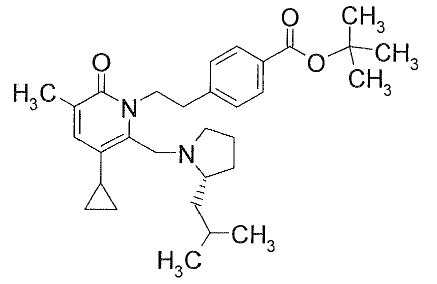 | ESI+: 493 |

**[Table 34]**

| | | | |
|---|---|---|---|
| 41 | 41 | 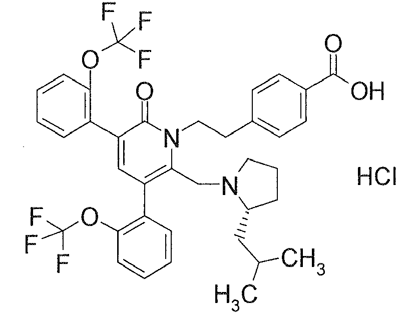 | ESI+: 577 |
| 42 | 42 | 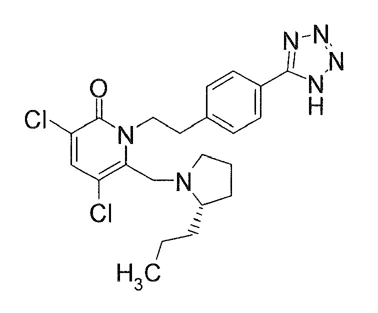 | NMR(400MHz): 0.81 (3H, t, J = 7.2 Hz), 0.94-1.08 (1H, m), 1.11-1.30 (2H, m), 1.30-1.42 (1H, m), 1.50-1.69 (3H, m), 1.85-1.97 (1H, m), 2.25-2.43 (2H, m), 2.79 (1H, t, J = 7.2 Hz), 3.07 (2H, dd, J = 6.8, 6.8 Hz), 3.58 (1H, d, J =13.6Hz), 3.91 (1H, d, J = 13.6 Hz), 4.35-4.47 (1H, m), 4.48-4.62 (1H, m), 7.47 (2H, d, J = 8.0 Hz), 8.00 (1H, s), 8.01 (2H, d, J = 8.0 Hz); ESI+: 461, 463 |
| 43 | 43 | 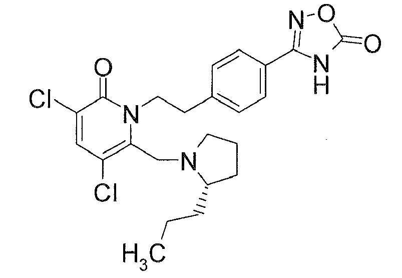 | ESI+: 477, 479, 481 |
| 44 | 44 | 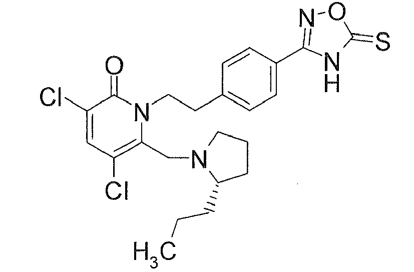 | ESI+: 493, 495 |

**[Table 35]**

| | | | |
|---|---|---|---|
| 45 | 45 | 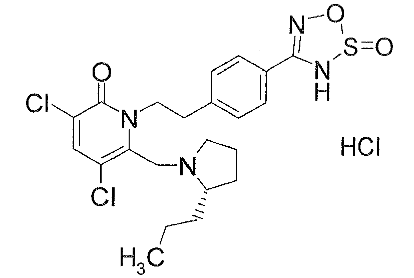 | ESI+: 497, 499 |
| 46 | 46 | 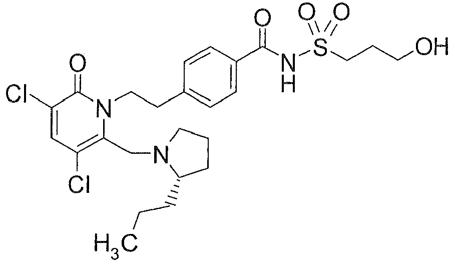 | ESI+: 558, 560, 562 |
| 47 | 47 | 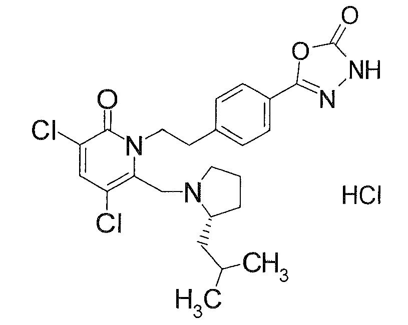 | ESI+: 491, 493, 495 |
| 48 | 48 | 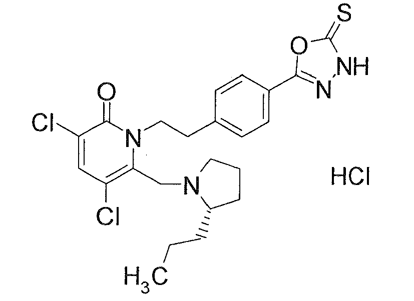 | ESI+: 493, 495 |

**[Table 36]**

| | | | |
|---|---|---|---|
| 49 | 49 | 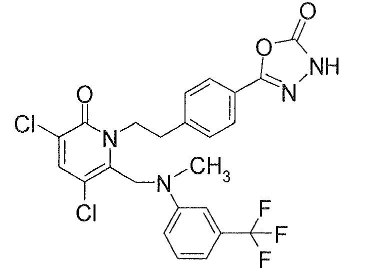 | ESI+: 539, 541 |
| 50 | 50 | 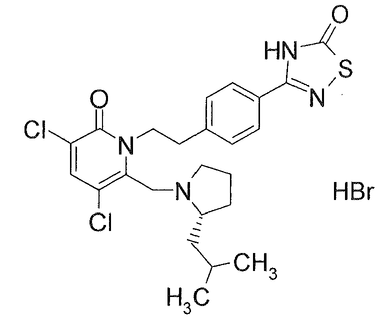 | ESI+: 507, 509, 511 |
| 51 | 51 | 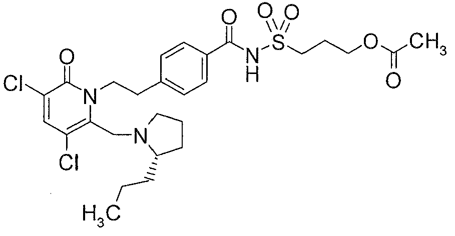 | ESI+: 600, 602 |
| 52 | 52 | 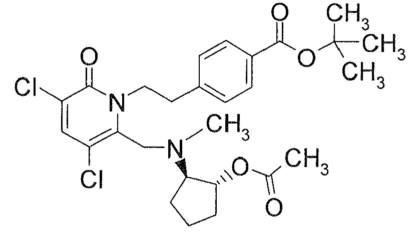 | ESI+: 537 |
| 53 | 53 | 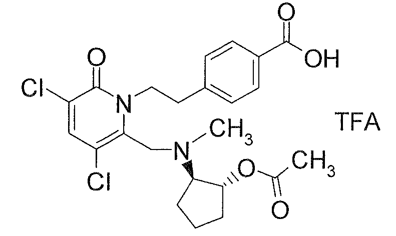 | ESI+: 481 |

**[Table 37]**

| | | | |
|---|---|---|---|
| 54 | 54 | 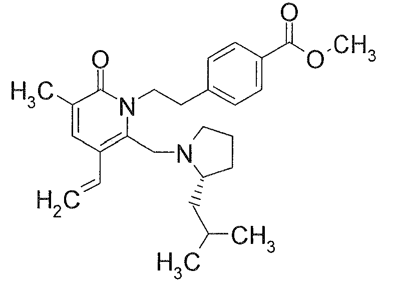 | ESI+: 437 |
| 55 | 1 | 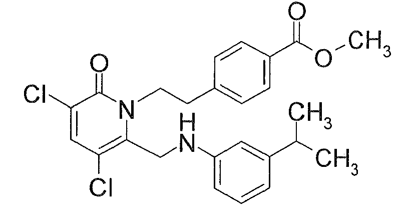 | ESI+: 473, 475, 477 |
| 56 | 1 | 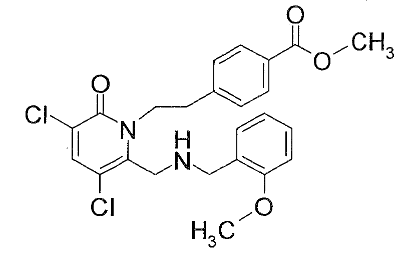 | FAB+: 475 |
| 57 | 1 | 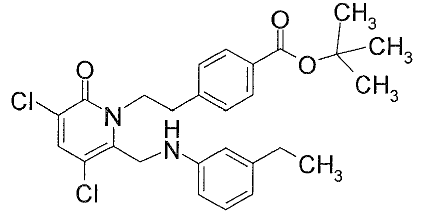 | ESI+: 501, 503 |
| 58 | 1 | 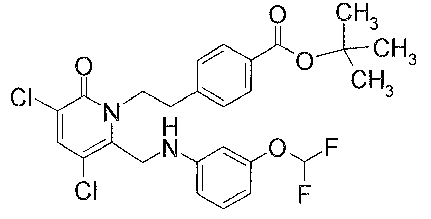 | ESI+: 539, 541 |

**[Table 38]**

| | | | |
|---|---|---|---|
| 59 | 1 | 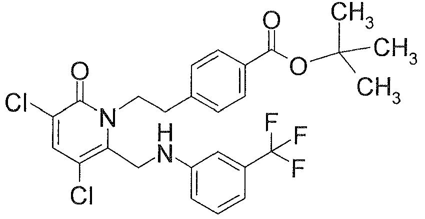 | FAB+: 540 |
| 60 | 1 | 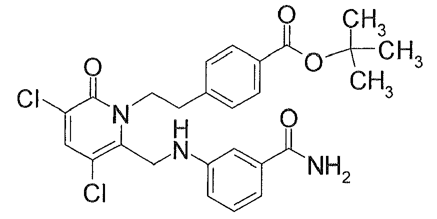 | ESI-: 514 |
| 61 | 1 | 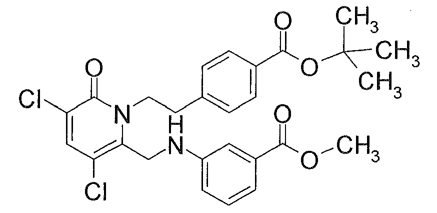 | ESI+: 531 |
| 62 | 1 | 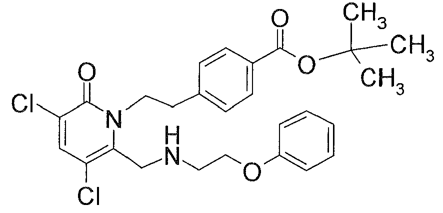 | ESI+: 517, 519 |
| 63 | 1 | 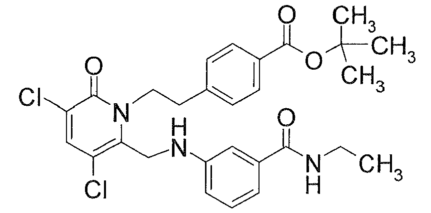 | ESI+: 544, 546 |
| 64 | 1 | 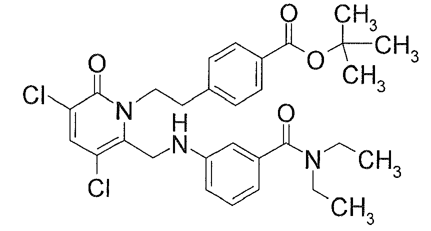 | ESI+: 572, 574, 576 |

**[Table 39]**

| | | | |
|---|---|---|---|
| 65 | 1 | 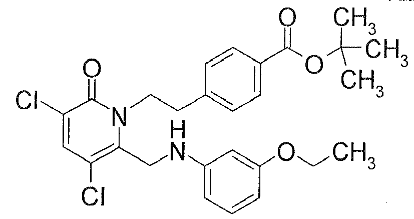 | ESI+: 517, 519, 521 |
| 66 | 1 | 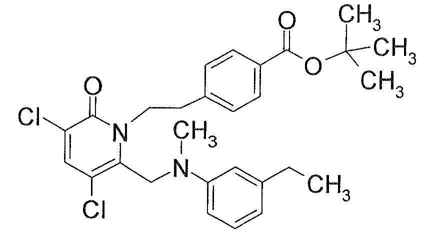 | ESI+: 515, 517 |
| 67 | 1 | 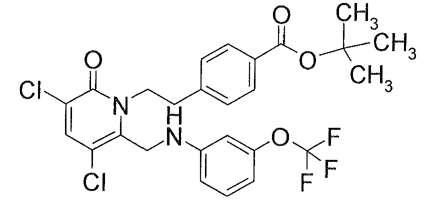 | FAB+: 556 |
| 68 | 1 | 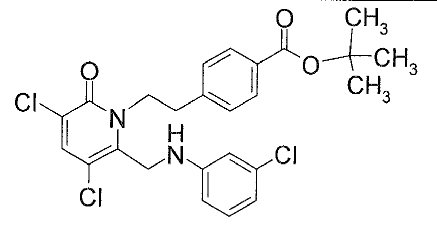 | FAB+: 508 |
| 69 | 1 | 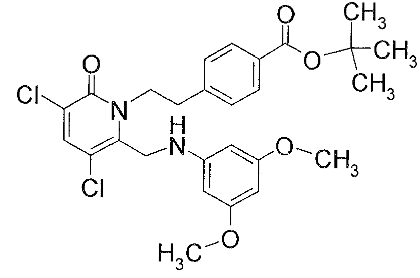 | ESI+: 533, 535 |

**[Table 40]**

| | | | |
|---|---|---|---|
| 70 | 1 | 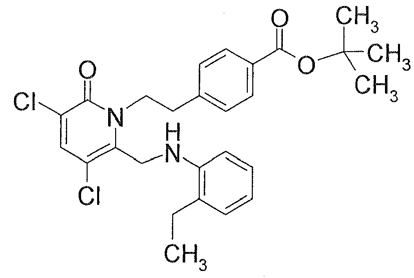 | FAB-: 499 |
| 71 | 1 | 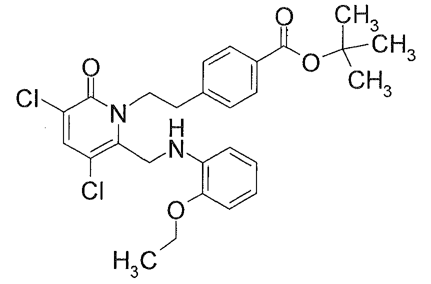 | ESI+: 517, 519 |
| 72 | 1 | 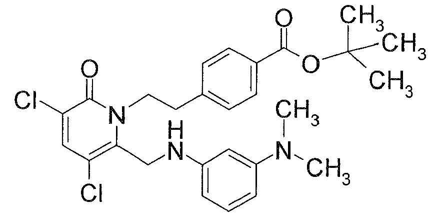 | ESI+: 516, 518, 520 |
| 73 | 1 | 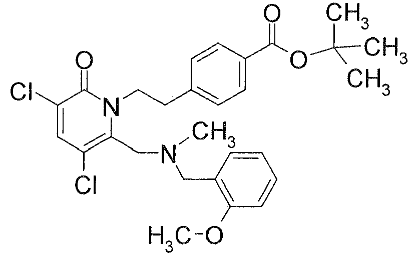 | ESI+: 531 |
| 74 | 1 | 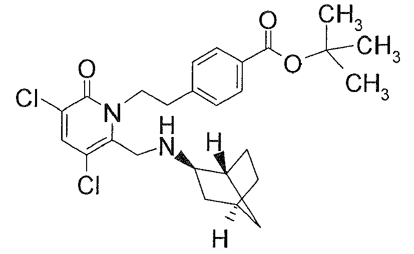 | ESI+: 491 |

**[Table 41]**

| | | | |
|---|---|---|---|
| 75 | 1 | 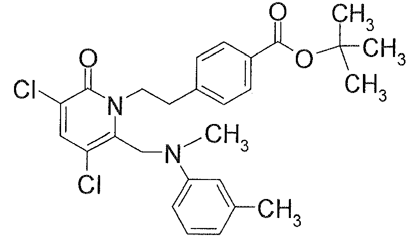 | ESI+: 501, 503, 505 |
| 76 | 1 | 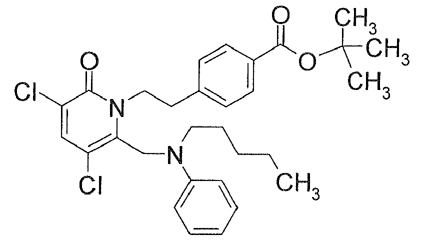 | ESI+: 543, 545 |
| 77 | 1 | 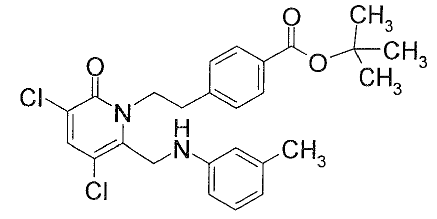 | ESI+: 487, 489, 491 |
| 78 | 1 | 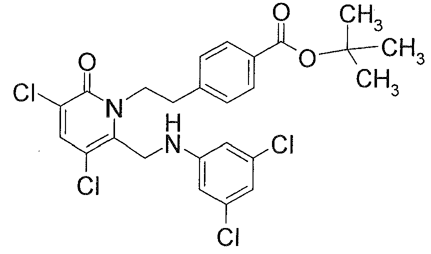 | FAB+: 542 |
| 79 | 1 | 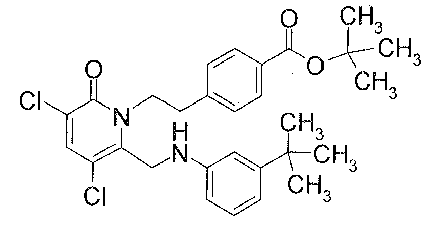 | ESI+: 529, 531 |

**[Table 42]**

| | | | |
|---|---|---|---|
| 80 | 1 | 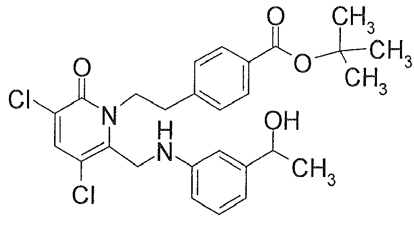 | ESI+: 517, 519 |
| 81 | 1 | 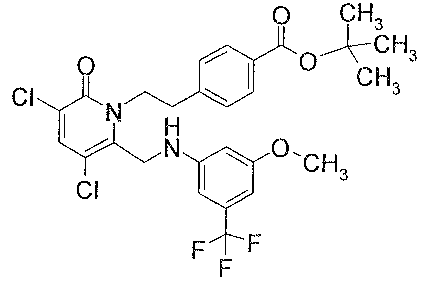 | ESI+: 571 |
| 82 | 1 | 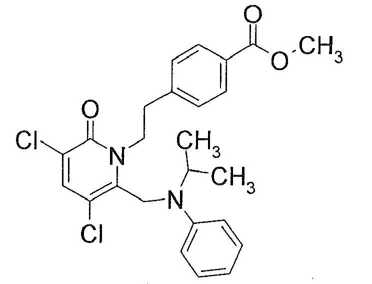 | ESI-: 471 |
| 83 | 1 | 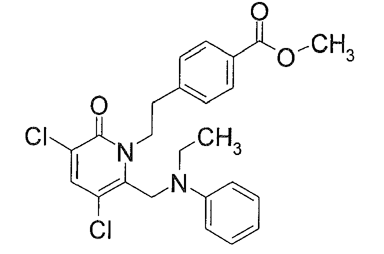 | ESI+: 459, 461, 463 |
| 84 | 1 | 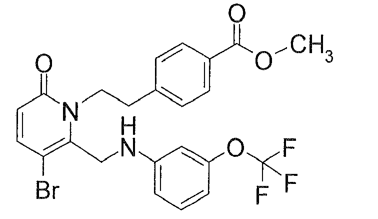 | APCI+: 525 |

**[Table 43]**

| | | | |
|---|---|---|---|
| 85 | 1 | 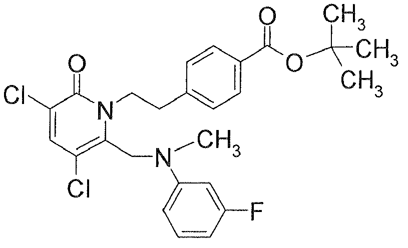 | ESI+: 505, 507 |
| 86 | 1 | 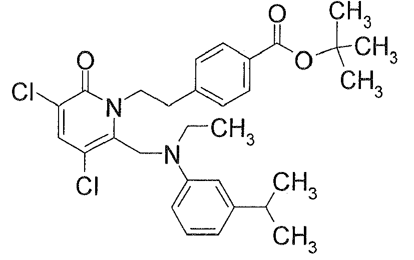 | FAB-: 541 |
| 87 | 1 | 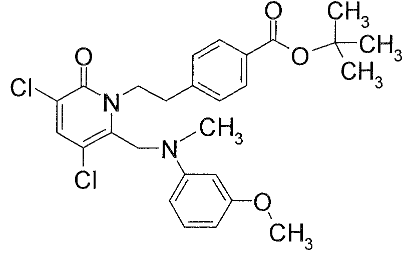 | FAB+; 517, 519 |
| 88 | 1 | 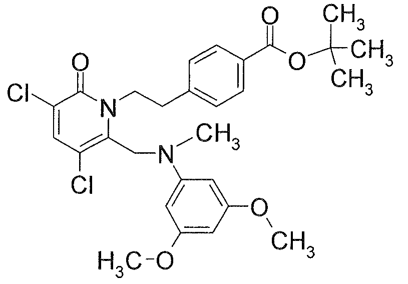 | ESI+: 547, 549, 551 |
| 89 | 1 | 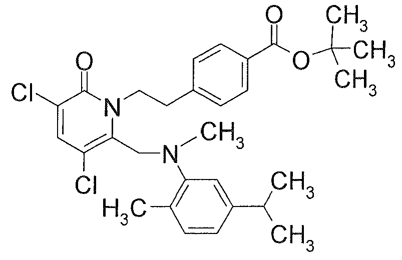 | ESI+: 543, 545 |

**[Table 44]**

| | | | |
|---|---|---|---|
| 90 | 1 | 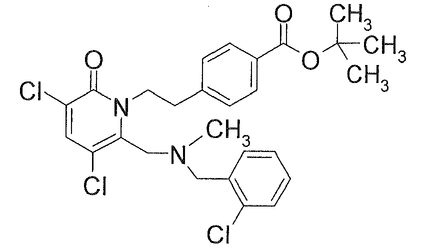 | ESI+: 536, 538, 540 |
| 91 | 5 | 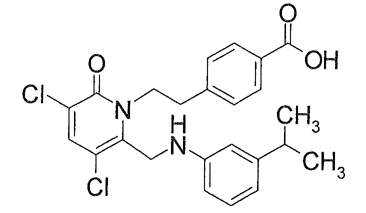 | FAB+: 459 |
| 92 | 5 | 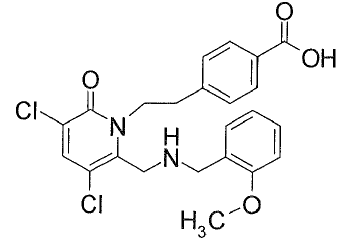 | FAB+: 461 |
| 93 | 5 | 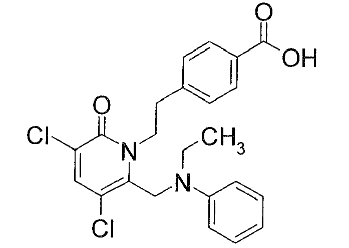 | ESI+: 445, 447 |
| 94 | 5 | 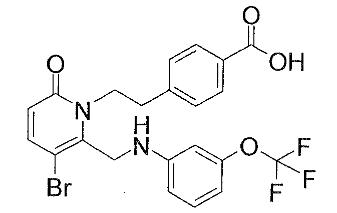 | ESI+: 511, 513 |

**[Table 45]**

| | | | |
|---|---|---|---|
| 95 | 5 | 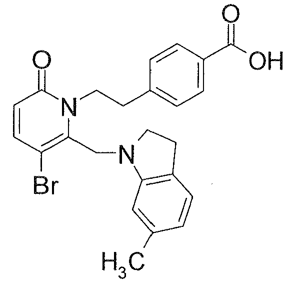 | ESI+: 467, 469 |
| 96 | 6 | 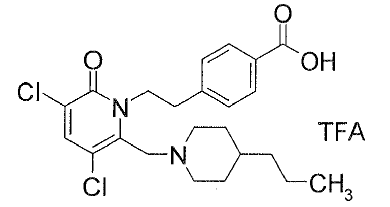 | ESI+: 451, 453, 455 |
| 97 | 6 | 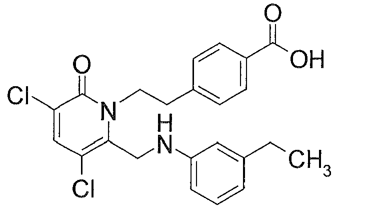 | FAB+: 445 |
| 98 | 6 | 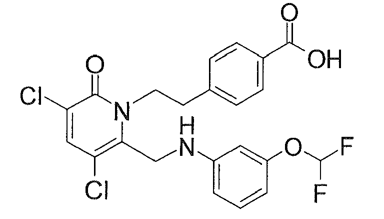 | FAB+: 483 |
| 99 | 6 | 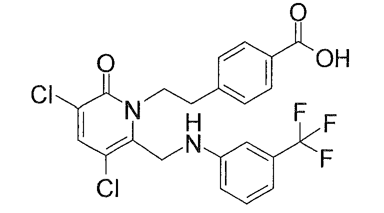 | ESI+: 485, 487, 489 |

**[Table 46]**

| | | | |
|---|---|---|---|
| 100 | 6 | 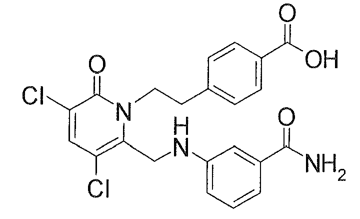 | ESI-: 458, 460 |
| 101 | 6 | 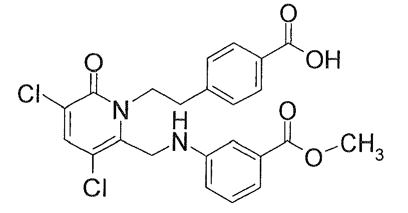 | ESI-: 473, 475 |
| 102 | 6 | 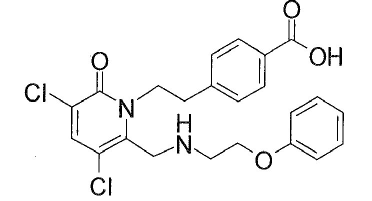 | ESI+: 461, 463 |
| 103 | 6 | 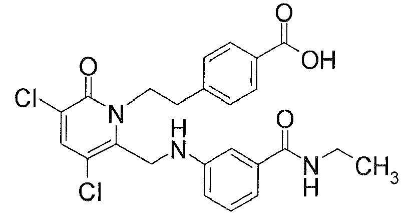 | ESI+: 488, 490 |
| 104 | 6 | 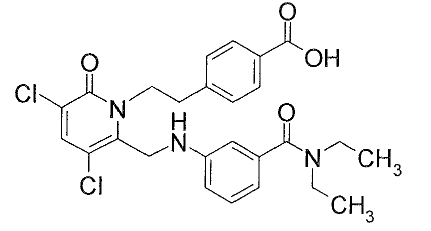 | ESI+: 516, 518 |
| 105 | 6 | 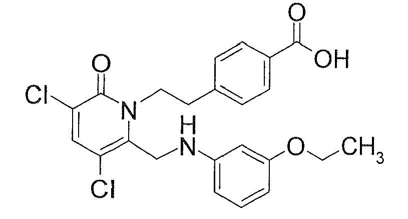 | ESI+: 461, 463, 465 |

**[Table 47]**

| | | | |
|---|---|---|---|
| 106 | 6 | 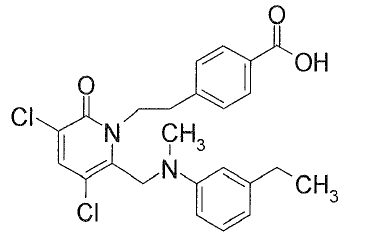 | ESI+: 459, 461 |
| 107 | 6 | 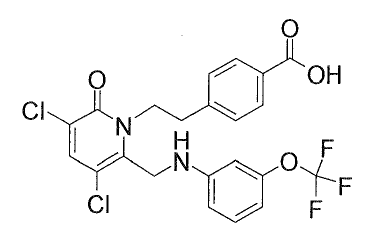 | NMR(400 MHz): 2.97-3.08 (2H, m), 4.12-4.22 (2H, m), 4.33 (2H, d, J = 4.4 Hz), 6.53-6.64 (3H, m), 6.68 (1H, dd, J = 1.6, 8.0 Hz), 7.21-7.27 (3H, m), 7.81 (2H, d, J = 8.0 Hz), 8.10 (1H, s), 12.88 (1H, s); FAB+: 501, 503 |
| 108 | 6 | 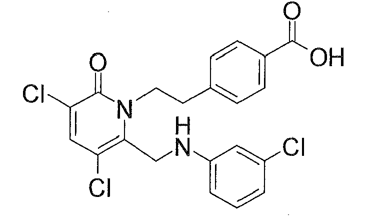 | FAB+: 451 |
| 109 | 6 | 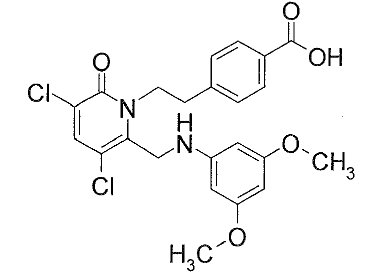 | FAB+: 477 |
| 110 | 6 | 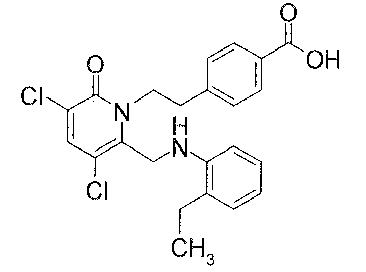 | ESI+: 445, 447, 449 |

**[Table 48]**

| | | | |
|---|---|---|---|
| 111 | 6 | 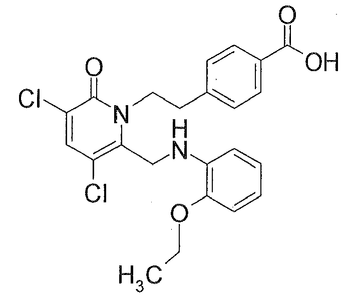 | ESI+: 461, 463, 465 |
| 112 | 6 | 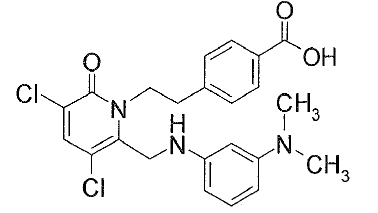 | FAB+: 460 |
| 113 | 6 | 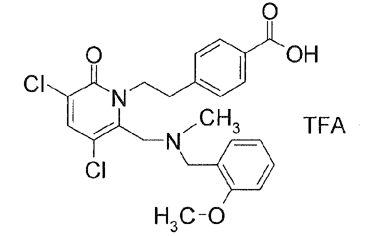 | ESI+: 475, 477, 479 |
| 114 | 6 | 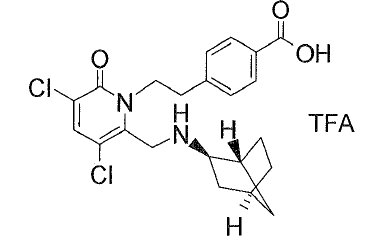 | ESI+: 435, 437, 439 |
| 115 | 6 | 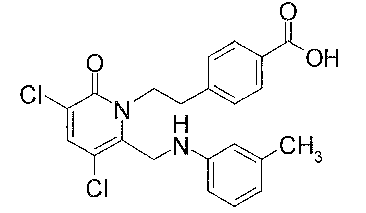 | ESI+: 431, 433 |

**[Table 49]**

| | | | |
|---|---|---|---|
| 116 | 6 | 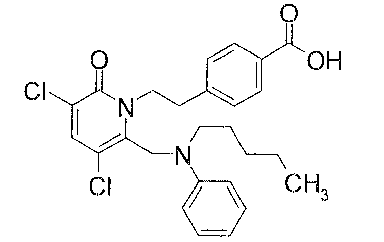 | FAB+: 487 |
| 117 | 5 | 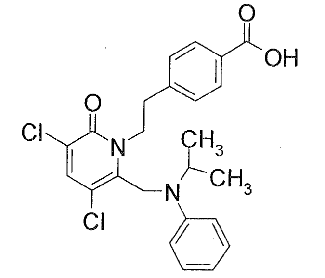 | ESI+: 459, 461, 463 |
| 118 | 6 | 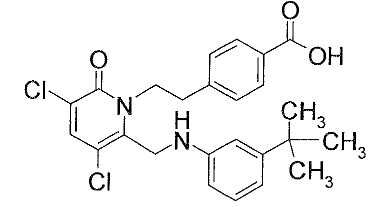 | ESI+: 473, 475, 477 |
| 119 | 6 | 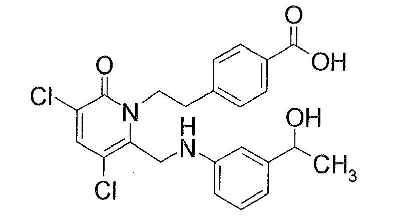 | ESI+: 461, 463, 465 |
| 120 | 6 | 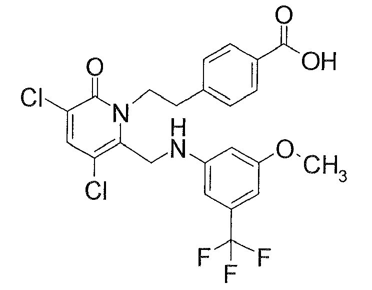 | ESI+: 515, 517, 519 |

**[Table 50]**

| | | | |
|---|---|---|---|
| 121 | 6 | 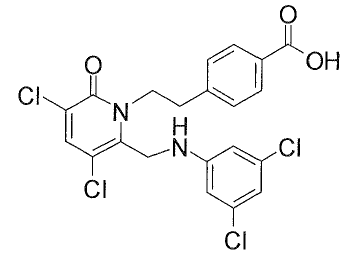 | ESI+: 485, 487, 489 |
| 122 | 6 | 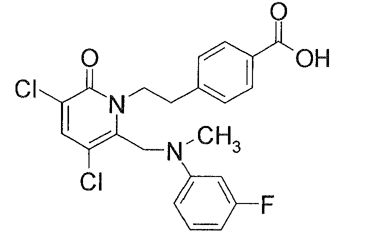 | ESI+: 449, 451 |
| 123 | 6 | 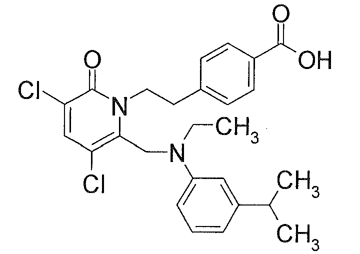 | FAB+: 487 |
| 124 | 6 | 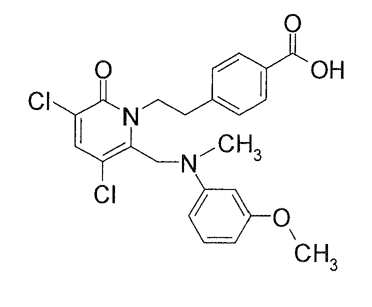 | NMR(400 MHz): 2.74 (3H, s), 2.88-2.96 (2H, m), 3.73 (3H, s), 4.01-4.08 (2H, m), 4.59 (2H, s), 6.45 (1H, dd, J = 8.0, 2.0 Hz), 6.53 (1H, dd, J = 2. 0, 2.0 Hz), 6.61 (1H, dd, J = 8.0, 2.0 Hz), 7.03 (2H, d, J = 8.0 Hz), 7.18 (1H, dd, J = 8.0, 8.0 Hz), 7.74 (2H, d, J = 8.0 Hz), 8.10 (1H, s), 12.87 (1H, s); ESI+: 461, 463 |

**[Table 51]**

| | | | |
|---|---|---|---|
| 125 | 6 | 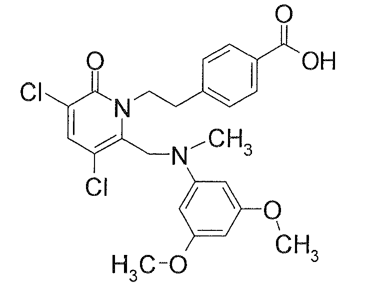 | ESI+: 491, 493 |
| 126 | 6 | 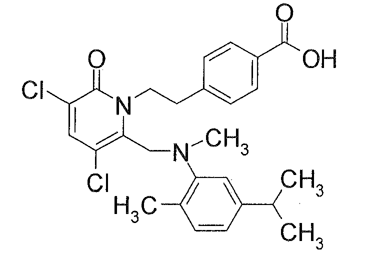 | ESI+: 487, 489 |
| 127 | 7 | 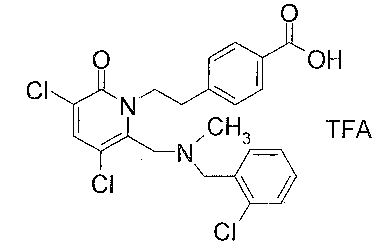 | ESI+: 479 |
| 128 | 7 | 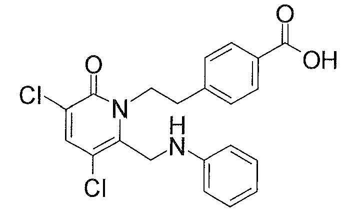 | ESI-: 415, 417 |
| 129 | 9 | 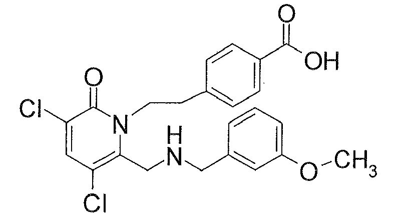 | FAB+: 461 |

**[Table 52]**

| | | | |
|---|---|---|---|
| 130 | 9 | 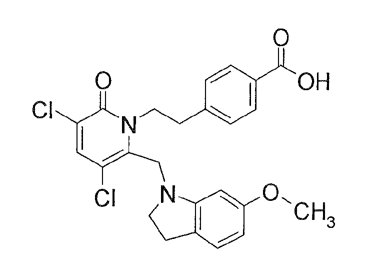 | NMR(300 MHz): 2.80 (2H, t, J = 7.8 Hz), 2.98-3.10 (2H, m), 3.27 (2H, t, J = 7.8 Hz), 3.69 (3H, s), 4.12-4.25 (2H, m), 4.43 (2H, s), 6.27 (1H, dd, J = 2.1, 8.1 Hz), 6.41 (1H, d, J = 2.1 Hz), 6.99 (1H, d, J = 8.1 Hz), 7.18 (2H, d, J = 8.1 Hz), 7.79 (2H, d, J = 8.1 Hz), 8.09 (1H, s), 12.88 (1H, s); ESI+: 473, 475, 477; Elemental Analysis: Found(C:60.69,H:4.72,N: 5.87,Cl:14.76), Calc(C:60.90,H:4.68,N:5. 92,Cl:14.98) |
| 131 | 9 | 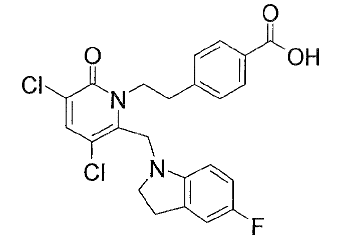 | ESI+: 461, 463 |
| 132 | 10 | 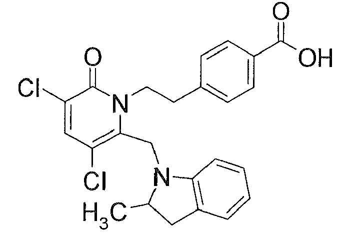 | ESI+: 457, 459 |
| 133 | 10 | 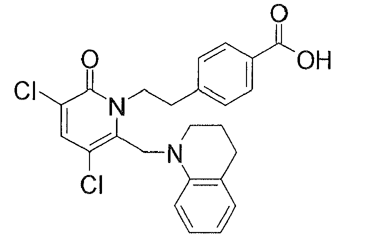 | ESI+: 457, 459 |

**[Table 53]**

| | | | |
|---|---|---|---|
| 134 | 10 | 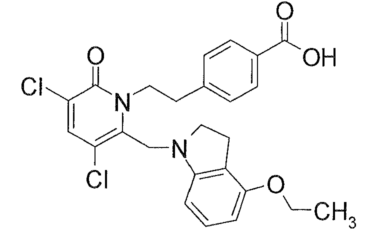 | ESI+: 487, 489 |
| 135 | 10 | 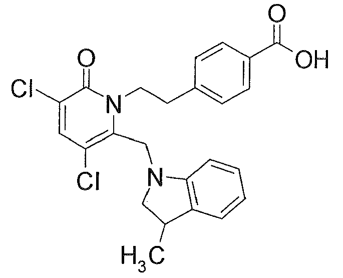 | ESI+: 457, 459, 461 |
| 136 | 10 | 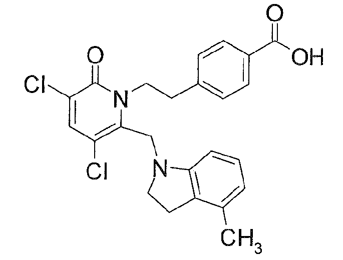 | ESI+ 457, 459 |
| 137 | 10 | 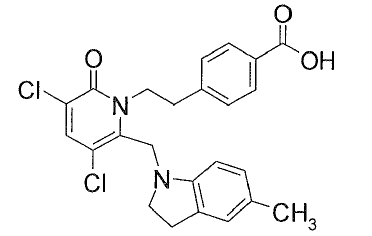 | ESI+: 457, 459 |
| 138 | 10 | 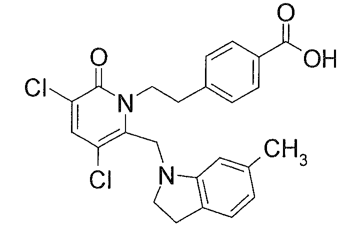 | ESI+: 457, 459, 461 |

**[Table 54]**

| | | | |
|---|---|---|---|
| 139 | 10 | 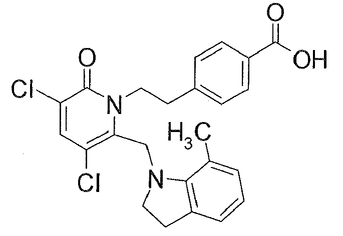 | ESI+: 457, 459 |
| 140 | 13 | 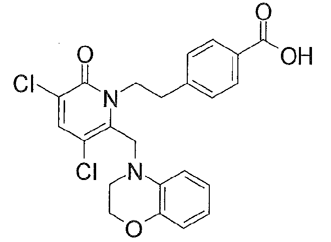 | ESI+: 459, 461 |
| 141 | 13 | 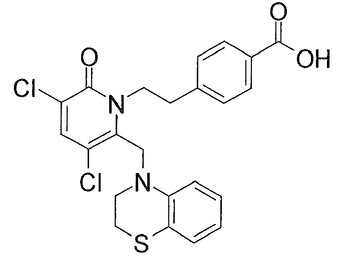 | FAB-: 473 |
| 142 | 13 | 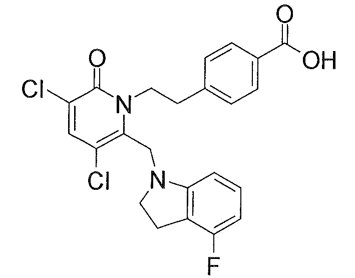 | ESI+: 461, 463 |

**[Table 55]**

| | | | |
|---|---|---|---|
| 143 | 13 | 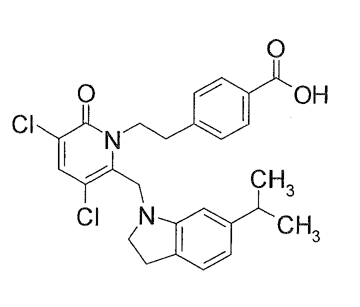 | NMR(400 MHz): 1.15 (6H, d, J = 6.8 Hz), 2.71-2.86 (3H, m), 3.02-3.10 (2H, m), 3.24 (2H, t, J = 8.0 Hz), 4.15-4.24 (2H, m), 4.43 (2H, s), 6.59 (1H, dd, J = 1.2, 7.6 Hz), 6.62-6.65 (1H, m), 7.00 (1H, d, J = 7.6 Hz), 7.19 (2H, d, J = 8.0 Hz), 7.78 (2H, d, J = 8.0 Hz), 8.09 (1H, s), 12.85 (1H, s); FAB-: 483; Elemental Analysis: Found(C:64.07,H:5.44,N: 5.73,Cl:14.57), Calc(C:64.33,H:5.40,N:5. 77,Cl:14.61) |
| 144 | 13 | 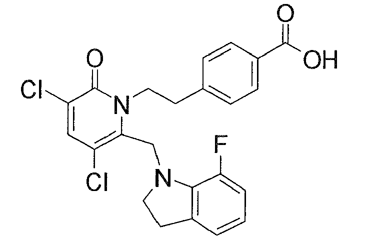 | ESI+: 461, 463 |
| 145 | 13 | 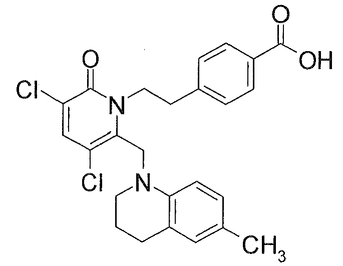 | FAB-: 469 |
| 146 | 13 | 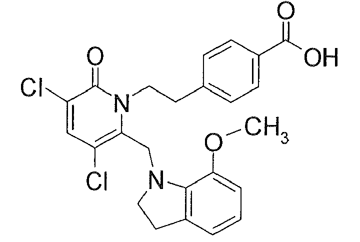 | ESI+: 473, 475, 477 |

**[Table 56]**

| | | | |
|---|---|---|---|
| 147 | 13 | 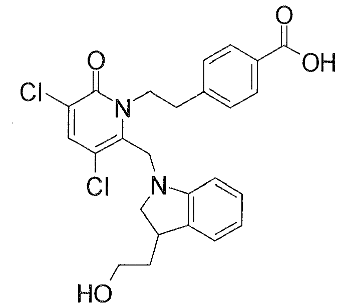 | ESI+: 487, 489 |
| 148 | 13 | 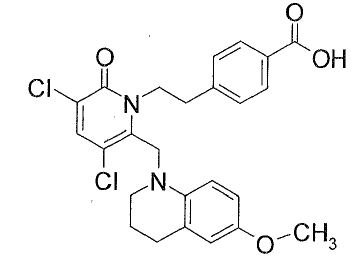 | ESI+: 487, 489 |
| 149 | 13 | 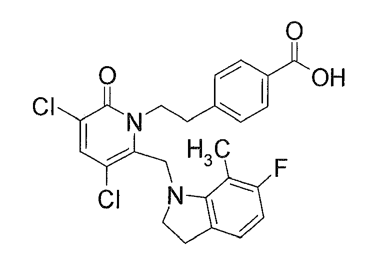 | NMR(300 MHz): 2.16 (3H, s), 2.81 (2H, t, J = 8.1 Hz), 3.06-3.15 (2H, m), 3.20 (2H, t, J = 8.1 Hz), 4.45-4.54 (2H, m), 4.59 (2H, s), 6.58 (1H, dd, J = 8.0, 10.2 Hz), 6.96 (1H, dd, J = 6.0, 8.0 Hz), 7.24 (2H, d, J = 8.1 Hz), 7.84 (2H, d, J = 8.1 Hz), 8.09 (1H, s), 12.90 (1H, s); ESI+: 475, 477 |
| 150 | 13 | 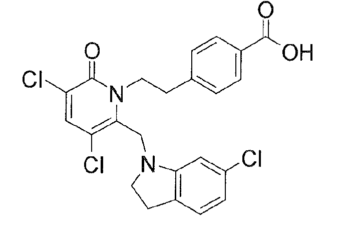 | FAB-: 475 |

**[Table 57]**

| | | | |
|---|---|---|---|
| 151 | 15 | 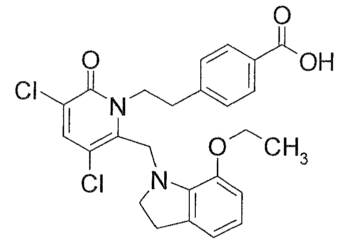 | FAB-: 485 |
| 152 | 15 | 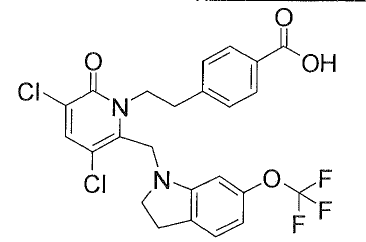 | ESI+: 527, 529, 531 |
| 153 | 15 | 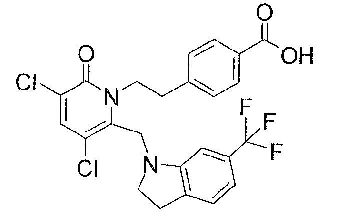 | ESI+: 511, 513 |
| 154 | 17 | 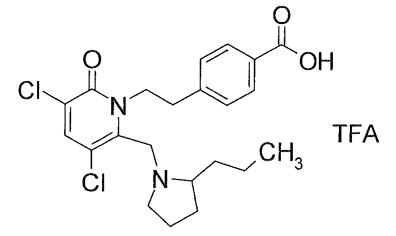 | ESI+: 437, 439 |
| 155 | 17 | 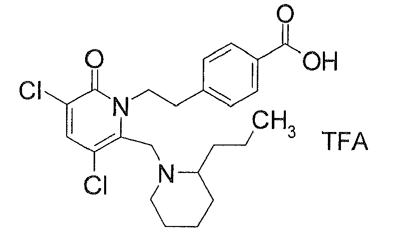 | ESI+: 451, 453 |

**[Table 58]**

| | | | |
|---|---|---|---|
| 156 | 18 | 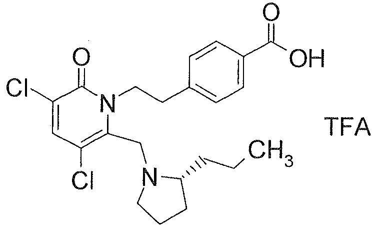 | APCI+: 437, 439 |
| 157 | 18 | 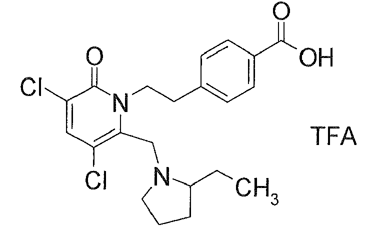 | ESI+: 423, 425 |
| 158 | 18 | 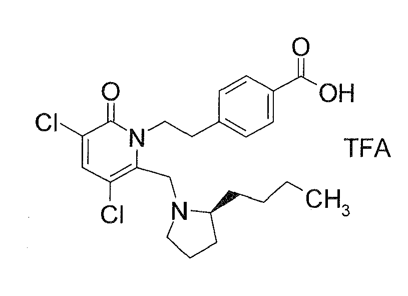 | NMR(300 MHz): 0.75-0.88 (br), 1.09-1.35 (br), 1.37-2.20 (br), 2.94-3.10 (2H, m), 4.29-4.56 (br), 7.39 (2H, d, J = 8.1 Hz), 7.90 (2H, d, J = 8.1 Hz), 8.10 (br); ESI+: 451, 453; Elemental Analysis: Found(C:52.91,H:5.11,N: 5.00,Cl:12.53,F:10.07), Calc(C:53.11,H:5.17,N:4. 95,Cl:12.54,F:10.08) |
| 159 | 19 | 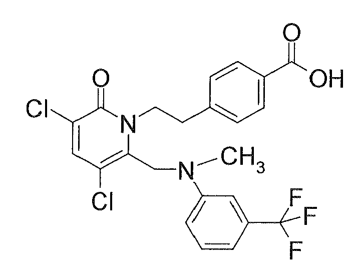 | NMR(400 MHz): 2.80 (3H, s), 2.88-2.96 (2H, m), 3.98-4.07 (2H, m), 4.69 (2H, s), 6.99 (2H, d, J = 8.0 Hz), 7.13 (1H, d, J = 8.0 Hz), 7.22-7.31 (2H, m), 7.47 (1H, dd, J = 8.0, 8.0 Hz), 7.72 (2H, d, J = 8.0 Hz), 8.11 (1H, s), 12.85 (1H, s); ESI+: 499, 501 |

**[Table 59]**

| | | | |
|---|---|---|---|
| 160 | 19 | 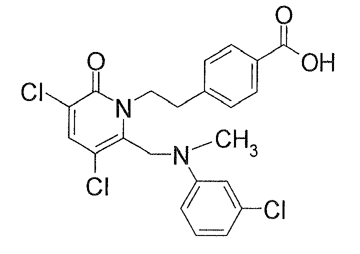 | NMR(400 MHz): 2.75 (3H, s), 2.89-2.97 (2H, m), 3.97-4.06 (2H, m), 4.63 (2H, s), 6.85 (1H, dd, J = 8.0, 2.0 Hz), 6.95 (1H, dd, J = 8.0, 2.0 Hz), 7.04-7.08 (3H, m), 7.27 (1H, dd, J= 8.0, 8.0 Hz), 7.76 (2H, d, J = 8.0 Hz), 8.11 (1H, s), 12.87 (1H, s); ESI+: 465, 467, 469 |
| 161 | 19 | 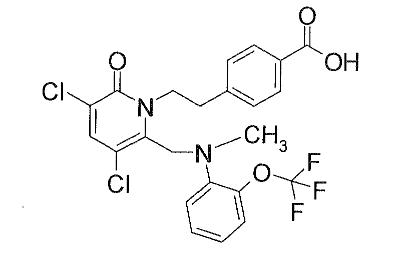 | ESI+: 515, 517 |
| 162 | 20 | 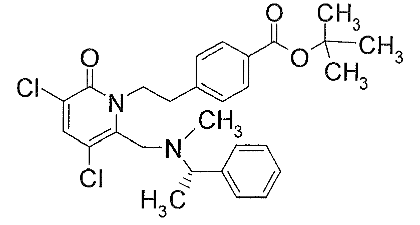 | ESI+: 515, 517, 519 |
| 163 | 20 | 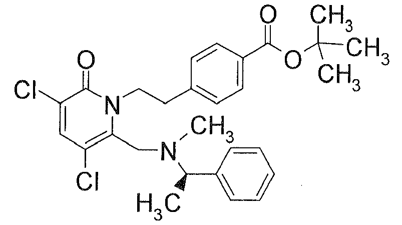 | ESI+: 515, 517, 519 |
| 164 | 20 | 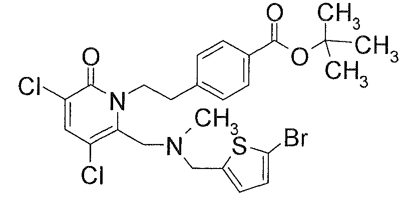 | ESI+: 585, 587, 589 |

**[Table 60]**

| | | | |
|---|---|---|---|
| 165 | 20 | 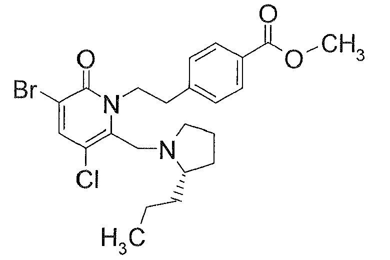 | ESI+: 495, 497, 499 |
| 166 | 26 | 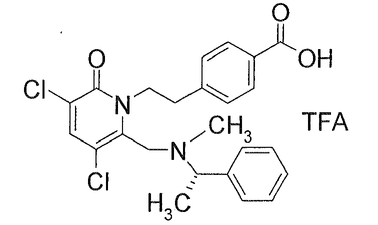 | ESI+: 459, 461 |
| 167 | 26 | 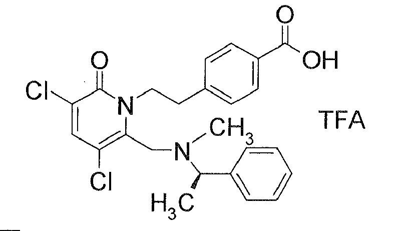 | ESI+: 459, 461, 463 |
| 168 | 26 | 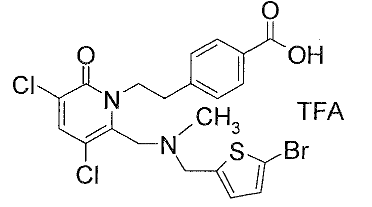 | ESI+: 529, 531, 533, 535 |
| 169 | 28 | 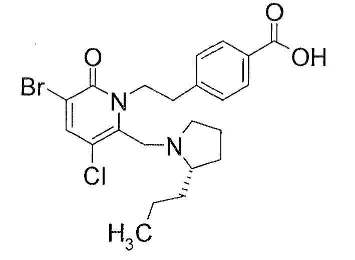 | ESI+: 481, 483 |

**[Table 61]**

| | | | |
|---|---|---|---|
| 170 | 20 | 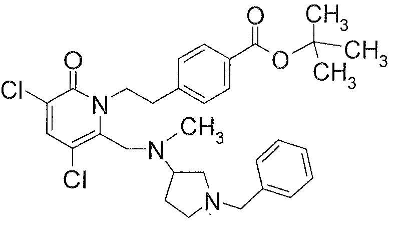 | ESI+: 570, 572 |
| 171 | 26 | 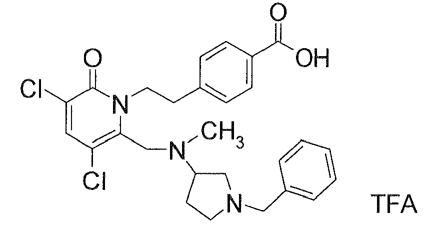 | ESI+: 514, 516, 518 |
| 172 | 20 | 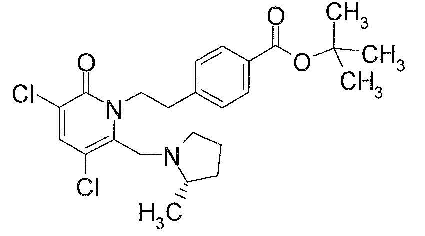 | ESI+: 465 |
| 173 | 20 | 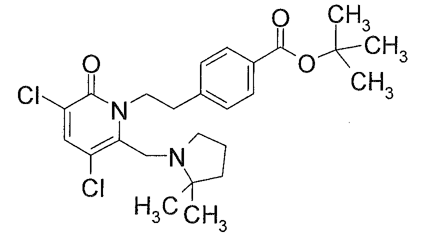 | ESI+: 479 |
| 174 | 20 | 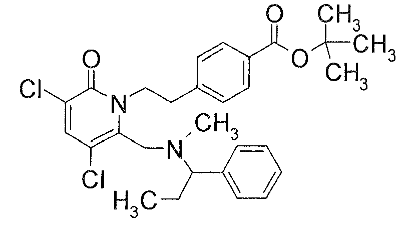 | ESI+: 529, 531, 533 |

**[Table 62]**

| | | | |
|---|---|---|---|
| 175 | 20 | 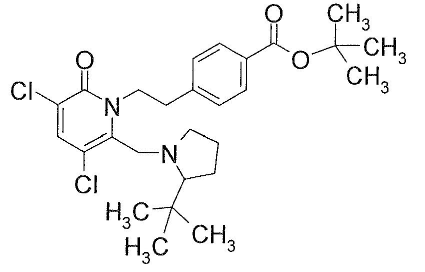 | ESI+: 507 |
| 176 | 20 | 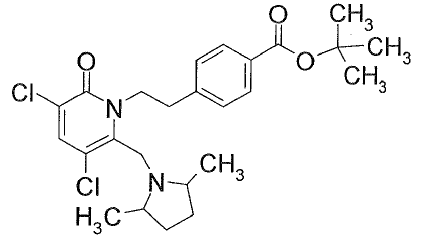 | ESI+: 479 |
| 177 | 20 | 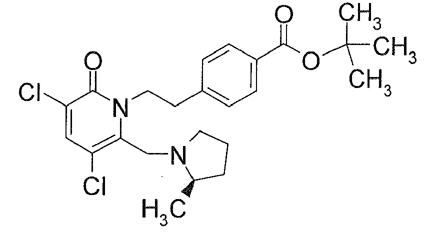 | ESI+: 465 |
| 178 | 26 | 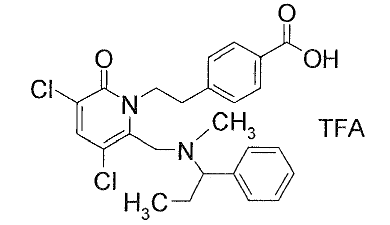 | ESI+: 473, 475 |
| 179 | 20 | 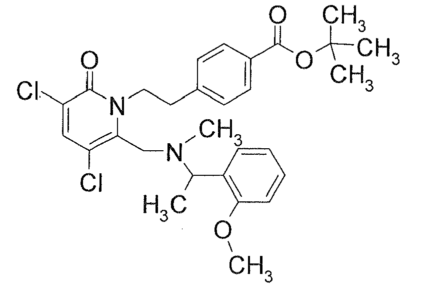 | ESI+: 545, 547, 549 |

**[Table 63]**

| | | | |
|---|---|---|---|
| 180 | 26 | 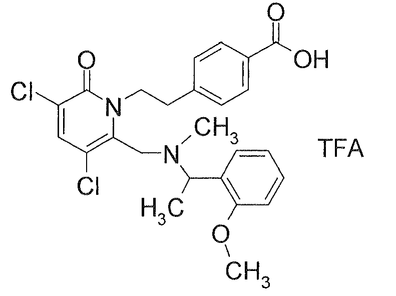 | ESI+: 489, 491, 493 |
| 181 | 26 | 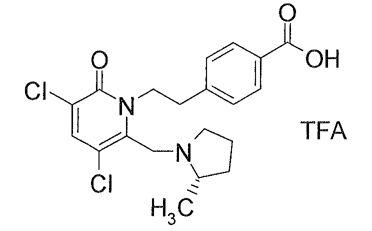 | ESI+: 409 |
| 182 | 26 | 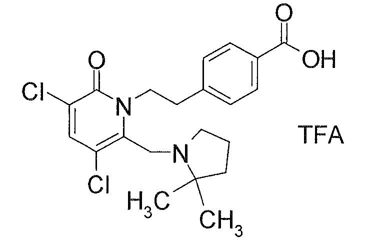 | ESI+: 423 |
| 183 | 26 | 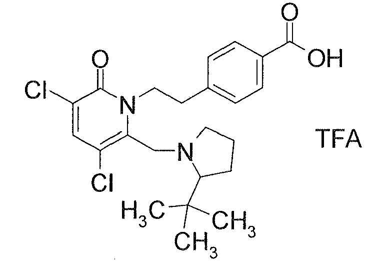 | ESI+: 451 |
| 184 | 26 | 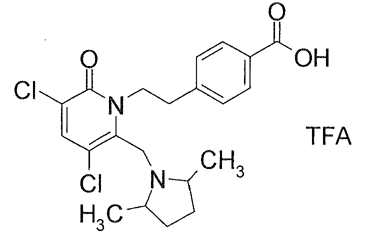 | ESI+: 423 |

**[Table 64]**

| | | | |
|---|---|---|---|
| 185 | 26 | 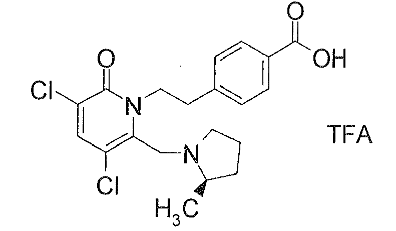 | ESI+: 409 |
| 186 | 20 | 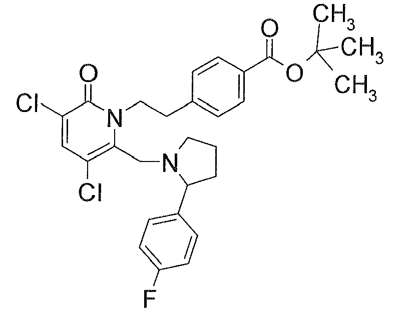 | ESI+: 545 |
| 187 | 26 | 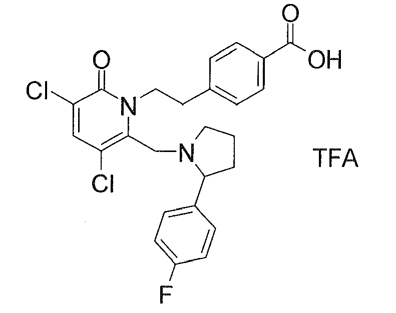 | ESI+: 489 |
| 188 | 20 | 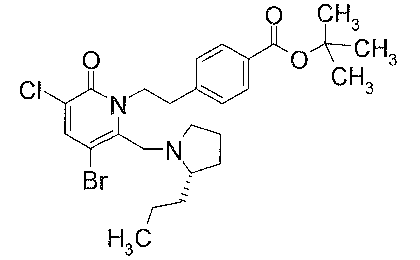 | ESI+: 537, 539, 541 |

**[Table 65]**

| | | | |
|---|---|---|---|
| 189 | 26 | 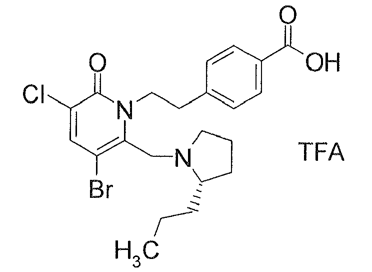 | ESI+: 481, 483, 485 |
| 190 | 20 | 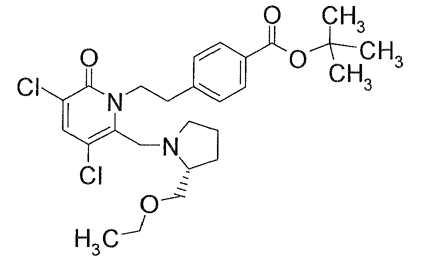 | ESI+: 509 |
| 191 | 20 | 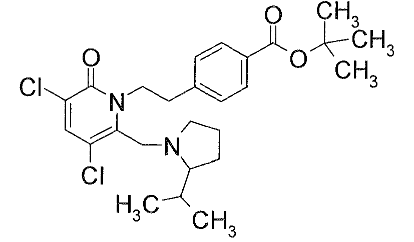 | ESI+: 493 |
| 192 | 20 | 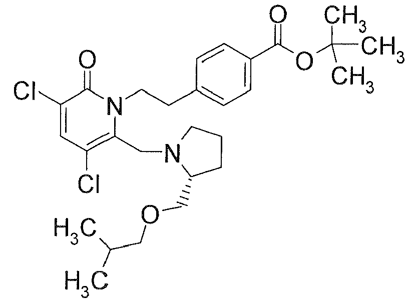 | ESI+: 537 |
| 193 | 20 | 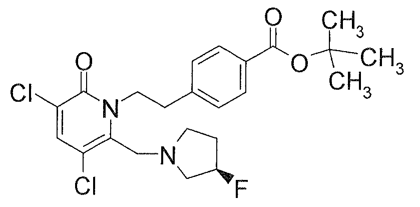 | ESI+: 469 |

**[Table 66]**

| | | | |
|---|---|---|---|
| 194 | 20 | 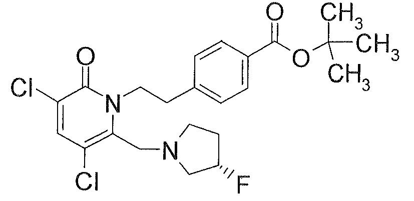 | ESI+: 469 |
| 195 | 26 | 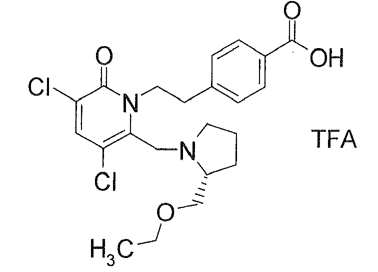 | ESI+: 453 |
| 196 | 20 | 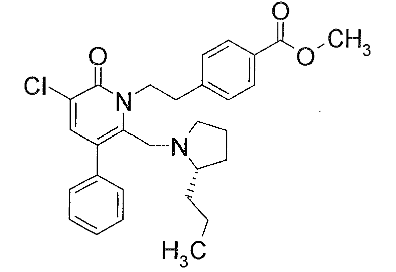 | ESI+: 493, 495 |
| 197 | 20 | 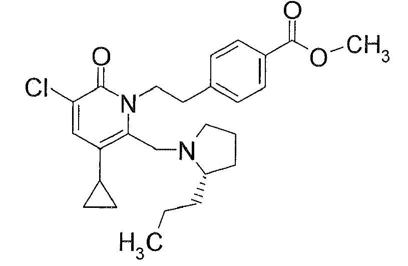 | ESI+: 457, 459 |
| 198 | 20 | 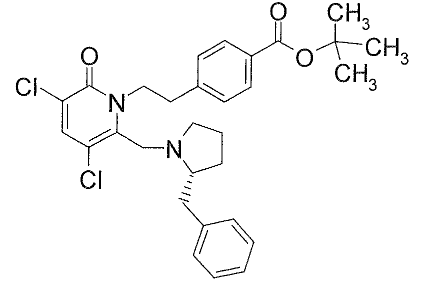 | ESI+: 541 |

**[Table 67]**

| | | | |
|---|---|---|---|
| 199 | 29 | 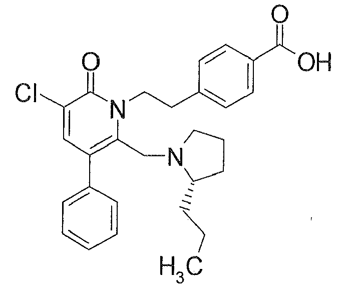 | ESI+: 479, 481 |
| 200 | 26 | 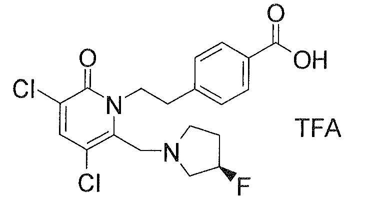 | ESI+: 413 |
| 201 | 26 | 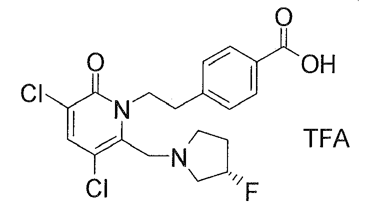 | ESI+: 413 |
| 202 | 26 | 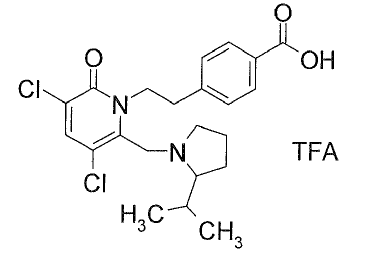 | ESI+: 437 |
| 203 | 26 | 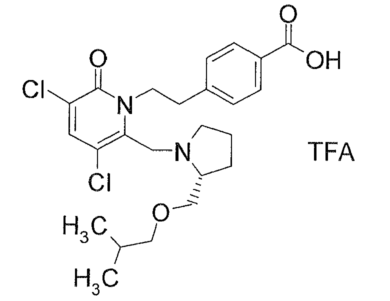 | ESI+: 481 |

**[Table 68]**

| | | | |
|---|---|---|---|
| 204 | 26 | 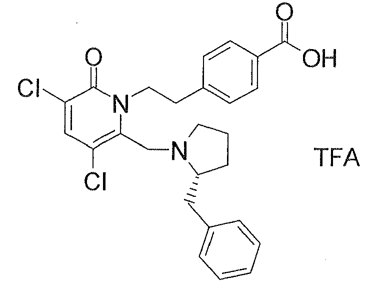 | ESI+: 485 |
| 205 | 20 | 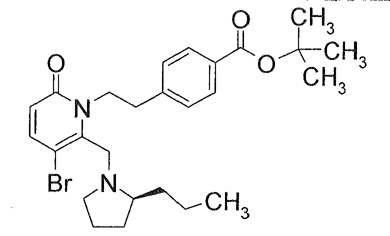 | ESI+: 503, 505 |
| 206 | 19 | 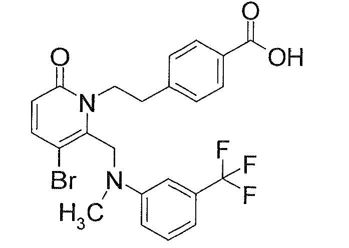 | ESI+: 509, 511 |
| 207 | 26 | 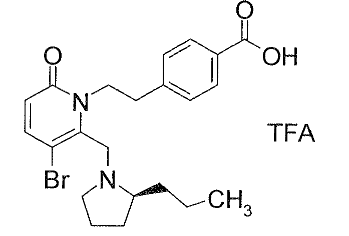 | ESI+: 447, 449 |

**[Table 69]**

| | | | |
|---|---|---|---|
| 208 | 47 | 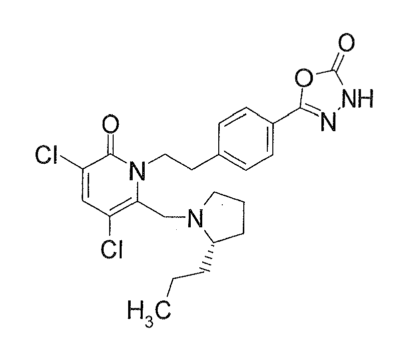 | NMR(400MHz): 0.81 (3H, t, J = 7.2 Hz), 0.92-1.05 (1H, m), 1.10-1.41 (3H, m), 1.49-1.69 (3H, m), 1.85-1.97 (1H, m), 2.25-2.40 (2H, m), 2.73-2.82 (1H, m), 2.98-3.11(2H, m), 3.56 (1H, d, J = 13.6 Hz), 3.89 (1H, d, J = 13.6 Hz), 4.32-4.44 (1H, m), 4.45-4.57 (1H, m), 7.43 (2H, d, J = 8.0 Hz), 7.76 (2H, d, J = 8.0 Hz), 8.01 (1H, s), 12.6 (br); ESI+: 477, 479, 481 |
| 209 | 20 | 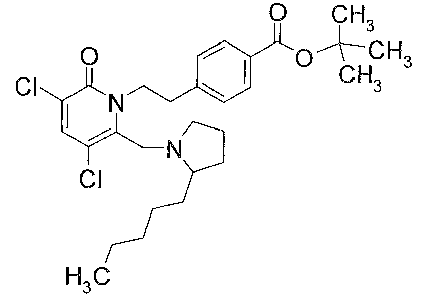 | ESI+: 521 |
| 210 | 20 | 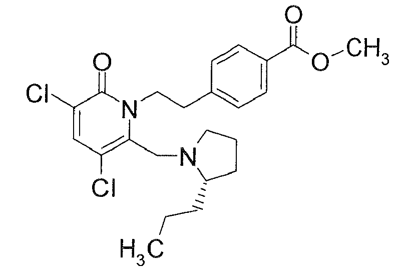 | ESI+: 451, 453, 455 |
| 211 | 20 | 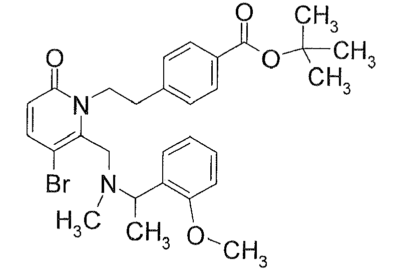 | ESI+: 555 |

**[Table 70]**

| | | | |
|---|---|---|---|
| 212 | 26 | 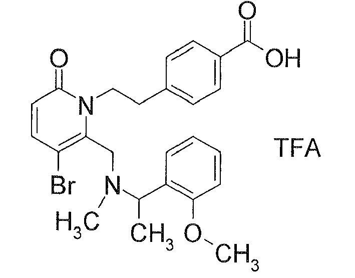 | ESI+: 499, 501 |
| 213 | 20 | 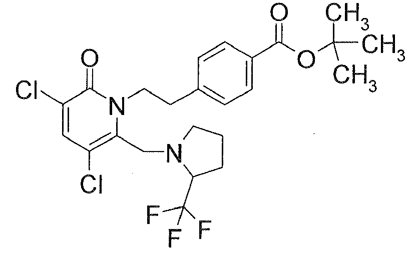 | ESI+: 519 |
| 214 | 20 | 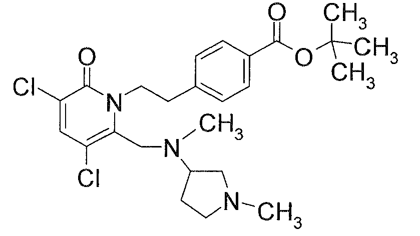 | ESI+: 494 |
| 215 | 26 | 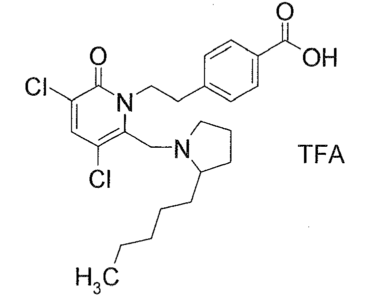 | ESI+: 465 |
| 216 | 20 | 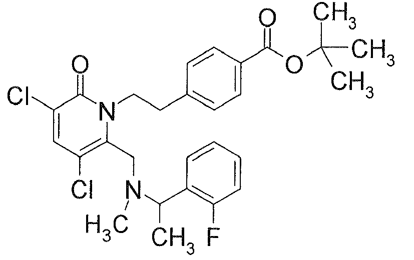 | ESI+: 533 |

**[Table 71]**

| | | | |
|---|---|---|---|
| 217 | 26 | 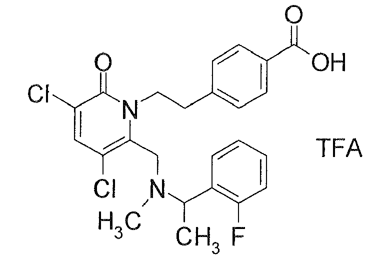 | ESI+: 477 |
| 218 | 20 | 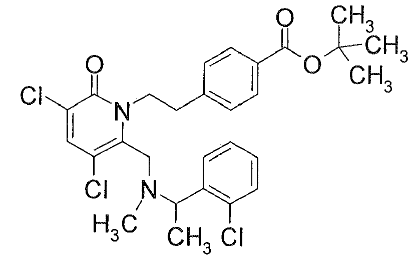 | ESI+: 549 |
| 219 | 26 | 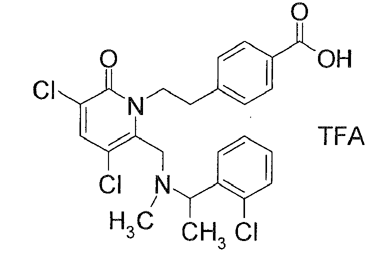 | ESI+: 493 |
| 220 | 20 | 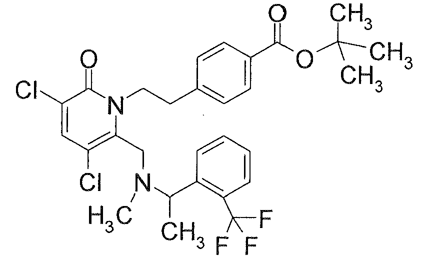 | ESI+: 583 |
| 221 | 26 | 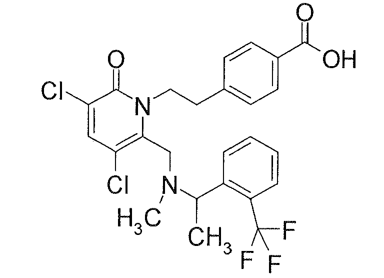 | ESI+: 527 |

**[Table 72]**

| | | | |
|---|---|---|---|
| 222 | 20 | 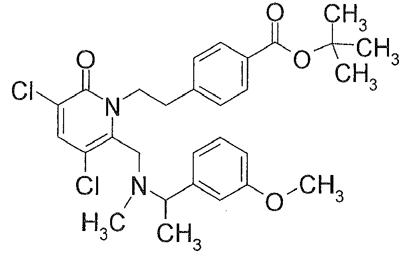 | ESI+: 545 |
| 223 | 26 | 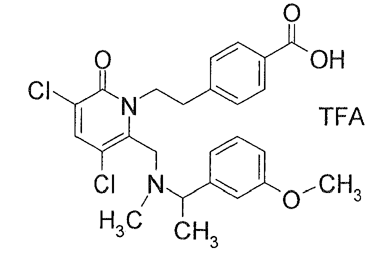 | ESI+: 489 |
| 224 | 26 | 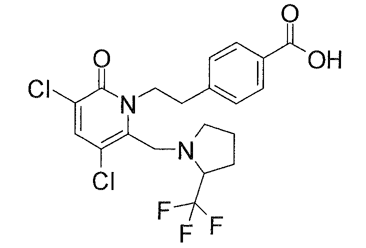 | ESI+: 463 |
| 225 | 26 | 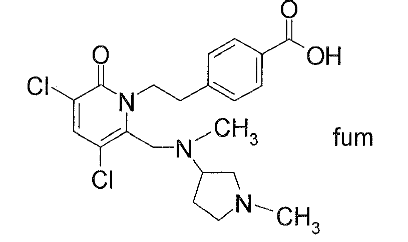 | ESI+: 438 |
| 226 | 20 | 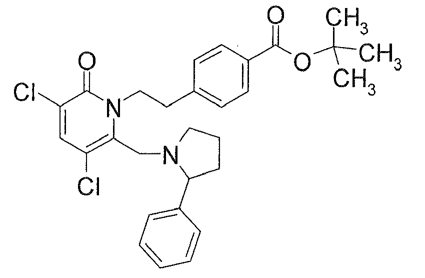 | ESI+: 527 |

**[Table 73]**

| | | | |
|---|---|---|---|
| 227 | 20 | 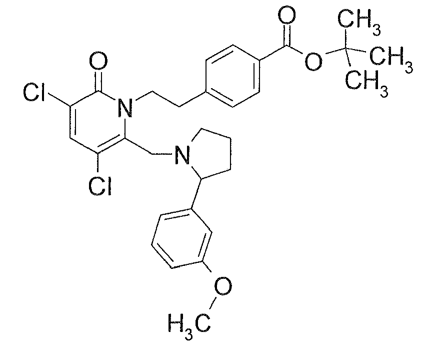 | ESI+: 557 |
| 228 | 20 | 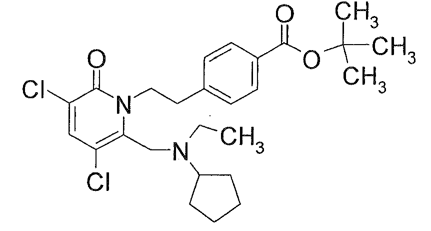 | ESI+: 493 |
| 229 | 20 | 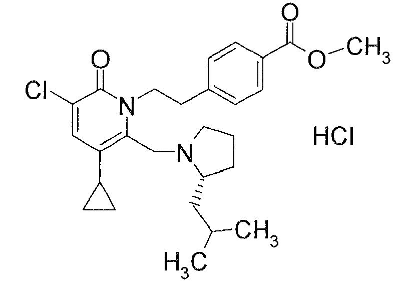 | ESI+: 471, 473 |
| 230 | 20 | 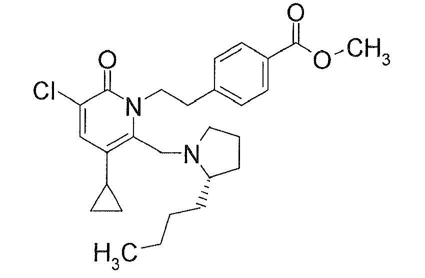 | ESI+: 471, 473 |

**[Table 74]**

| | | | |
|---|---|---|---|
| 231 | 20 | 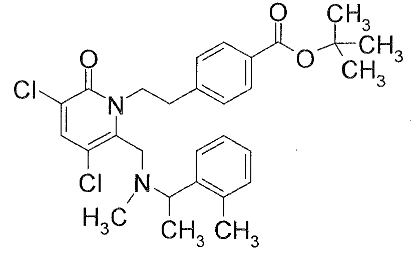 | ESI+: 529, 531 |
| 232 | 26 | 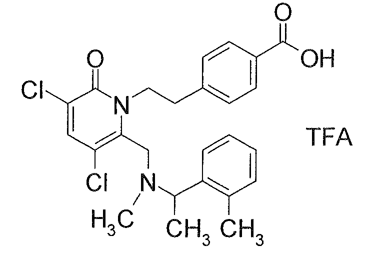 | ESI+: 473, 475 |
| 233 | 29 | 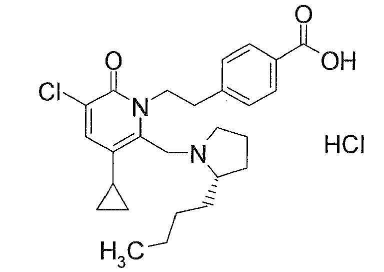 | ESI+: 457, 459 |
| 234 | 29 | 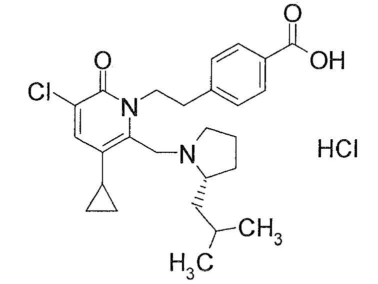 | ESI+: 457, 459 |
| 235 | 20 | 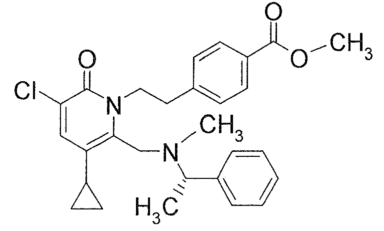 | ESI+: 479 |

**[Table 75]**

| | | | |
|---|---|---|---|
| 236 | 29 | 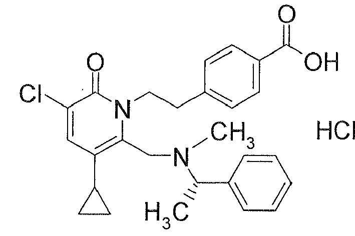 | ESI+: 465, 467 |
| 237 | 26 | 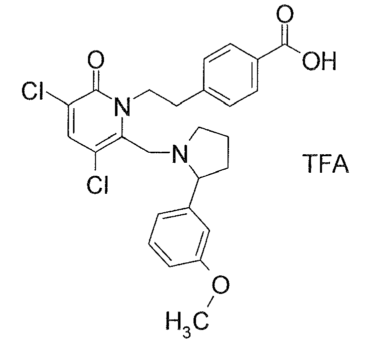 | ESI+: 501 |
| 238 | 26 | 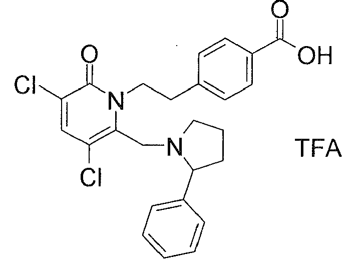 | ESI+: 471 |
| 239 | 20 | 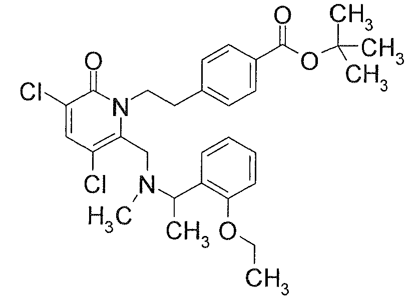 | ESI+: 559 |

**[Table 76]**

| | | | |
|---|---|---|---|
| 240 | 26 | 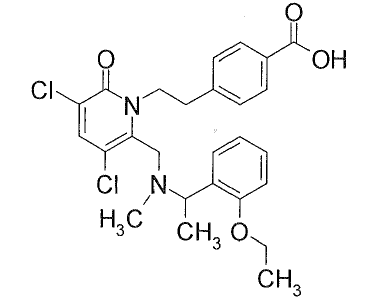 | ESI+: 503, 505 |
| 241 | 20 | 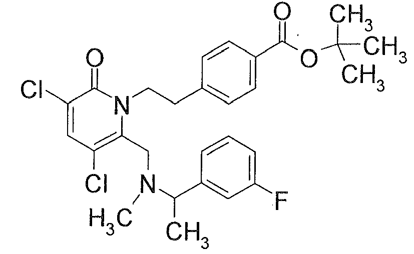 | ESI+: 533 |
| 242 | 26 | 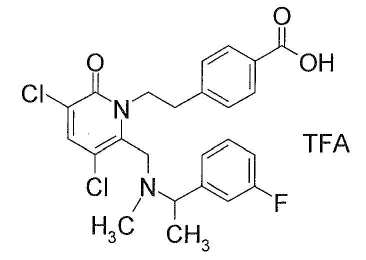 | ESI+: 477, 479 |
| 243 | 20 | 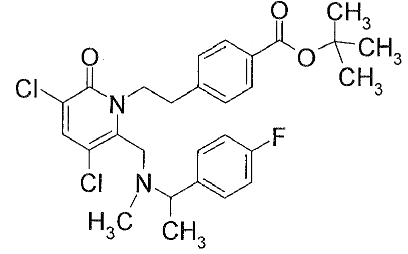 | ESI+: 533 |
| 244 | 26 | 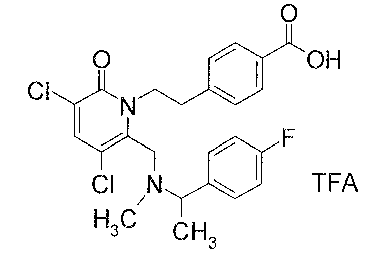 | ESI+: 477, 479 |

**[Table 77]**

| | | | |
|---|---|---|---|
| 245 | 20 | 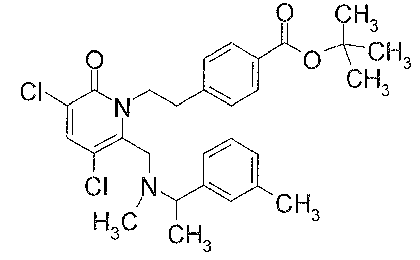 | ESI+: 529 |
| 246 | 26 | 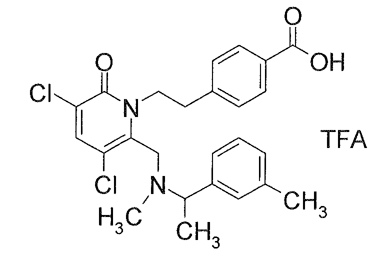 | ESI+: 473, 475 |
| 247 | 20 | 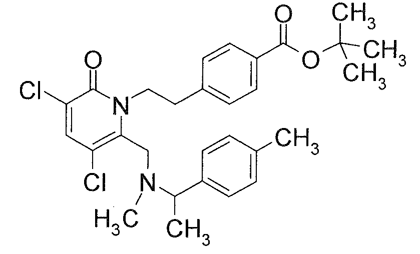 | ESI+: 529 |
| 248 | 26 | 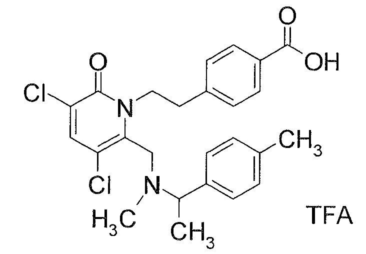 | ESI+: 473, 475 |
| 249 | 20 | 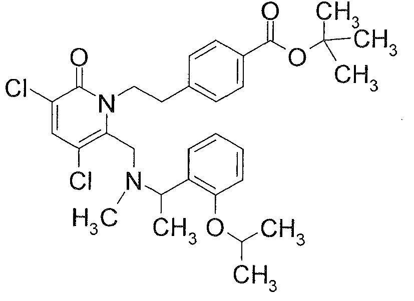 | ESI+: 573, 575 |

**[Table 78]**

| | | | |
|---|---|---|---|
| 250 | 26 | 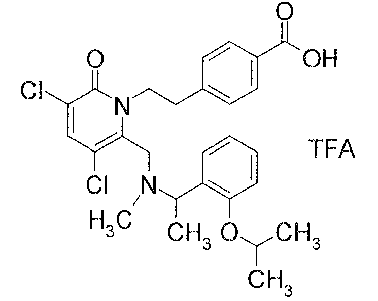 | ESI+: 517, 519 |
| 251 | 20 | 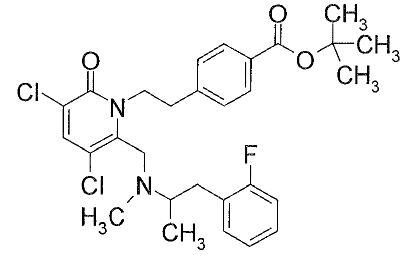 | ESI+: 547, 549 |
| 252 | 26 | 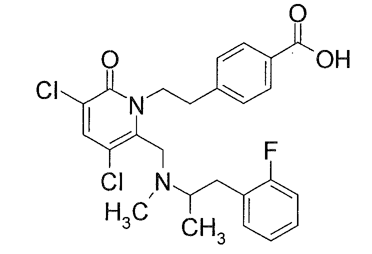 | ESI+: 491, 493 |
| 253 | 20 | 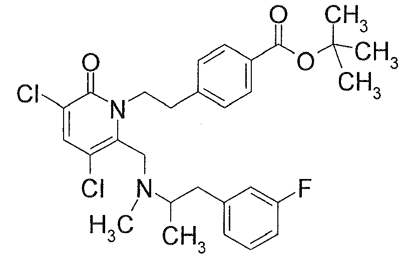 | ESI+: 547, 549 |
| 254 | 26 | 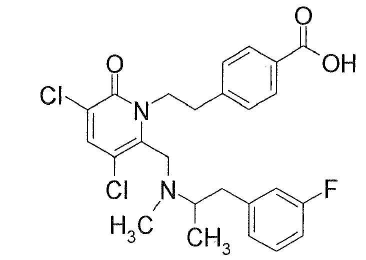 | ESI+: 491, 493 |

**[Table 79]**

| | | | |
|---|---|---|---|
| 255 | 20 | 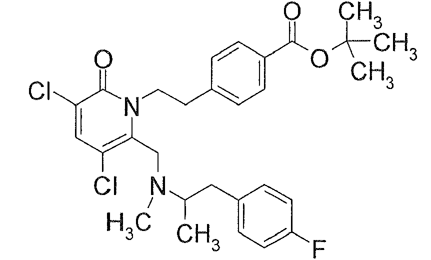 | ESI+: 547, 549 |
| 256 | 26 | 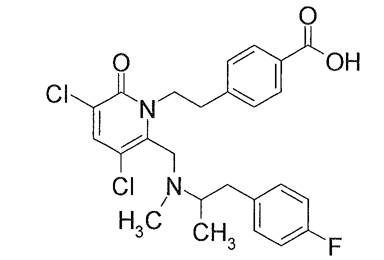 | ESI+: 491, 493 |
| 257 | 20 | 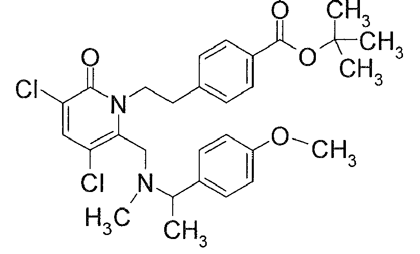 | ESI+: 545, 547 |
| 258 | 30 | 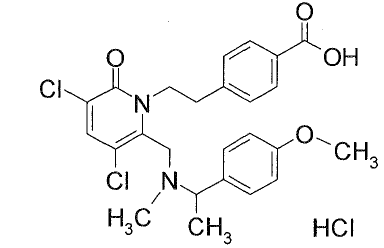 | ESI+: 489, 491 |

**[Table 80]**

| | | | |
|---|---|---|---|
| 259 | 20 | 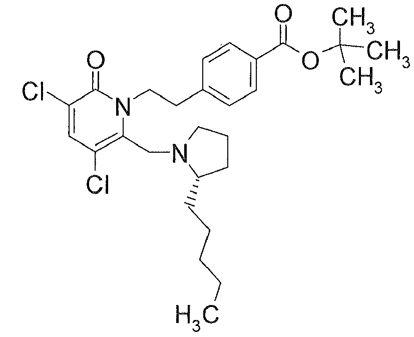 | ESI+: 521 |
| 260 | 20 | 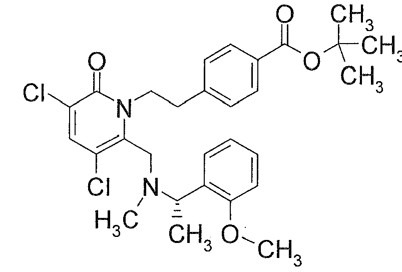 | ESI+: 545, 547 |
| 261 | 26 | 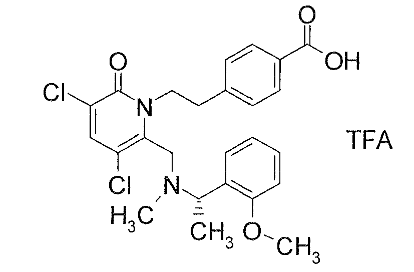 | ESI+: 489, 491 |
| 262 | 20 | 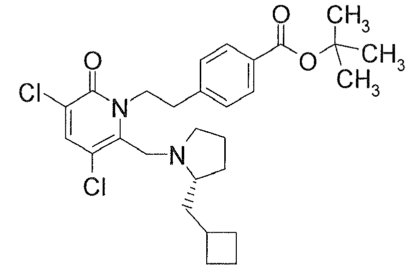 | ESI+: 519 |

**[Table 81]**

| | | | |
|---|---|---|---|
| 263 | 20 | 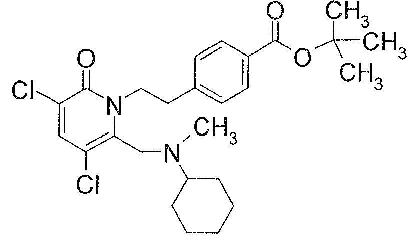 | ESI+: 493 |
| 264 | 26 | 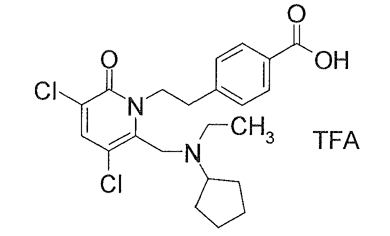 | ESI+: 437 |
| 265 | 26 | 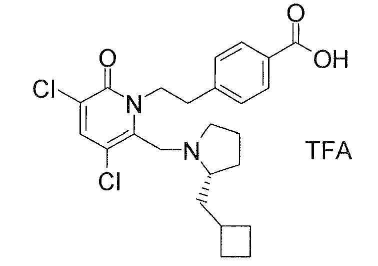 | ESI+: 463 |
| 266 | 26 | 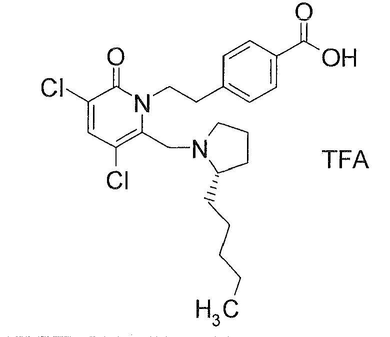 | ESI+: 465 |

**[Table 82]**

| | | | |
|---|---|---|---|
| 267 | 20 | 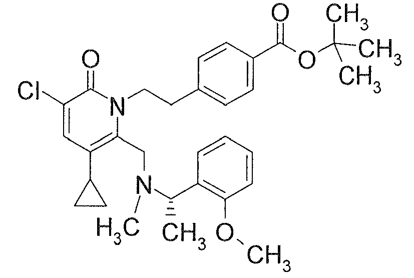 | ESI+: 551, 553 |
| 268 | 20 | 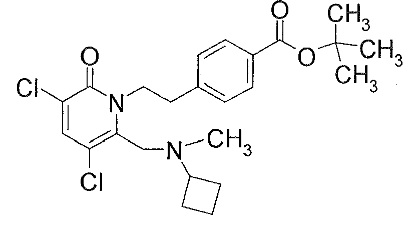 | ESI+: 465 |
| 269 | 20 | 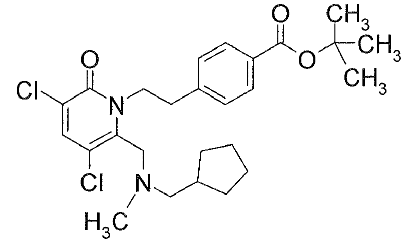 | ESI+: 493 |
| 270 | 26 | 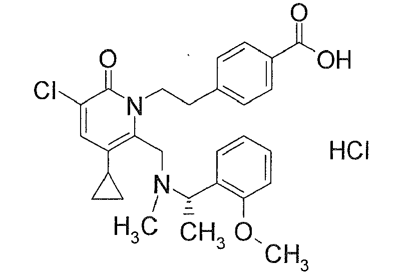 | ESI+: 495, 497 |
| 271 | 20 | 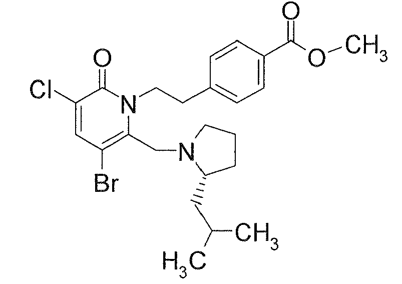 | ESI+: 509, 511, 513 |

**[Table 83]**

| | | | |
|---|---|---|---|
| 272 | 26 | 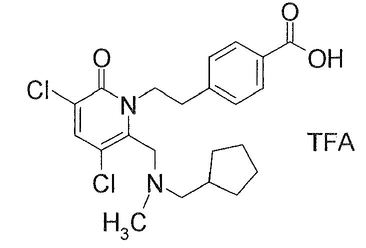 | ESI+: 437 |
| 273 | 26 | 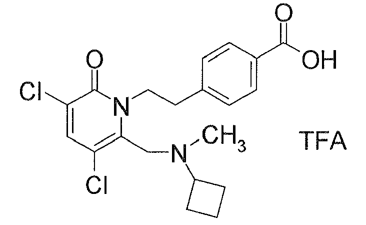 | ESI+: 409 |
| 274 | 26 | 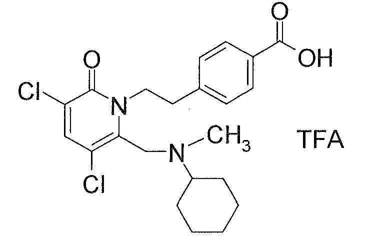 | ESI+: 437 |
| 275 | 20 | 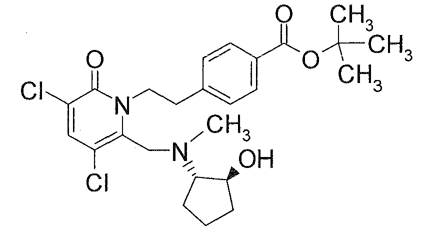 | ESI+: 495 |
| 276 | 20 | 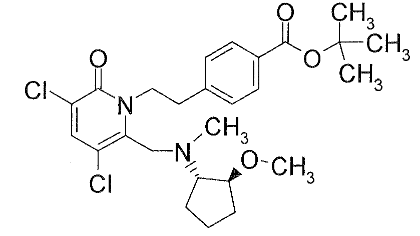 | ESI+: 509 |

**[Table 84]**

| | | | |
|---|---|---|---|
| 277 | 37 | 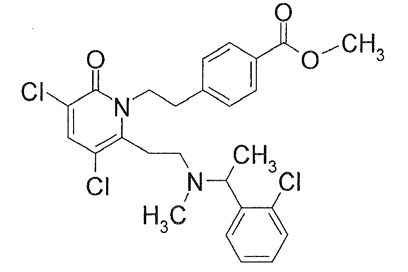 | ESI+: 521 |
| 278 | 31 | 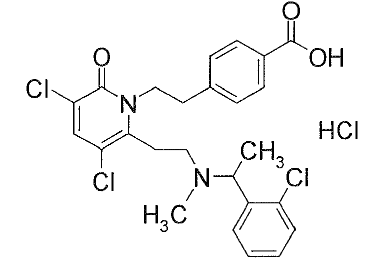 | ESI+: 507 |
| 279 | 29 | 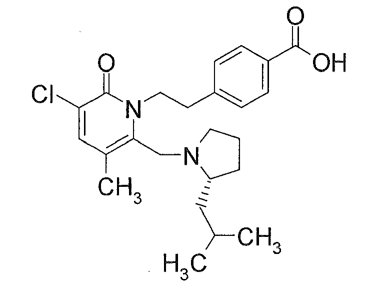 | ESI+: 431, 433 |
| 280 | 26 | 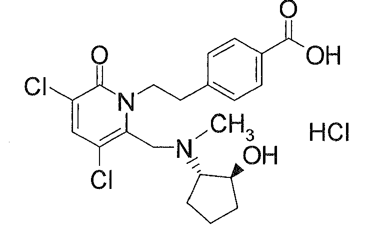 | ESI+: 439 |
| 281 | 26 | 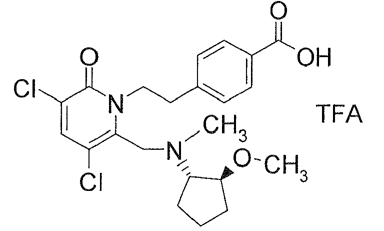 | ESI+: 453 |

**[Table 85]**

| | | | |
|---|---|---|---|
| 282 | 20 | 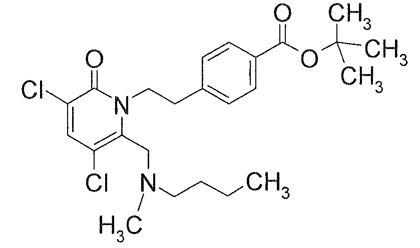 | ESI+: 467 |
| 283 | 1 | 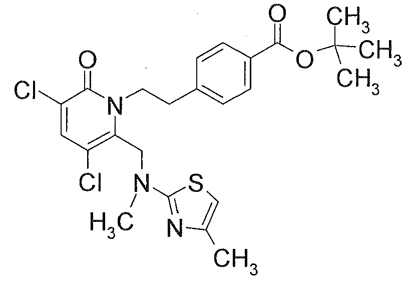 | ESI+: 508 |
| 284 | 26 | 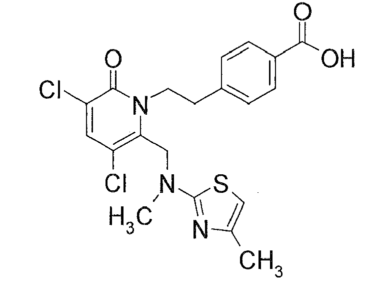 | ESI+: 452 |
| 285 | 20 | 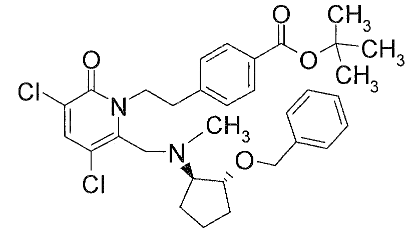 | ESI+: 585 |
| 286 | 20 | 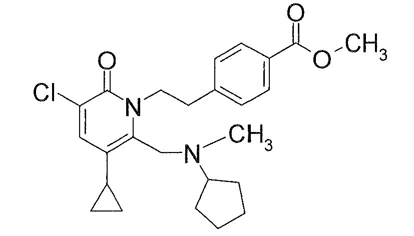 | ESI+: 443, 445 |

**[Table 86]**

| | | | |
|---|---|---|---|
| 287 | 20 | 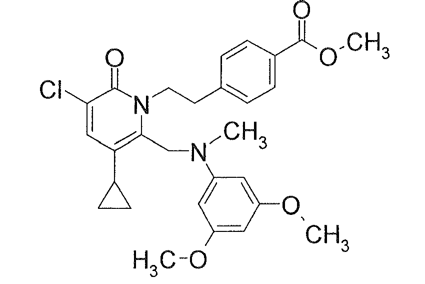 | ESI+: 511, 513, 515 |
| 288 | 26 | 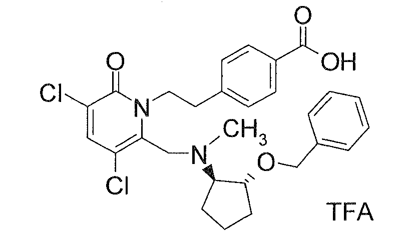 | ESI+: 529 |
| 289 | 26 | 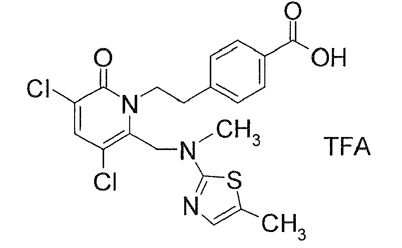 | ESI+: 452 |
| 290 | 29 | 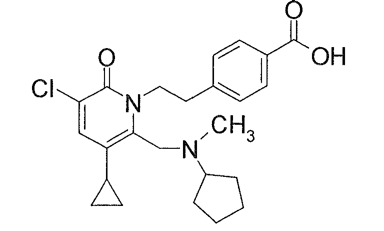 | ESI+: 429, 431 |
| 291 | 29 | 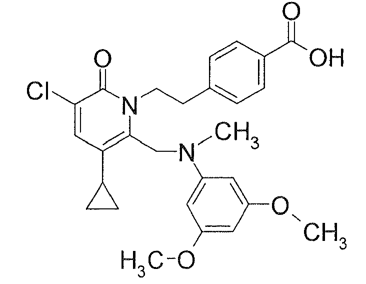 | ESI+: 497, 499 |

**[Table 87]**

| | | | |
|---|---|---|---|
| 292 | 29 | 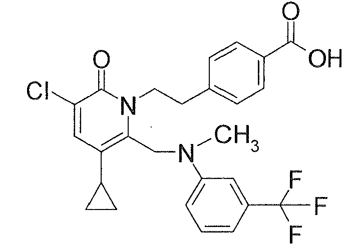 | ESI+: 505, 507 |
| 293 | 47 | 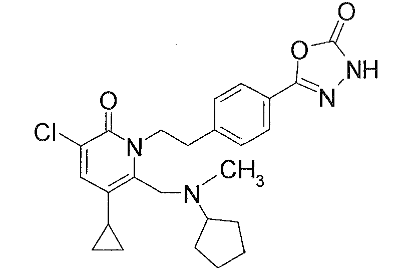 | ESI+: 469, 471 |
| 294 | 47 | 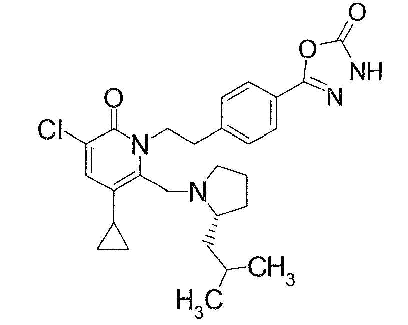 | ESI+:497,499 |
| 295 | 21 | 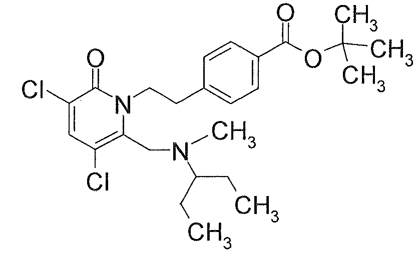 | ESI+: 481 |

**[Table 88]**

| | | | |
|---|---|---|---|
| 296 | 26 | 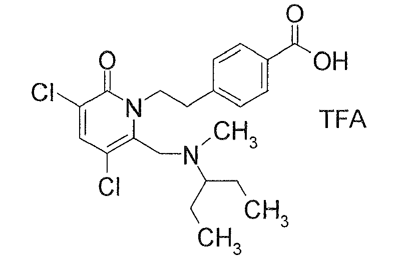 | ESI+: 425 |
| 297 | 20 | 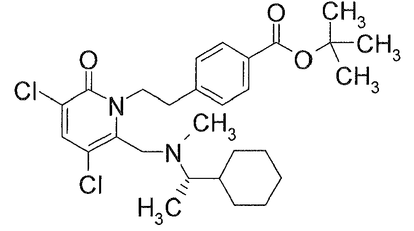 | ESI+: 521 |
| 298 | 20 | 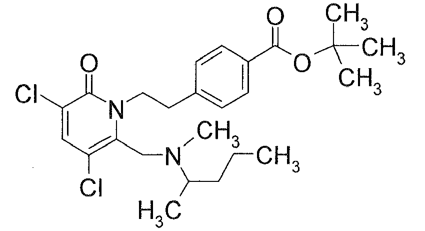 | ESI+: 481 |
| 299 | 26 | 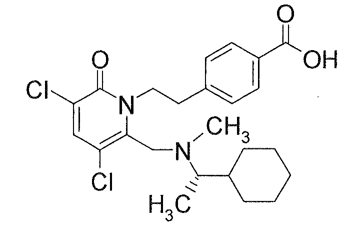 | ESI+: 465 |
| 300 | 26 | 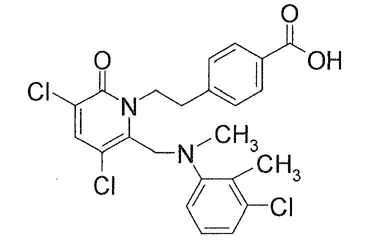 | ESI+: 479 |

**[Table 89]**

| | | | |
|---|---|---|---|
| 301 | 26 | 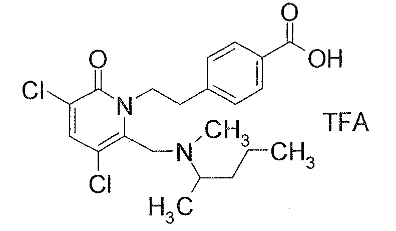 | ESI+: 425 |
| 302 | 20 | 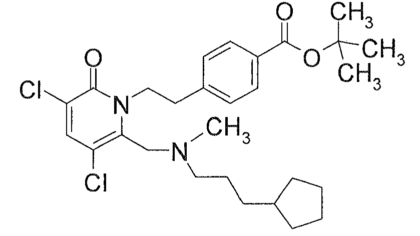 | ESI+: 521 |
| 303 | 26 | 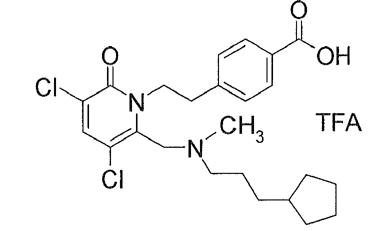 | ESI+: 465 |
| 304 | 20 | 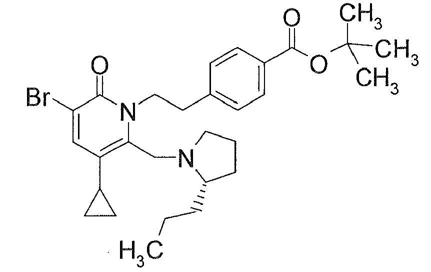 | ESI+: 543, 545 |
| 305 | 20 | 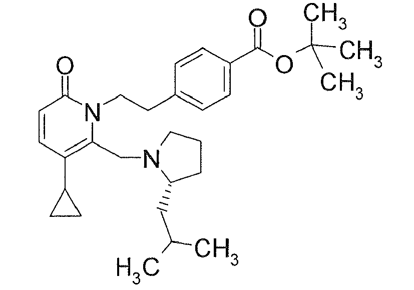 | ESI+: 479 |

**[Table 90]**

| | | | |
|---|---|---|---|
| 306 | 40 | 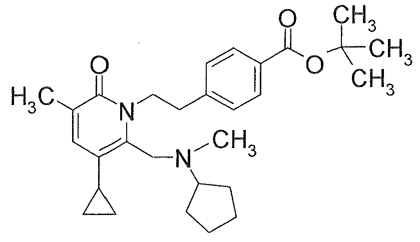 | ESI+: 465 |
| 307 | 40 | 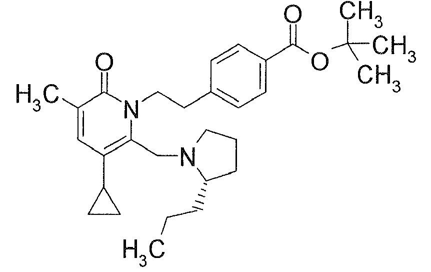 | ESI+: 479 |
| 308 | 26 | 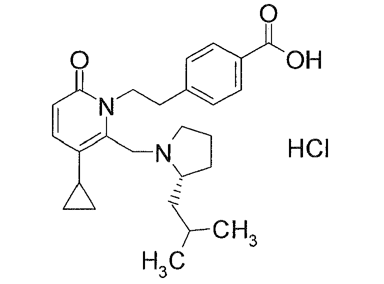 | ESI+: 423 |
| 309 | 26 | 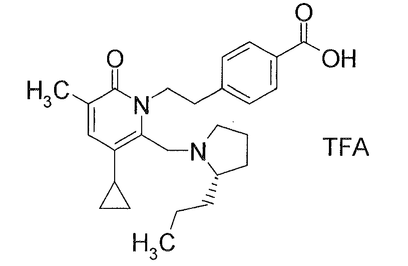 | ESI+: 423 |
| 310 | 26 | 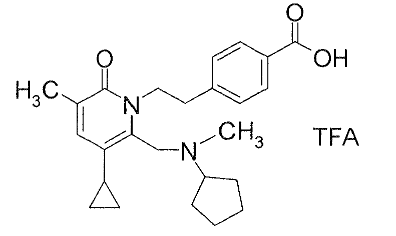 | ESI+: 409 |

**[Table 91]**

| | | | |
|---|---|---|---|
| 311 | 20 | 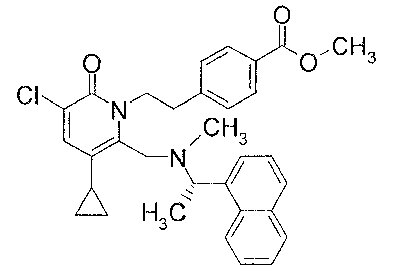 | ESI+: 529 |
| 312 | 29 | 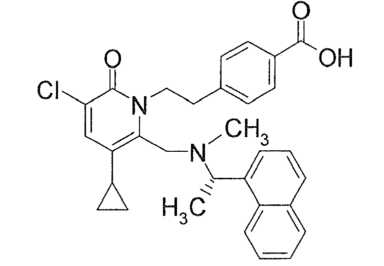 | ESI+: 515, 517 |
| 313 | 22 | 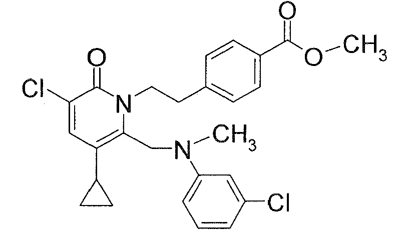 | ESI+: 485, 487 |
| 314 | 29 | 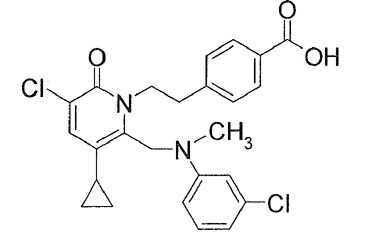 | ESI+: 471,473 |
| 315 | 34 | 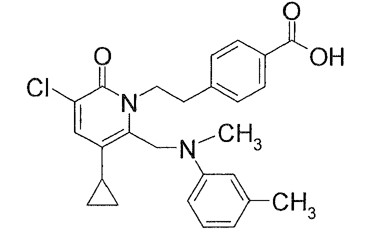 | ESI+: 451 |

**[Table 92]**

| | | | |
|---|---|---|---|
| 316 | 23 | 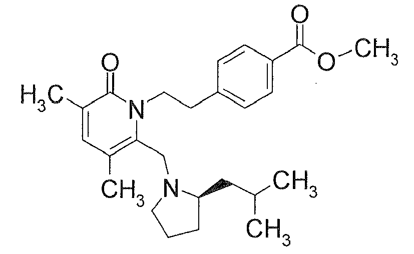 | ESI+: 425 |
| 317 | 20 | 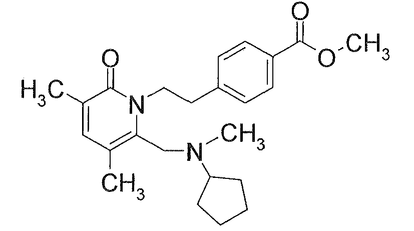 | ESI+: 397 |
| 318 | 22 | 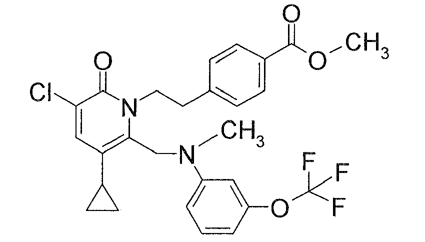 | ESI+: 535 |
| 319 | 31 | 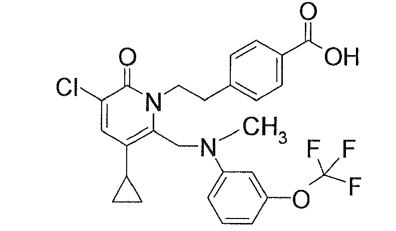 | ESI+: 521 |
| 320 | 31 | 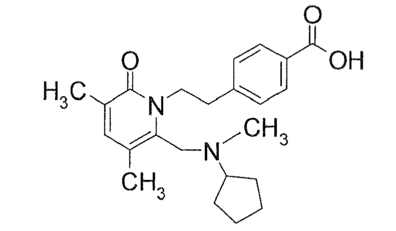 | ESI+: 383 |

**[Table 93]**

| | | | |
|---|---|---|---|
| 321 | 22 | 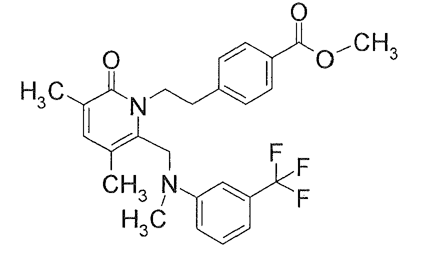 | ESI+: 473 |
| 322 | 31 | 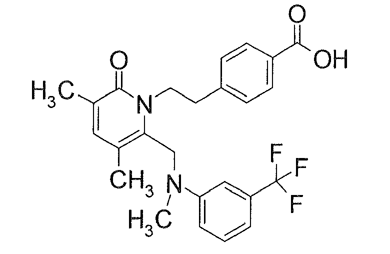 | ESI+: 459 |
| 323 | 41 | 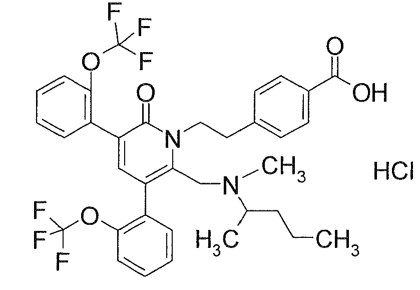 | ESI+: 551 |
| 324 | 23 | 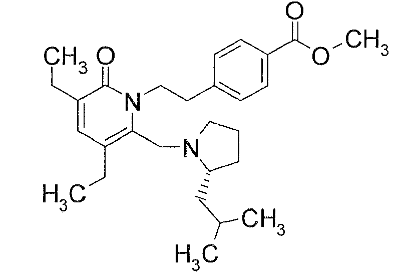 | ESI+: 453 |
| 325 | 22 | 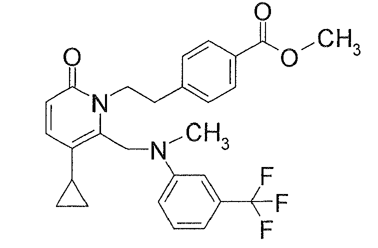 | ESI+: 485 |

**[Table 94]**

| | | | |
|---|---|---|---|
| 326 | 31 | 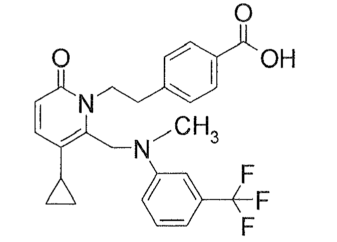 | ESI+: 471 |
| 327 | 31 | 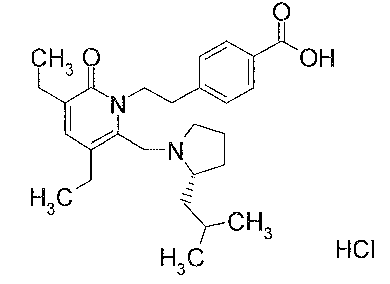 | ESI+: 439 |
| 328 | 23 | 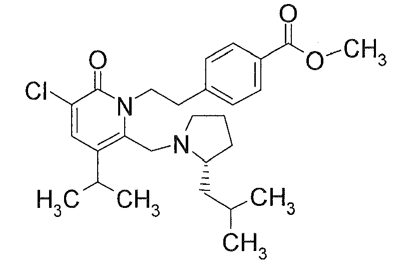 | ESI+: 473, 475 |
| 329 | 29 | 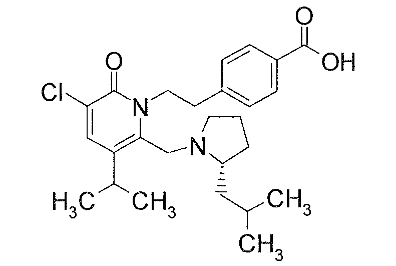 | ESI+: 459, 461 |
| 330 | 23 | 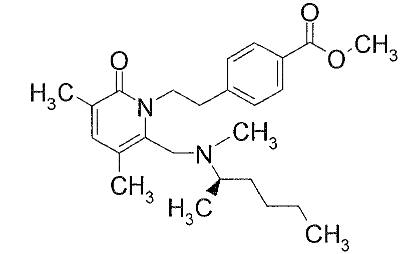 | ESI+: 413 |

**[Table 95]**

| | | | |
|---|---|---|---|
| 331 | 23 | 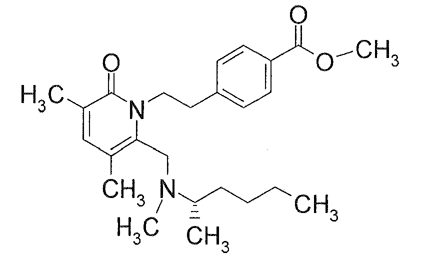 | ESI+: 413 |
| 332 | 20 | 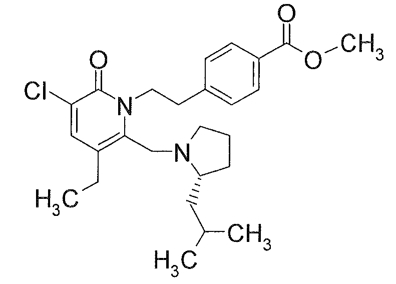 | ESI+: 459, 461 |
| 333 | 29 | 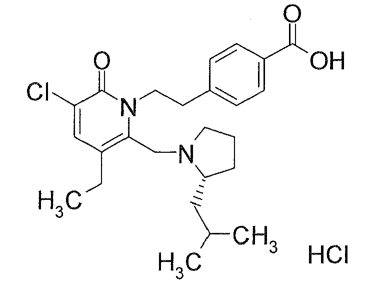 | ESI+:445,447 |
| 334 | 31 | 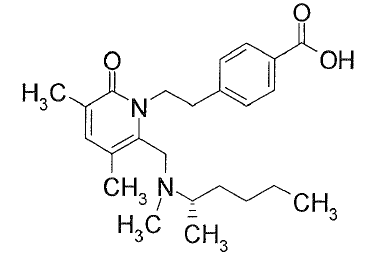 | ESI+: 399 |
| 335 | 31 | 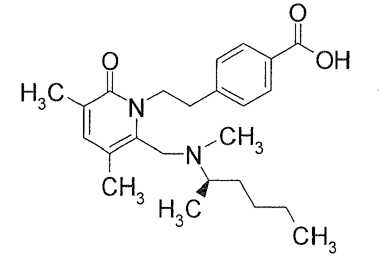 | ESI+: 399 |

**[Table 96]**

| | | | |
|---|---|---|---|
| 336 | 29 | 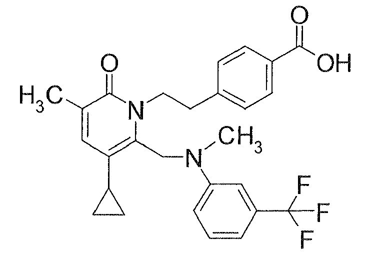 | ESI+: 485 |
| 337 | 20 | 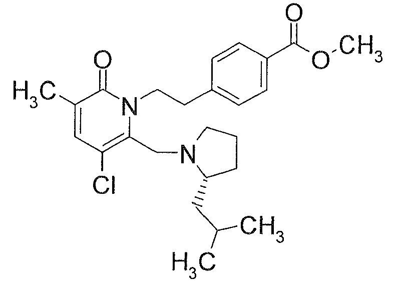 | ESI+: 445 |
| 338 | 31 | 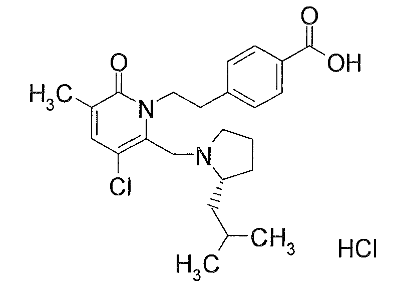 | ESI+: 431 |
| 339 | 29 | 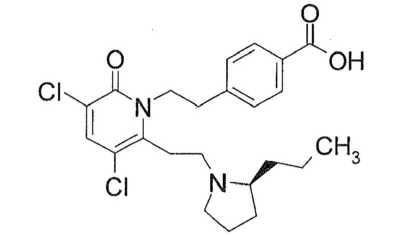 | ESI+: 451 |
| 340 | 20 | 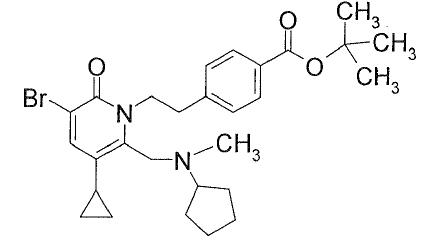 | ESI+: 529 |

**[Table 97]**

| | | | |
|---|---|---|---|
| 341 | 20 | 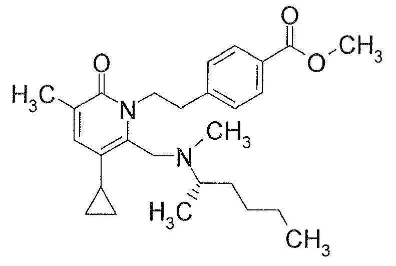 | ESI+: 439 |
| 342 | 29 | 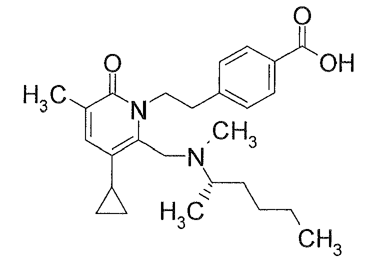 | ESI+: 425 |
| 343 | 22 | 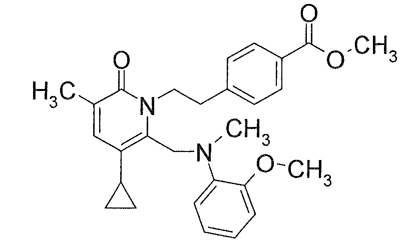 | APCI/ESI+:461 |
| 344 | 29 | 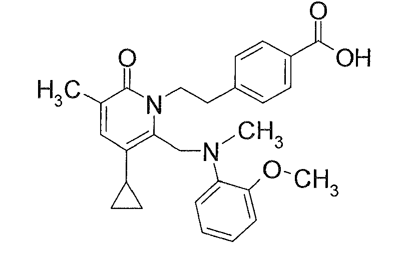 | ESI+: 447 |

**[Table 98]**

| | | | |
|---|---|---|---|
| 345 | 47 | 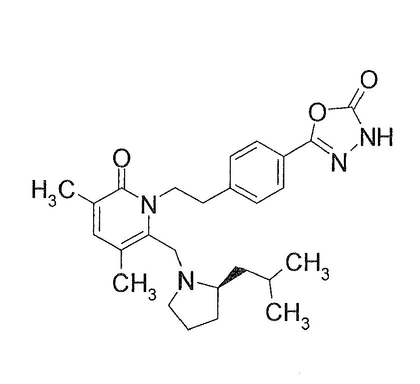 | NMR(400MHz): 0.81 (3H, d, J = 6.4 Hz), 0.84 (3H, d, J = 6.4 Hz), 0.93-1.05 (1H, m), 1.25-1.67 (5H, m), 1.84-1.99 (1H, m), 2.02 (3H, s), 2.11 (3H, s), 2.19 (1H, dd, J = 17.2, 8.8 Hz), 2.29-2.41 (1H, m), 2.70 (1H, t, J = 7.2 Hz), 2.98 (2H, dd, J = 8.0, 8.0 Hz), 3.24 (1H, d, J = 13.6 Hz), 3.29 (1H, d, J = 13.6 Hz), 4.25-4.38 (1H, m), 4.39-4.52 (1H, m), 7.19 (1H, s), 7.42 (2H, d, J = 8.4 Hz), 7.74 (2H, d, J = 8.4 Hz), 12.5 (br); ESI+: 451 |
| 346 | 47 | 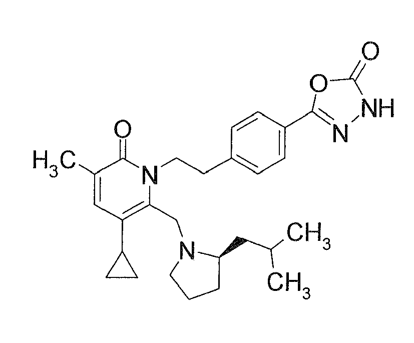 | NMR(400MHz): 0.47-0.62 (2H, m), 0.78-0.89 (8H, m), 0.96-1.05 (1H, m), 1.27-1.39 (1H, m), 1.40-1.67 (4H, m), 1.81-1.99 (2H, m), 2.01 (3H, s), 2.25 (1H, dd, J = 17.2, 8.8 Hz), 2.30-2.42 (1H, m), 2.65-2.77 (1H, m), 2.98 (2H, dd, J = 8.0, 8.0 Hz), 3.59 (1H, d, J = 13.6 Hz), 3.79 (1H, d, J = 13.6 Hz), 4.27-4.41 (1H, m), 4.42-4.55 (1H, m), 7.05 (1H, s), 7.40 (2H, d, J = 8.4 Hz), 7.73 (2H, d, J = 8.4 Hz), 12.55 (br); ESI+: 477 |
| 347 | 22 | 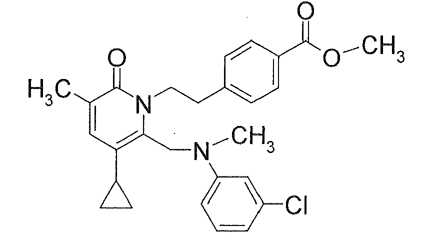 | ESI+: 465, 467 |

**[Table 99]**

| | | | |
|---|---|---|---|
| 348 | 29 | 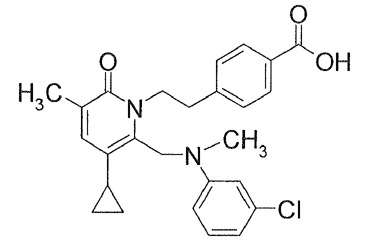 | ESI+: 451 |
| 349 | 22 | 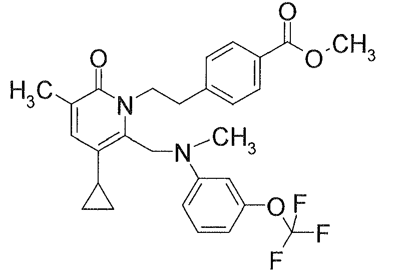 | ESI+: 515 |
| 350 | 29 | 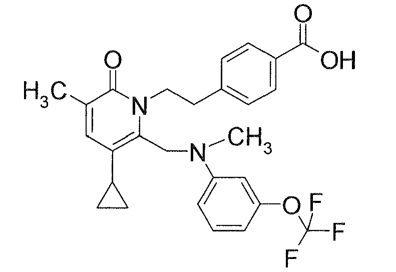 | ESI+: 501 |
| 351 | 20 | 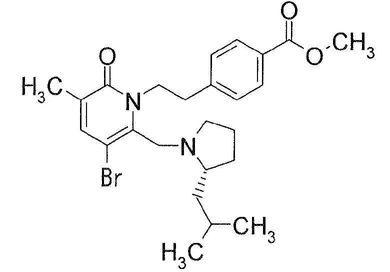 | ESI+: 489, 491 |

**[Table 100]**

| | | | |
|---|---|---|---|
| 352 | 29 | 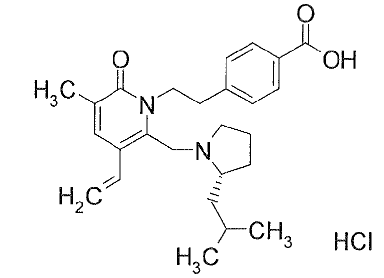 | ESI+: 423 |

**[Table 101]**

| No | Str |
|---|---|
| 1 | 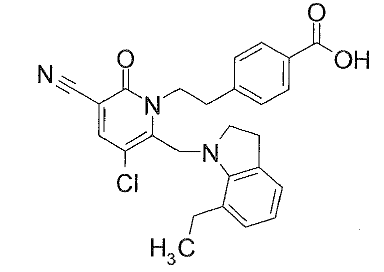 |
| 2 | 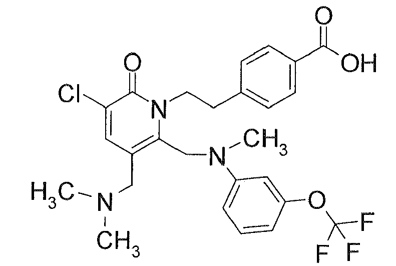 |
| 3 | 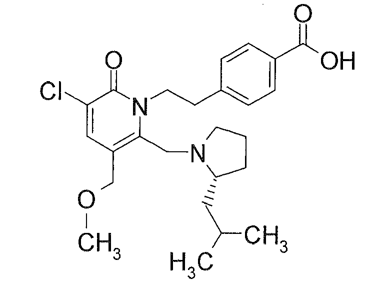 |
| 4 | 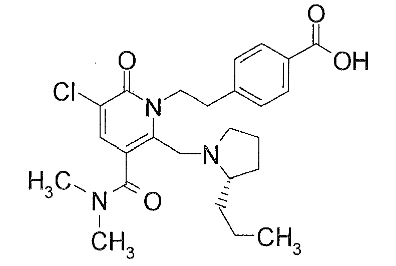 |

### Sequence Listing Free Text

The nucleotide sequence of a rat EP4 (Sequence Number 1) is described in the numeral index <400> of the following Sequence Listing.

## Claims

1. A compound of the formula (I) or a pharmaceutically acceptable salt thereof: 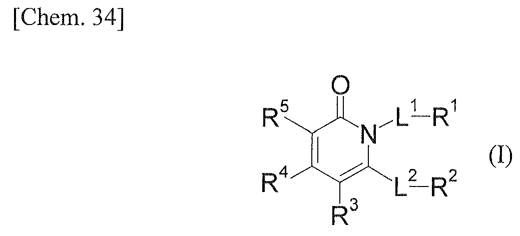 [wherein the symbols in the formula have the following meanings:
L¹: lower alkylene, lower alkenylene, -(lower alkylene)-O-(lower alkylene)-, or -(lower alkylene)-S-(lower alkylene)-, in which lower alkylene and lower alkenylene in L¹ may each be substituted,
L²: lower alkylene, lower alkenylene, -C(O)-, -(lower alkylene)-C(O)-, or -(lower alkenylene)-C(O)-, in which lower alkylene and lower alkenylene in L² may each be substituted,
R¹: R⁶ or a group represented by the following formula (II): 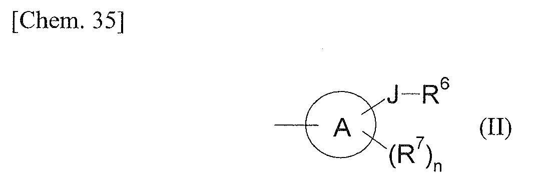
Ring A: aryl or heteroaryl,
R⁶: -CO₂R⁰, -C(O)N(R⁰)₂, -C(O)N(H)S(O)₂-R⁸, -C(O)N(H)S(O)₂N(R⁰)-R⁸, -N(R⁰)C(O)N(H)S(O)₂-R⁸, or a group represented by any one of the following formulae (III) to (XIV): 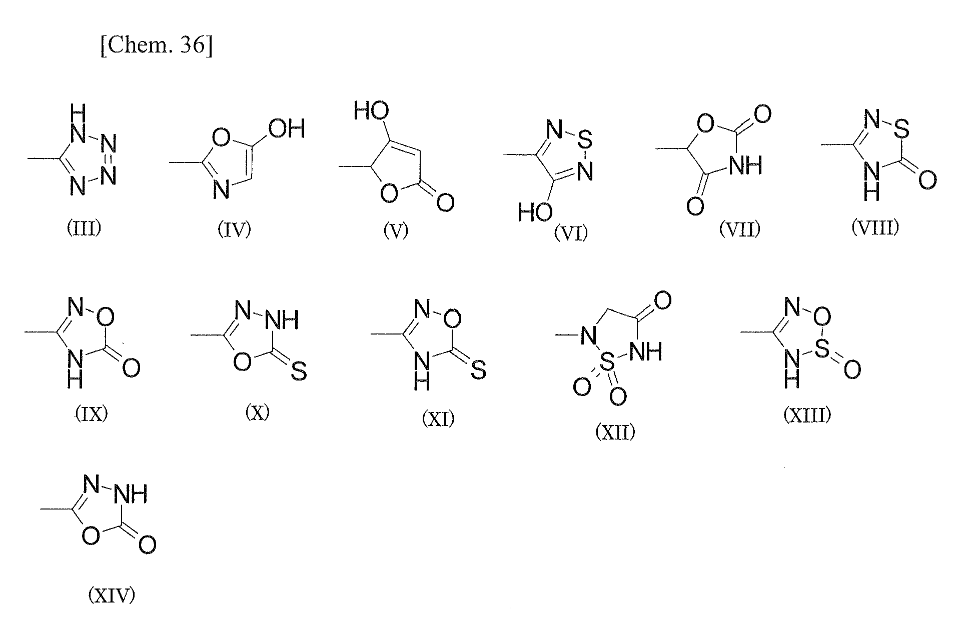
R⁰: the same as or different from each other, each representing -H or lower alkyl,
R⁸: lower alkyl, halogeno-lower alkyl, -(lower alkylene)-OR⁰, or -(lower alkylene)-OC(O)R⁰,
J: a single bond or lower alkylene,
R⁷: the same as or different from each other, each representing lower alkyl, lower alkenyl, halogen, halogeno-lower alkyl, -OR⁰, -O-(halogeno-lower alkyl), -O-(cycloalkyl), -O-(lower alkylene)-OR⁰, -O-(lower alkylene)-aryl, -OC(O)R⁰, -N(R⁰)₂, -(lower alkylene)-OH, -(lower alkylene)-OR⁰, -(lower alkylene)-N(R⁰)₂, -(lower alkylene)-cycloalkyl, -(lower alkylene)-aryl, -CO-R⁰, -S(O)₂-R⁰, -CO₂R⁰, -C(O)N(R⁰)₂, aryl, or a hetero ring group, in which aryl and the hetero ring group in R⁷ may each be substituted,
n: an integer of 0 to 3,
R²: -N(R⁰)-lower alkyl or a group represented by any one of the following formulae (XV) to (XVII): 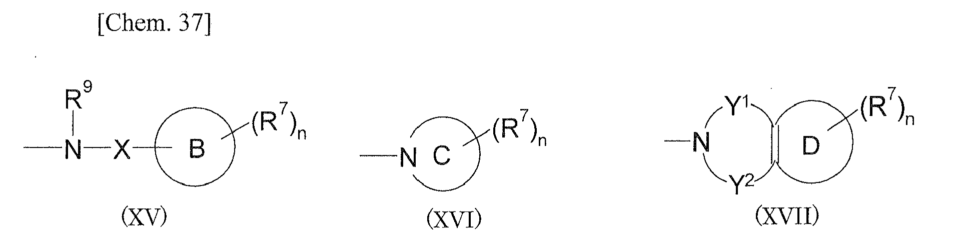
Ring B: cycloalkyl, aryl, or a hetero ring,
Ring C: a nitrogen-containing saturated hetero ring,
Ring D: aryl or heteroaryl,
X: a single bond, lower alkylene, -C(O)-, -C(O)-(lower alkylene)-, or -(lower alkylene)-O-,
R⁹: H, lower alkyl, -C(O)R⁰, or aryl, in which aryl in R⁹ may be substituted,
Y¹ and Y²: the same as or different from each other, each representing a single bond, -[C(R¹⁰)(R¹¹)]ₛ-, -[C(R¹⁰)(R¹¹)]ₛ-Q-, -Q-[C(R¹⁰)(R¹¹)]ₛ-, or -[C(R¹⁰)(R¹¹)]ₛ-Q-[C(R¹⁰)(R¹¹)]ₜ-,
R¹⁰ and R¹¹: the same as or different from each other, each representing H, lower alkyl, halogen, halogeno-lower alkyl, -OR⁰, -N(R⁰)₂, -(lower alkylene)-OH, -(lower alkylene)-OR⁰, -(lower alkylene)-N(R⁰)₂, or a hetero ring group, or R¹⁰ and R¹¹ on the same carbon atom may be combined to form oxo,
Q: O, S(O)ₚ, or N(R¹²),
R¹²: H, lower alkyl, -C(O)R⁰, or -S(O)₂-(lower alkyl),
s and t: the same as or different from each other, each representing an integer of 1 to 4,
p: an integer of 0 to 2, and
R³, R⁴, and R⁵: the same as or different from each other, each representing H, halogen, -CN, lower alkyl, lower alkenyl, halogeno-lower alkyl, -OR⁰, -O-(halogeno-lower alkyl), -(lower alkylene)-OR⁰, -(lower alkylene)-N(R⁰)₂, -CO₂R⁰, -C(O)N(R⁰)₂, cycloalkyl, or aryl, in which aryl in R³, R⁴, and R⁵ may be substituted].

2. The compound as described in claim 1, wherein R⁴ is -H.

3. The compound as described in claim 2, wherein R⁵ is -H, halogen, or lower alkyl.

4. The compound as described in claim 3, wherein R³ is halogen, lower alkyl, or cycloalkyl.

5. The compound as described in claim 4, wherein L¹ is lower alkylene.

6. The compound as described in claim 5, wherein L² is lower alkylene.

7. The compound as described in claim 6, wherein R¹ is -CO₂R⁰; or phenyl which is substituted with a group selected from the group consisting of -CO₂R⁰ and the groups represented by the following formula (III), the following formula (IX), the following formula (X), and the following formula (XIV): 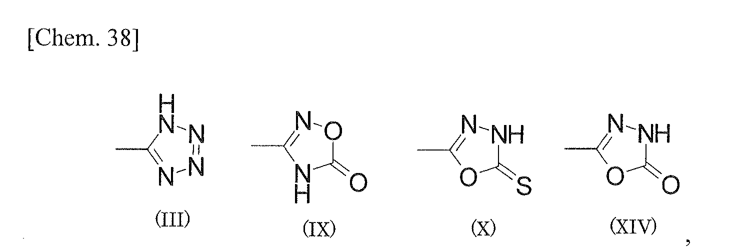 and which may be further substituted with 1 to 3 lower alkyl or halogen.

8. The compound as described in claim 7, wherein R² is -N(lower alkyl)₂; -N(R⁰)-cycloalkyl; -N(R⁰)-(aryl which may be substituted with 1 to 3 groups selected from the group consisting of halogen, lower alkyl, halogeno-lower alkyl, -OR⁰, and -O-halogeno-lower alkyl); or -N(R⁰)-(lower alkylene)-(aryl which may be substituted with 1 to 3 groups selected from the group consisting of halogen, lower alkyl, halogeno-lower alkyl, -OR⁰, and -O-halogeno-lower alkyl).

9. The compound as described in claim 7, wherein R² is a group described by the following formula (XVI): 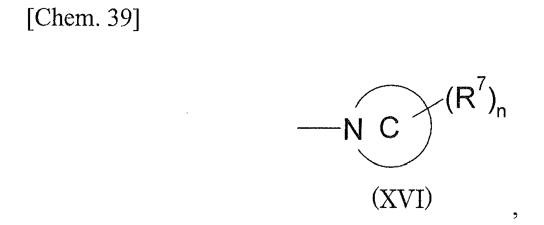 Ring C is a monocyclic 5- to 7-membered nitrogen-containing saturated hetero ring, and R⁷ is halogen, lower alkyl, lower alkenyl, halogeno-lower alkyl, -OR⁰, -O-halogeno-lower alkyl, or -(lower alkylene)-cycloalkyl.

10. The compound as described in claim 7, wherein R² is 2,3-dihydro-1H-indol-1-yl in which 1 to 3 groups selected from the group consisting of halogen, lower alkyl, lower alkenyl, halogeno-lower alkyl, -OR⁰, and -O-halogeno-lower alkyl may be substituted at the 4- to 7-positions.

11. The compound as described in claim 1, which is selected from the group consisting of:
4-{2-[6-{[(2S)-2-butylpyrrolidin-1-yl]methyl}-3,5-dichloro-2-oxopyridin-1(2H)-yl]ethyl}benzoic acid,
4-{2-[3,5-dichloro-6-{[(2R)-2-isobutylpyrrolidin-1-yl]methyl}-2-oxopyridin-1(2H)-yl]ethyl}benzoic acid,
4-{2-[3,5-dichloro-2-oxo-6-{[(2S)-2-propylpyrrolidin-1-yl]methyl}pyridin-1(2H)-yl]ethyl}benzoin acid,
3,5-dichloro-6-{[(2S)-2-propylpyrrolidin-1-yl]methyl}-1-{2-[4-(1H-tetrazol-5-yl)phenyl]ethyl}pyridin-2(1H)-one,
3,5-dichloro-1-{2-[4-(5-oxo-4,5-dihydro-1,3,4-oxadiazol-2-yl)phenyl]ethyl}-6-{[(2S)-2-propylpyrrolidin-1-yl]methyl}pyridin-2(1H)-one,
4-{2-[3,5-dichloro-6-{[cyclopentyl(methyl)amino]methyl}-2-oxopyridin-1(2H)-yl]ethyl}benzoic acid,
3,5-dichloro-1-{2-[4-(5-oxo-4,5-dihydro-1,3,4-oxadiazol-2-yl)phenyl]ethyl}-6-{[(2R)-2-isobutylpyrrolidin-1-yl]methyl}pyridin-2(1H)-one,
4-{2-[5-cyclopropyl-6-{[(2R)-2-isobutylpyrrolidin-1-yl]methyl}-3-methyl-2-oxopyridin-1(2H)-yl]ethyl}benzoic acid,
4-{2-[6-{[(2R)-2-isobutylpyrrolidin-1-yl]methyl}-3,5-dimethyl-2-oxopyridin-1(2H)-yl]ethyl}benzoic acid,
1-{2-[4-(5-oxo-4,5-dihydro-1,3,4-oxadiazol-2-yl)phenyl]ethyl}-6-{[(2R)-2-isobutylpyrrolidin-1-yl]methyl}-3,5-dimethylpyridin-2(1H)-one,
5-cyclopropyl-1-{2-[4-(5-oxo-4,5-dihydro-1,3,4-oxadiazol-2-yl)phenyl]ethyl}-6-{[(2R)-2-isobutylpyrrolidin-1-yl]methyl}-3-methylpyridin-2(1H)-one,
4-{2-[3,5-dichloro-2-oxo-6-({[3-(trifluoromethoxy)phenyl]amino}methyl)pyridin-1(2H)-yl]ethyl}benzoic acid,
4-{2-[3-chloro-5-cyclopropyl-2-oxo-6-{[(2S)-2-propylpyrrolidin-1-yl]methyl}pyridin-1(2H)-yl]ethyl}benzoic acid,
4-{2-[3-chloro-5-cyclopropyl-6-{[(2R)-2-isobutylpyrrolidin-1-yl]methyl}-2-oxopyridin-1(2H)-yl]ethyl}benzoic acid,
4-{2-[3-chloro-5-ethyl-6-{[(2R)-2-isobutylpyrrolidin-1-yl]methyl}-2-oxopyridin-1(2H)-yl]ethyl}benzoic acid,
4-{2-[3,5-dichloro-6-{[methyl(3-methylphenyl)amino]methyl}-2-oxopyridin-1(2H)-yl]ethyl}benzoic acid,
4-{2-[3,5-dichloro-6-({methyl[3-(trifluoromethyl)phenyl]amino}methyl)-2-oxopyridin-1(2H)-yl]ethyl}benzoic acid,
4-{2-[3,5-dichloro-6-{[(3-chlorophenyl)(methyl)amino]methyl}-2-oxopyridin-1(2H)-yl]ethyl}benzoic acid,
4-{2-[3-chloro-5-cyclopropyl-6-{[methyl(3-methylphenyl)amino]methyl}-2-oxopyridin-1(2H)-yl]ethyl}benzoic acid,
4-(2-{3,5-dichloro-6-[(6-ethyl-2,3-dihydro-1H-indol-1-yl)methyl]-2-oxopyridin-1(2H)-yl}ethyl)benzoic acid,
4-{2-[3,5-dichloro-2-oxo-6-{[6-(trifluoromethoxy)-2,3-dihydro-1H-indol-1-yl]methyl}pyridin-(2H)-yl]ethyl}benzoic acid,
4-(2-{3,5-dichloro-6-[(6-ethoxy-2,3-dihydro-1H-indol-1-yl)methyl]-2-oxopyridin-1(2H)-yl}ethyl)benzoic acid,
4-(2-{3,5-dichloro-6-[(6-fluoro-2,3-dihydro-1H-indol-1-yl)methyl]-2-oxopyridin-1(2H)-yl}ethyl)benzoic acid,
4-(2-{3,5-dichloro-6-[(6-fluoro-7-methyl-2,3-dihydro-1H-indol-1-yl)methyl]-2-oxopyridin-1(2H)-yl}ethyl)benzoic acid,
4-(2-{3,5-dichloro-6-[(7-ethyl-2,3-dihydro-1H-indol-1-yl)methyl]-2-oxopyridin-1(2H)-yl}ethyl)benzoic acid, and
4-(2-{3,5-dichloro-6-[(7-methyl-2,3-dihydro-1H-indol-1-yl)methyl]-2-oxopyridin-1(2H)-yl}ethyl)benzoic acid,
or a pharmaceutically acceptable salt thereof.

12. A pharmaceutical composition comprising the compound as described in claim 1 or a pharmaceutically acceptable salt thereof, and a pharmaceutically acceptable carrier.

13. The pharmaceutical composition as described in claim 12, which is an EP4 agonist.

14. The pharmaceutical composition as described in claim 12, which is an agent for preventing or treating peripheral arterial occlusive disease.

15. Use of the compound as described in claim 1 or a pharmaceutically acceptable salt thereof for the manufacture of an agent for preventing or treating peripheral arterial occlusive disease.

16. A method for preventing or treating peripheral arterial occlusive disease, comprising administering to a patient an effective amount of the compound as described in claim 1 or a pharmaceutically acceptable salt thereof.
